(19) Europäisches Patentamt
European Patent Office
Office européen des brevets

(11) Veröffentlichungsnummer: **0 190 105 A2**

# (12) EUROPÄISCHE PATENTANMELDUNG

(21) Anmeldenummer: **86810046.2**

(22) Anmeldetag: **27.01.86**

(51) Int. Cl.[4]: **A 01 N 25/32**
A 01 N 57/22, A 01 N 47/36
C 07 D 407/12, C 07 D 317/58
C 07 D 217/06
//(A01N57/22, 47:36),
(A01N47/36, 43:90, 43:84,
43:78, 43:76, 43:60, 43:40,
43:36, 43:30, 37:18)

(30) Priorität: **31.01.85 CH 418/85**

(43) Veröffentlichungstag der Anmeldung:
**06.08.86 Patentblatt 86/32**

(84) Benannte Vertragsstaaten:
**BE CH DE FR GB IT LI NL**

(71) Anmelder: **CIBA-GEIGY AG**
**Klybeckstrasse 141**
**CH-4002 Basel(CH)**

(72) Erfinder: **Föry, Werner, Dr.**
**Inzlingerstrasse 11**
**CH-4125 Riehen(CH)**

(72) Erfinder: **Nyffeler, Andreas, Dr.**
**Gründlerstrasse 4**
**CH-4312 Magden(CH)**

(72) Erfinder: **Gerber, Hans-Rudolf, Dr.**
**Blözenweg 49**
**CH-4133 Pratteln(CH)**

(72) Erfinder: **Martin, Henry, Dr.**
**Schützenmattstrasse 52**
**CH-4051 Basel(CH)**

## (54) Herbizides Mittel.

(57) Es wird ein selektives herbizides Mittel beschrieben, welche neben inerten Zutaten

a) den herbizid wirksamen Sulfonylharnstoff N-(2-Methoxycarbony-1-phenylsulfonyl)-N'-(bis-difluoro-methoxypyrimidin-2-yl)-harnstoff der Formel I

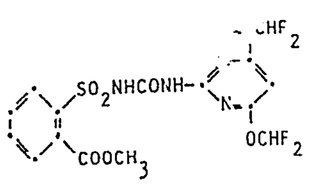

(I)

und

b) zur Erhöhung der Toleranz der Kultur eine nicht-phytotoxische Menge eines Dichloracetamides der Formel II enthält

$R_a-N(R_b)-COCHCl_2$ (II)

worin $R_a$ und $R_b$ unabhängig voneinander je Wasserstoff einen unsubstituierten oder substituierten alicyclischen Kohlenwasserstoffrest oder eines davon ein Amin oder beide zusammen mit dem Stickstoffatom, an das sie gebunden sind auch einen 5-12-gleidrigen heterocyclischen Rest bedeuten.

Das Mittel eignet sich zur Unkrautbekämpfung in Nutzflanzenkulturen z.B. von Getreide, Mais, Reis und Hirse etc.

EP 0 190 105 A2

0190105

CIBA-GEIGY AG

Basel (Schweiz)

5-15247/1+2

## Herbizides Mittel

Die Erfindung betrifft ein selektiv wirkendes Mittel, welches als herbiziden Wirkstoff den Sulfonylharnstoff N-(2-Methoxycarbonyl)-phenylsulfonyl-N'-(4,6-bis-difluoromethoxy-pyrimidin-2-yl)-harnstoff der Formel I enthält

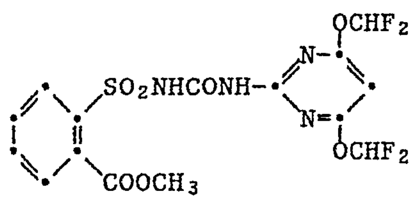

(I)

zusammen mit einem antagonistisch wirkenden Amid der Dichloressigsäure (Antidote), der Formel II

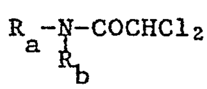

worin $R_a$ und $R_b$ unabhängig voneinander je Wasserstoff einen unsubstituierten oder substituierten alicyclischen Kohlenwasserstoffrest oder eines davon einen Aminorest oder beide zusammen mit dem Stickstoffatom, an das sie gebunden sind auch einen 5-12-gliedrigen heterocyclischen Rest bedeuten, welche die Toleranz der Kulturpflanzen gegenüber der phytotoxischen Wirkung erhöht, ohne dass deswegen die Herbizidwirkung gegenüber den Unkräutern merklich abfällt. Die Erfindung betrifft ebenfalls die Verwendung dieses Mittels zur Unkrautbekämpfung in Nutzpflanzenkulturen.

Der Sulfonylharnstoff als Herbizid ist bekannt, siehe beispielsweise die EP-A 84 020, ZA 127/83, USP 4,478,635.

N-(2-Methoxycarbonyl)phenylsulfonyl-N'-(4,6-bis-difluormethoxy-pyrimidin-2-yl)-harnstoff ist ein Herbizid mit recht starker Wirkung. Dies bringt die Gefahr schädigender Ueberdosierung mit sich. Schadwirkungen sind nicht nur auf Ueberdosierung zurückzuführen, sie können auch auftreten wegen abnormalen klimatischen Verhältnissen oder wegen Vorbehandlung des Bodens.

Es hat sich deshalb als wünschenswert erwiesen, die Resistenz oder Toleranz der Kulturpflanzen gegenüber der phytotoxischen Wirkung der Sulfonylharnstoff-Herbizide zu erhalten. Im US Patent No. 4 343 649 wird beschrieben, dass die bekannten Herbizid-Antidoten 1,8-Naphthalinsäureanhydrid, N,N-Diallyldichloracetamid sowie α-Cyano-methoximino-2-phenylacetonitril diese Aufgabe erfüllen, wenn man sie in Nutzpflanzenkulturen gleichzeitig mit dem Sulfonylharnstoff-Herbizid verwendet. Hingegen ist die Toleranz dieser bekannten Herbizid-Safener-Mischungen speziell in Maiskulturen für einen praktischen Einsatz ungenügend.

Ueberraschenderweise wurde nun gefunden, dass sich die Toleranz von Kulturpflanzen speziell Mais gegenüber der phytotoxischen Wirkung des obigen Sulfonylharnstoff-Herbizides stark erhöht, ohne dass dadurch die Herbizidwirkung auf die Grosszahl der Unkräuter und Ungräser beeinträchtigt wird, wenn man das Sulfonylharnstoff-Herbizid zusammen mit einem Amid der Dichloressigsäure verwendet welches als Herbizid-Antagonist oder Antidot wirkt.

Die Herstellung des N-(2-Methoxycarbonylphenylsulfonyl)-N'-(4,6-bis-(difluormethoxy)-pyrimidin-2-yl-harnstoffes der Formel I geschieht nach an sich bekannten Methoden und ist z.B. in der veröffentlichten Europäischen Patentanmeldung EP-A 84 020 beschrieben.

Man setzt beispielsweise in einem inerten organischen Lösungsmittel 2-Methoxycarbonylphenylsulfonylamid mit dem 2-Phenoxycarbamat des 4,6-bis(difluoromethoxy)-pyrimidins um gemäss dem Reaktionsschema:

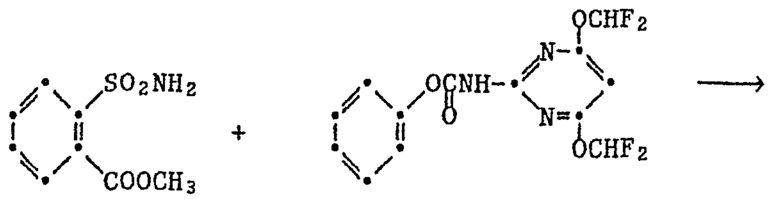

I

Ferner kann man ebenfalls in einem inerten organischen Lösungsmittel das 2-Methoxycarbonylphenylsulfonylisocyanat mit 2-Amino-4,6-bis(difluoromethoxy)pyrimidin umsetzen, gemäss dem Reaktionsschema

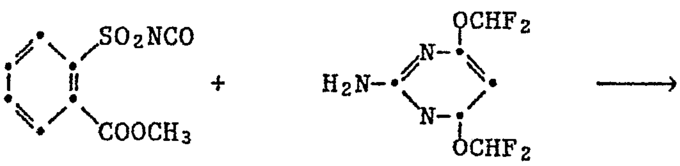

I

Ebensogut kann man in einem inerten organischen Lösungsmittel 2-Methoxycarbonyl-phenyl-sulfonamid mit 2-Isocyanato-4,6-bis(difluormehoxy)pyrimidin umsetzen gemäss dem Reaktionsschema

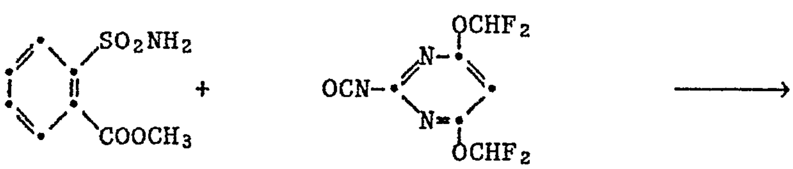

I

Schliesslich kann man in einem inerten organischen Lösungsmittel, das Phenoxycarbamat des 2-Methoxycarbonylphenylsulfonamides mit dem 2-Amino-4,6-bis(difluoromethoxy)pyrimidin umsetzt, gemäss dem Reaktionsschema

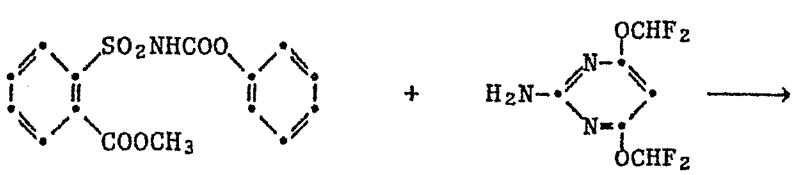

I

Die Umsetzungen werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe von äquimolaren oder katalytischen Mengen von Basen verkürzt werden.

Als Basen können sowohl organische Basen wie Amine, wie Triäthylamin, Chinuclidin, Pyridin etc. als auch anorganische Basen wie Hydride wie Natrium- oder Calciumhydrid, Carbonate wie Natrium- und Kaliumcarbonat verwendet werden.

Das Endprodukt kann durch Einengen und/oder Verdampfen des Lösungsmittels isoliert und durch Umkristallisieren oder Zerreiben des festen Rückstandes in Lösungsmitteln in denen es sich nicht gut lösten, wie Aether, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglichen Massnahme.

Die Zwischenprodukte zur Herstellung des N-(2-Methoxycarbonyl-phenylsulfonyl-N'-[4,6-bis-(difluoromethoxy)pyrimidin-2-yl]-harnstoffes sind bekannt oder können nach bekannten Methoden hergestellt werden.

Als Gegenmittel oder Antidote zum Sulfonylharnstoff der Formel I werden erfindungsgemäss Amide der Dichloressigsäure der Formel II verwendet.

Eine grössere Anzahl dieser Verbindungen sind als Safener oder Antidotes für Herbizide mit anderer als Sulfonylharnstoff-Struktur beschrieben worden. Solche Herbizide müssen in vielen Fällen in höheren Aufwandmengen angewendet werden. Ueberraschenderweise hat es sich nun gezeigt, dass sie auch ganz besonders den Sulfonylharnstoff der Formel I, welcher eine sehr starke herbizide Wirkung aufweist, kulturspezifisch speziell in Maiskulturen zu antagonisieren vermögen.

Die Verbindungen der Formel II bis XVI sind zum Schützen von Kulturpflanzen gegen die schädigende Wirkung des Sulfonylharnstoffes geeignet und können daher in bezug auf ihre Anwendung in Kombination mit dem vorgenannten Herbizid als Gegenmittel, "Antidote" oder auch als "Safener" bezeichnet werden.

Ein solches Gegenmittel oder Antidote kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalien erfolgen. Die Behandlung der Pflanzen kann jedoch durch gleichzeitige Applikation des Sulfonamides der Formel I und des Gegenmittels (Tankmischung) erfolgen. Die preemergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:100 bis 5:1. In der Regel wird bei einem Mengenverhältnis von Gegenmittel zu

Herbizid von 1:5 bis 1:50 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,1 - 10 g Gegenmittel benötigt. In der Regel wird mit 0,1 - 5 g Gegenmittel pro kg Samen bereits die volle Schutzwirkung erreicht. Falls das Gegenmittel kurz vor der Aussaat durch Samenquellung appliziert werden soll, so werden zweckmässig Lösungen des Gegenmittels verwendet, welche den Wirkstoff in einer Konzentration von 1 - 10'000 ppm enthalten. In der Regel wird mit Konzentrationen des Gegenmittels von 100 - 1'000 ppm die volle Schutzwirkung erreicht.

In der Regel liegt zwischen protektiven Massnahmen, wie Samenbeizung und Behandlung von Stecklingen mit einem Gegenmittel der Formel II und der möglichen späteren Feldbehandlung mit Agrarchemikalien ein längerer Zeitraum. Vorbehandeltes Saat- und Pflanzengut kann später in der Landwirtschaft, im Gartenbau und in der Forstwirtschaft mit unterschiedlichen Chemikalien in Berührung kommen.

Erfindungsgemässe Mittel können gegebenenfalls zusätzlich jene Agrarchemikalien enthalten, vor deren Einfluss die Kulturpflanze geschützt werden soll.

Als Kulturpflanzen gelten im Rahmen vorliegender Erfindung alle Pflanzen, die in irgendeiner Form Ertragsstoffe, wie Samen, Wurzeln, Stengel, Knollen, Blätter, Blüten, ferner Inhaltsstoffe, wie Oele, Zucker, Stärke, Eiweiss usw., produzieren und zu diesem Zweck angebaut werden. Zu diesen Pflanzen gehören beispielsweise sämtliche Getreidearten, wie Weizen, Roggen, Gerste und Hafer, daneben vor allem Reis, Kulturhirse, Mais, Baumwolle, Zuckerrüben, Zuckerrohr, Soja, Bohnen und Erbsen.

Das Gegenmittel kann überall dort eingesetzt werden, wo eine Kulturpflanze der vorgenannten Art vor der phytotoxischen Wirkung des N-(2-Methoxycarbonylphenylsulfonyl)-N'-(4,6-bis-difluoromethoxy-pyrimidin-2-yl-harnstoffes der Formel I geschützt werden soll.

Als Antogonisten oder Gegenmittel werden erfindungsgemäss Dichloracetamide entsprechend der Formel II verwendet

$$R_a-\underset{\displaystyle R_b}{\underset{|}{N}}-COCHCl_2 \qquad (II) \quad .$$

In dieser Formel bedeuten $R_a$ und $R_b$ unabhängig voneinander je Wasserstoff, einen Alkyl-, Alkenyl- , einen Cycloalkyl-, Cycloalkenyl-Rest welcher unsubstituiert oder durch Halogen, Cyano; Hydroxy; Alkylcarbonyloxy; Alkenylcarbonyloxy; Alkinylcarbonyloxy; Alkoxyalkylcarbonyloxy; Alkylcarbonylthio; Alkoxycarbonyloxy; Formyloxy; Benzoyloxy; subst. Benzoyloxy; Haloalkylcarboxy; Alkyloxy; Alkenyloxy; Alkinyloxy; Phenyloxy; subst. Phenyloxy; Heterocyclyloxy- und Heterocyclylthio; subst. Heterocyclyloxy- und Heterocyclylthio; Alkylthio; Alkenylthio; Phenylthio; subst. Phenylthio; Alkoxyalkoxy; Alkoxyalkoxyalkoxy; Alkenyloxyalkoxy; Tetraalkoxy; Alkylthioalkoxy; Carbamoyloxy; N-mono- und di-Alkylcarbamoyloxy; N-Cycloalkyl- und N,N-Alkylencarabamoyloxy; Carbamoylthio; Heterocyclyl- und substituiertes Heterocyclylaminocarbonyloxy; N-mono- und di-Alkylcarbamoylthio; Thiocarbamoylthio; N-mono- und di-Alkylthiocarbamoylthio; N-Cycloalkyl- und N,N-Alkylenthiocarbamoylthio; Alkyl- und Alkenylsulfinyl; Alkyl- und Alkenylsulfonyl; Alkyl- und Alkenylsulfonyloxy; Phenyl- und substituiertes Phenylsulfonyloxy; Phenylsulfinyl und -sulfonyl; substituiertes Phenylsulfinyl und -sulfonyl; Heterocyclyl- und substituiertes Heterocyclylsulfonyl; Sulfamoyl; N-Alkyl-, -N-Cycloalkyl-, N-Phenyl-, substituiertes N-Phenylsulfamoyl, Heterocyclyl- und substituiertes Heterocyclylsulfamoyl; N-Alkenyl-, N,N-di-Alkyl-, N,N-di-Alkenyl, N,N-Alkyl, Alkenyl-Sulfamoyl; Aminooxy; Mono- und di-Alkylaminooxy; Alkylcarbonylaminooxy; Phenyl-und substituiertes Phenylcarbonylaminooxy; Alkanaliminooxy; Benzaldehyd- und substituiertes Benzaldehydiminooxy; Dialkylketoniminooxy; Phenylalkylketoniminooxy; Dialkylphosphoryl; Phenyl-, Alkyl-Phosphoryl; Amino; Anilino; substituiertes Anilino; Heterocyclylamino- und substituiertes Heterocyclylamino; Mono- und di-Alkylamino; Alkenylamino; Alkinylamino; di-Alkenylamino; di-Alkinylamino; N,N-Alkyl, Alkenylamino; N,N,N-trialkylammonium; Alkylamido; Alkenylamido; Alkinylamido; Alkyl-N-alkylamido; Benz- und substituiertes Benzamido; Phenylalkylamido; Phenylalkyl-N-alkylamido; Benz- und substituiertes Benz-N-Alkylamido; Heterocyclylamido- und substituiertes Heterocyclylamido; Haloalkyl und Alkoxyalkylamido; Haloalkyl und Alkoxy-N-alkylamido; Alkoxycarbonylamino; Alkoxycarbonyl-N-alkylamino; Alkenyloxycarbonylamino- und N-Alkyl-amino; Hydroxy- und Alkoxyamino; Hydroxy-und Alkoxy-N-Alkylamino; Alkenyloxyamino; Phenyl- und Alkylaminocarbonylamino; N-Phenyl-N-Alkylaminocarbonylamino; N,N-Alkylenaminocarbonylamino; N-Phenyl-N-Alkylaminocarbonyl-N-alkylamino; N-Phenylaminocarbonyl-N'-alkylamino; N,N'-di- und tri-Alkylaminocarbonylamino; Heterocyclyl- und substituiertes Heterocyclylaminocarbonylamino; N-Heterocyclyl- und substituiertes Heterocylyl-N,N'-mono- und di-alkylaminocarbonylamino; Alkyl- und Alkenylsulfonylamino; Alkyl- und Alkenylsulfonyl-N-alkylamino; Phenyl- und substituiertes Phenylsulfonylamino; Phenyl- und substituiertes Phenylsulfonyl-N-alkylamino; Heterocyclyl- und substituiertes Heterocyclylsulfonylamino; Alkanal-imino; Benzaldehyd- und substituiertes Benzaldehydimino; Phenyl- und substituiertes Phenylalkylketonimino; Dialkylketonimino; Guanidino; N,N',N''-mono-di-tri-und tetra-Alkylguanidino; Amidino; N,N'-mono-und Dialkylamidino, Phenyl- und substituiertes Phenylamidino; Phenyl- und substituiertes Phenyl-N,N'-mono-und di-alkylamidino; Di-alkylphosphonyl; Alkyl-o-alkylphosphinyl; Alkyl- und Alkenyloxycarbonyl; Phenylalkyloxycarbonyl; Alkyl- und Alkenylthiocarbonyl; Alkoxyalkoxycarbonyl; Haloalkyloxycarbonyl; Aminocarbonyl; mono- und di-Alkylaminocarbonyl; mono und di-Alkenylaminocarbonyl; mono- und di-Alkinylaminocarbonyl; Phenyl-und substituiertes Phenylalkylaminocarbonyl; Anilino- und substituiertes Anilinocarbonyl; N-Phenyl-N-alkylaminocarbonyl; N,N-Alkylenaminocarbonyl; Cycloalkylamino- und cycloalkyl-N-alkylaminocarbonyl; Hydroxy- und Alkoxyaminocarbonyl; Haloalkyl-und Alkoxyalkylaminocarbonyl; Formyl; Alkyl- und Alkenyl-und Alkinylcarbonyl; Phenyl- und substituiertes Phenylalkylcarbonyl; Phenyl- und substituiertes Phenylalkenylcarbonyl; Benzoyl; substituiertes Benzoyl; Heterocyclylcarbonyl; substituiertes Heterocyclylcarbonyl; Phenyl; Naphthyl; substituiertes Phenyl- und Naphthyl; Heterocyclische Reste; substituierte heterocyclische Reste substituiert sein kann. Weiter bedeuten $R_a$ und $R_b$ unabhängig voneinander Alkinyl; durch Phenyl, substituiertes Phenyl, Halogenalkyl, Alkylthio- und Alkenylthioalkyl, Phenylthio- und substituiertes Phenylthioalkyl, Alkoxy- und Phenoxyalkyl, Alkoxycarbonyl, Aminocarbonyl, mono- und di-Alkylaminocarbonyl, Anilino- und substituiertes Anilinocarbonyl, N-Anilino- und substituiertes Anilino-N-Alkylaminocarbonyl, N,N-Alkylenaminocarbonyl, Heterocyclyl- und substituiertes Heterocyclylaminocarbonyl, Aminoalkyl, mono- und di-Alkyl- und Alkenylaminoalkyl, N,N-Alkylenaminoalkyl, Anilino-und substituiertes Anilinoalkyl, Phenyl- und substituiertes Phenylalkylaminoalkyl, Heterocyclylamino- und substituiertes Heterocyclylaminoalkyl, Heterocyclyl- und substituiertes Heterocyclylaminoalkylaminoalkyl, substituierter Alkinylrest. Phenyl- und Naphthylreste, heterocyclische Reste und benzannellierte Heterocyclen können ihrerseits ein- oder mehrfach durch Halogen; Nitro; Cyano; Pseudohalogen; Alkyl- und Alkenyloxy; Alkyl-und Alkenylthio; Alkyl; Alkenyl; Alkinyl; Phenylalkinyl; Alkylsulfinyl und -sulfonyl; Hydroxycarbonyl; Alkoxy- und Benzyloxycarbonyl; Alkenyloxycarbonyl; Alkylendioxy; Aminocarbonyl; mono-und di-Alkylaminocarbonyl; Amino; Ammonio; mono- und di-Alkylamino; Alkan- und Alkencarbonyl; Phenyloxy; durch Halogen, Halogenalkyl, Nitro, Alkoxy, Alkylthio, Alkyl, substituiertes Phenyloxy; Sulfo; Di-Alkylphosphoryl- und phosphonyl; Sulfamoyl; mono- und di-Alkyl-sulfamoyl; mono- und di-und tri-Halogenalkyl; Alkylsulfinyl- und -sulfonyl; substituiert sein.

Einer der Reste $R_a$ und $R_b$ kann auch Amino; mono- oder di-Alkyl und Alkenylamino; mono- oder di-Alkinylamino; substituiertes oder unsubstituiertes Phenylalkylamino; substituiertes oder unsubstituiertes N-Phenylalkyl-N-alkylamino; substituiertes oder unsubstituiertes Heterocyclylamino; substituiertes oder unsubstituiertes N-Heterocyclyl-N'-Alkylamino; Alkanalimino, substituiertes oder unsubstituierte Benzaldehydimino, Dialkylketonimino, substituiertes oder unsubstituiertes Phenylalkylketonimino; Alkyloxycarbonylamino- und N-Alkylamino; substituiertes oder unsubstituiertes Phenylalkoxycarbonylamino- und N-Alkylamino; durch 1-3-Alkyl substituiertes Ureido; durch 1-2 Alkyl substituiertes Phenylureido; durch 1-2 Alkyl substituiertes Heterocyclylureido; Alkyl-, Alkenyl- und Alkinylamido und N-Alkylamido; substituiertes und unsubstituiertes Phenylalkylamido; substituiertes und unsubstituiertes Phenyl- und Heterocyclylamido; substituiertes und unsubstituiertes Phenyl- und Heterocyclyl-N-Alkylamido; Alkylkoxy- und Alkylthiothionoamino bedeuten, wobei die Alkylreste wie oben angegeben, substituiert sein können.

Unter Alkylresten werden Reste mit 1 bis 18 Kohlenstoffatomen verstanden. Diese Reste können geradkettig oder verzweigt sein. Die gebräuchlichsten Reste sind beispielsweise Methyl, Aethyl, n-Propyl, Isopropyl, n-Butyl, Isobutyl, sek.Butyl, tert.Butyl, n-Pentyl, Isopentyl, n-Hexyl und n-Octyl. Die Alkenyl- und Alkinylreste können ebenfalls geradkettig oder verzweigt sein und umfassen 3 bis 18 Kohlenstoffatome. Die am verbreitesten Reste sind beispielsweise Allyl, Methallyl, Buten, Butadien, Propinyl, Methylpropinyl, 1-Butinyl, 2-Butinyl. Cycloalkyl- oder Cycloalkenylreste haben vorzugsweise 3 - 12 Kohlenstoffatome, sie können auch benzanneliert sein. Typische Vertreter sind beispielsweise Cyclopropyl, Cyclopentyl, Cyclohexyl, Cyclohexenyl, Indan, Tetrahydronaphthalin, Decalin. Unter Halogen wird Fluor, Chlor, Brom, Jod, insbesondere Fluor und Chlor verstanden. Halogenalkyl- und Halogenalkenyl-Reste können ein- oder mehrfach mit Halogen substituiert sein.

Die obgenannten alicyclischen und cyclischen Kohlenwasserstoffreste können unsubstituiert oder substituiert sein. Typische Substituenten dieser Reste sind beispielsweise die Halogenatome, über Sauerstoff, Schwefel oder eine Iminogruppe gebundene Alkyl-, Alkenyl-, Alkinyl-, oder Cycloalkyl-, Aryl- oder Aralkylreste die ihrerseits wieder

substituiert sein können. Diese Substituenten können aber auch über eine Sulfinyl-, Sulfonyl-, Carbonyl-, Carbonyloxy, Carbonylthio, Carbamoyl-, Sulfamoyl- oder eine Amino-oxybrücke an die alicyclischen Kohlenwasserstoffreste gebunden sein.

Die Reste $R_a$ und $R_b$ können zusammen mit dem Stickstoffatom an das sie gebunden sind auch einen gesättigten oder gesättigten 5 bis 12-gliedrigen Heterocyclus bilden, der noch ein zwei oder drei weitere Heteroatome oder eine Sulfinyl- resp. Sulfonylgruppe enthält, durch ein oder zwei Carbonylgruppen unterbrochen sein kann, und welcher benzannelliert, unsubstituiert oder substituiert sein kann.

Als Heteroatome kommen dabei ein zwei oder drei weitere Stickstoffatome, bis zwei Schwefel- oder Sauerstoffatome in Frage, wobei 2 Sauerstoffatome nicht direkt benachbart sein können

Beispiele für solche Heterocyclen, sind untenstehend aufgeführt: Pyrrolin, Pyrrolidin, Imidazolin, Imidazolidin, Pyrazolin, Pyrazolidin, Isocazolin, Isocazolidin, Oxazolin, Oxazolidin, Isothiazolidin, Thiazoline, Thiazolidine, Dithiazolidine, Oxadiazolidine, Piperidin, Piperazin, Tetrahydropyrimidin und -pyrazin, Morpholin, Thiomorpholin, Thiazine, Hexahydrotriazine, Tetrahydrotriazine, Oxadiazine, Oxatriazine, Hexahydroazepin, Hexahydrodiazepine, Diazepine, Hexahydrooxazepine, Azacyclooctan, Indolin, Isoindolin, Benzimidazolin, Benzindazolin, Benzoxazolin, Benzthiazoline, Benzisoxazolin, Benztriazol, Tetrahydrochinolin, Tetrahydroisochinolin, Tetrahydro-chinolin, -chinazolin, -chinoxalin, -phtalazin, Benzmorpholin, Benzothiomorpholin, Tetrahydrobenzazepine und -diazepine, Tetrahydrobenzoxazepine, 1,5-diazabicyclo[4,3,0]-nonane, Dihydrobenzoxazine, 1,6-diazabicyclo[5,3,0]decane, 1,4-diazabicyclo[3,3,0]octane, 1,5-diazabicyclo[4,4,0]decane.

Oben erwähnte Heterocyclen können auch die Bedeutung von Substituenten haben. Weitere Beispiele von heterocyclischen Systemen mit Substituentenfunktion sind beispielsweise Pyrrol, Imidazol, Pyrazol, Isoxazol, Oxazol, Isothiazol, Thiazol, Triazole, Oxadiazole, Thiadiazole, Tetrazole, Oxatriazole, Thiatriazole, Furan, Tetrahydrofuran, Dioxole, Dioxolane, Oxathiole, Oxathiolane, Thiophen, Tetrahydrothiophen, Dithiolane, Dithiazole, Pyridin, Pyrane, Thiopyrane, Pyridazin, Pyrimidin, Pyrazin, Tetrahydropyran, Tetrahydrothiopyran, Dioxine, Dioxane, Dithiine, Dithiane, Oxazine, Thiazine, Oxathiine, Oxathiane, Triazine, Oxadiazine, Thiadiazine, Oxathiazine, Dioxazine, Azepine, Oxepine, Thiepine, Diazepine, Oxazepine, Indole, Benzofurane, Benzothiophene, Indazole, Benzimidazole, Benzdioxole, Benzdithiole, Benzisoxazole, Benzthiazole, Benzoxazole, Benzoxathiole, Benztriazole, Benzoxadiazole, Benzofurazan, Benzothiadiazole, Chinolin, Isochinolin, Chromene, Chroman, Isochromen, Isochroman, Thiochromene, Isothiochromene, Thiochroman, Isothiochroman, Cinnolin, Chinazolin, Chinoxalin, Phtalazin, Benzdioxine, Benzdithiine, Benzoxazine, Benzdioxane, Benzoxathiane, Benzotriazine, Benzazepine, Benzdilazepine, Benzoxazepine, Purine, Pteridine, Phenoxazine, Phenothiazine.

Die heterocyclischen Reste können wie oben erwähnt substituiert sein.

Die Verbindungen der Formel II sind grösstenteils aus der Literatur bekannt oder können nach bekannten Methoden hergestellt werden.

Die Dichloramide der Formel II werden z.B. hergestellt, indem man ein Dichloressigsäurehalid, z.B. das Chlorid oder Bromid in einem inerten organischen Lösungsmittel, mit einem Amin der Formel $HNR_a,R_b$ umsetzt entsprechend dem Reaktionsschema

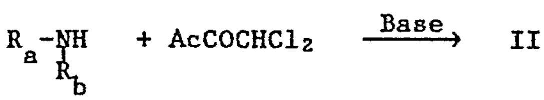

Ac steht für ein Halogenatom oder einen Rest $-OCOCHCl_2$.

Die Reaktion wird zweckmässigerweise in einem reaktionsinerten Lösungsmittel bei Normaldruck durchgeführt. Als Lösungsmittel eignen sich beispielsweise aliphatische oder aromatische Kohlenwasserstoffe wie Benzol, Toluol, Xylole, Cyclohexan, Petroläther; halogenierte Kohlenwasserstoffe wie Chlorbenzol, Methylenchlorid, Aethylenchlorid, Chloroform; Aether und ätherartige Verbindungen wie Diäthyläther, Diisopropyläther, t-Butylmethyläther, Dimethoxyäthan, Dioxan, Tetrahydrofuran, Anisol; Ketone wie Aceton, Methyläthylketon; Ester wie Aethylacetat, Butylacetat und Gemische solcher Lösungsmittel untereinander.

Als säurebindende Mittel sind Basen, insbesondere tertiäre Amine wie Trimethylamin, Triäthylamin, Chinuclidin, 1,4-Diazabicyclo-(2,2,2)-octan, 1,5-Diazabicyclo(4,3,0)non-5-en oder 1,5-Diazabicyclo(5,4,0)-undec-7-en geeignet. Es können aber auch anorganische Basen wie Hydride wie Natrium- oder Calciumhydrid, Hydroxide wie Natrium- und Kaliumhydroxid, Carbonate wie Natrium-und Kaliumcarbonat oder Hydrogencarbonate wie Kalium- oder Natriumhydrogencarbonat verwendet werden.

Die Dichloracetamide der Formel II sind grösstenteils bekannt und in vielen Publikationen beschrieben. Sie können nach bekannten Methoden hergestellt und entsprechend ihrer chemischen Struktur verschiedenen Untergruppen zugeordnet werden:

Eine erste Gruppe wird durch die Verbindungen der Formel II verkörpert,

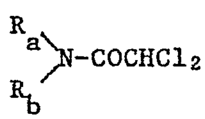

worin $R_a$ Wasserstoff, $C_1$-$C_8$-Alkyl oder $C_3$-$C_8$-Cycloalkyl, unsubstituiert oder durch $-PO(R_1)(R_2)$ , $-NR_3R_4$, $-(O)_mCOOR_5$, $-(X')_mCX''R_6$, $-O(AO)_{m'}R_7$, $-XR_7$, Cyano-, X"'-Het, Het, $C_5$-$C_6$-Cycloalkyl, $CR(OR_8)(OR_9)$ oder Halogen substsituiert, oder $R_a$ ist ein $C_3$-$C_8$-Alkenyl- oder $C_3$-$C_8$-Cycloalkenylrest, der unsubstituiert oder durch Halogen, Phenyl, $C_5$-$C_6$-Cycloalkyl oder $C_1$-$C_4$-Alkyl substituiert ist, oder $R_a$ ist ein $C_3$-$C_8$-Alkinylrest, der unsubstituiert oder durch Phenyl substituiert ist, wobei die Phenylkerne unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sind, oder $R_a$ ist ein Allkoxy-iminoalkylrest $-CH(R_r)-C(R_s)=NOR_t$, $R_r$ und $R_s$ je Wasserstoff oder $C_1$-$C_4$-Alkyl und $R_t$ Wasserstoff $C_1$-$C_6$-Alkyl, $C_3$-$C_6$-Alkenyl oder $C_3$-$C_6$-Alkinyl,

$R_b$ Wasserstoff, dasselbe wie $R_a$ oder einen Rest $-NR_3R_4$,

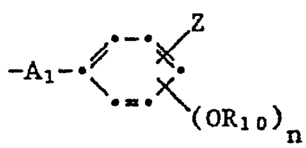

oder $-(\overset{R_{11}}{CH})_n-Het$

A eine $C_1$-$C_4$-Alkylenkette,

$A_1$ eine $C_1$-$C_8$-Alkylenkette die geradkettig oder verzweigt ist und die unsubstsituiert oder durch Cyan oder $C_1$-$C_4$-Alkoxy oder einen Alkylamido- oder Halogenalkylamido-Rest substituiert ist,

Het einen gegebenenfalls durch $C_1$-$C_4$-Alkyl, $C_1$-$C_4$-Alkoxy, $C_1$-$C_4$-Alkoxycarbonyl oder $C_1$-$C_4$-Alkylthio optimal substituierten 5-6-gliedrigen Heterocyclus mit 1-3 Heteroatomen resp. $S(O)_n$, Gruppen, wobei Sauerstoff und Schwefel nie direkt benachbart im Ring vorhanden sein können, sondern in einer 1.3-Anwendung vorliegen müssen, wie im 1,3-Dioxolan-2-yl-, 1,3-Dioxan-2-yl oder dem 2,4-Dioxan-1-ylrest und welcher zusätzlich durch $R_n$ in der 1-Stellung und $R'_n$ an einem Ring Kohlenstoffatom substituiert sein kann,

n Null oder eine Zahl von 1 bis 3,

m Null oder Zahl 1,

m' Null oder 1-4,

n' Null oder 1-2,

R, R', $R_1$ und $R_2$ unabhängig voneinander je $C_1$-$C_4$-Alkyl oder $C_1$-$C_4$-Alkoxy,

$R_3$ Wasserstoff, $C_1$-$C_4$-Alkyl oder $C_3$-$C_8$-Cycloalkyl,

$R_4$ Wasserstoff, $C_1$-$C_4$-Alkyl, Phenyl, durch Halogen, $C_1$-$C_4$-Alkyl, $C_1$-$C_4$-Alkoxy, $C_1$-$C_4$-Halogenalkyl oder $C_1$-$C_4$Alkoxy substituiertes Phenyl oder einen Rest -COOR'$_5$ oder -COR'$_6$,

$R_5$ und R'$_5$ unabhängig voneinander je $C_1$-$C_4$-Alkyl, oder $C_1$-$C_4$-Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist,

$R_6$ und R'$_6$ unabhängig voneinander je Wasserstoff, $C_1$-$C_4$-Alkyl, $C_1$-$C_4$-Halogenalkyl, $C_2$-$C_5$-Alkoxyalkyl, Phenyl oder $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist, oder $R_6$ und R'$_6$ bedeuten einen $C_2$-$C_4$-Alkenyl- oder einen Rest -N($R_{12}$)($R_{13}$),

$R_7$ Wasserstoff, $C_1$-$C_4$-Alkyl, $C_3$-$C_6$-Alkenyl, $C_3$-$C_6$-Alkinyl, Phenyl und Phenyl($C_1$-$C_4$)Alkyl, wobei die Phenylkerne durch Halogen, $C_1$-$C_4$Alkyl, $C_1$-$C_4$Alkoxy oder $C_1$-$C_4$Halogenalkyl substituiert sind,

$R_8$ und $R_9$ unabhängig voneinander je $C_1$-$C_6$-Alkyl oder $C_3$-$C_6$-Alkenyl

$R_{10}$ $C_1$-$C_4$-Alkyl, $C_3$-$C_6$-Alkenyl, $C_2$-$C_6$-Alkoxyalkyl, $C_1$-$C_4$-Halogenalkenyl oder in Zusammenhang mit 2 Sauerstoffatomen auch eine $C_1$-$C_5$-Alkylenbrücke,

$R_{11}$ Wasserstoff oder $C_1$-$C_4$-Alkyl und

$R_{12}$ und $R_{13}$ unabhängig voneinander je Wasserstoff, $C_1$-$C_8$Alkyl, $C_3$-$C_8$-Cycloalkyl, $C_3$-$C_8$-Alkenyl, Phenyl oder $C_1$-$C_4$Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyano, Nitro und

$R_{12}$ und $R_{13}$ zusammen eine $C_1$-$C_7$-Alkylenkette, die durch Sauerstoff, Schwefel, >NH, oder >N($C_1$-$C_4$)-Alkyl unterbrochen sein kann.

Z Wasserstoff, $C_1$-$C_4$-Alkyl, Halogen, $C_1$-$C_4$-Halogenalkyl, Nitro, $C_1$-$C_4$-Alkoxy, $C_1$-$C_4$-Halogenalkoxy, $C_2$-$C_8$-Alkoxyalkoxy, der Dioxymethylenrest, ein 1.3-Dioxolan-2-yl- oder 1,3-Dioxan-2-ylrest,

der über eine $C_1$-$C_4$-Alkylenbrücke gebunden sein kann bedeuten.

X', X", X"' Sauerstoff, Schwefel, X Sauerstoff, Schwefel $-S(O)_n$,

Solche Verbindungen sind zum Teil in den veröffentlichten Patentanmeldungen resp. den Patentschriften BE-A 884,911, DE-A 2,747,814, DE-A 2,855,229, EP-A 31 686, HU-A T-26,239A, USP 3,923,494, 4,021,224, 4,396,419, 4,400,203, 4,443,628 sowie der schweizerischen Patentanmeldung 4,202/84-0 beschrieben. Folgende Dichloracetamide eignen sich erfindungsgemäss als Antidote zum Sulfonylharnstoff der Formel I besonders gut.

Tabelle 1

| Nr. | $R_a$ | $R_b$ |
|---|---|---|
| 1.1 | $-CH_2CH=CH_2$ | $-CH_2CH=CH_2$ |
| 1.2 | $-CH_2CH=CH_2$ | $-C_2H_5$ |
| 1.3 | $-CH_2CH=CH_2$ | $-C_2H_4CN$ |
| 1.4 | $-CH_2CH=CH_2$ | H |
| 1.5 | $-C_3H_7n$ | $-CH(CH_3)CN$ |
| 1.6 | $-CH_2PO(OC_2H_5)_2$ | H |
| 1.7 | $-C(CH_3)_3$ | H |
| 1.8 | Cyclopropyl | H |
| 1.9 | $-CH_2CH=CH_2$ | $-CH_2C(CH_3)=CH_2$ |
| 1.10 | $-CH_2CH=CH_2$ | $-CH_2C\equiv CH$ |
| 1.11 | $-CH_2CH=CH_2$ | $-CH_2CCl=CH_2$ |
| 1.12 | $-C_2H_5$ | $-CH_2CCl=CH_2$ |
| 1.13 | $-CH_2CH(CH_3)_2$ | $-C_2H_4OCH_2CH=CH_2$ |
| 1.14 | Cyclopropyl | $-CH_2-CH=CH_2$ |
| 1.15 | $-CH_2CH=CH_2$ | $-CH(CH_3)CN$ |
| 1.16 | $-CH_2CCl=CH_2$ | $-CH_2CCl=CH_2$ |
| 1.17 | $-CH_2CCl=CH_2$ | H |
| 1.18 | $-CH_2CH=CHCl$ | $-CH_2-CH=CHCl$ |
| 1.19 | $-CH_2CH(C_2H_5)C_4H_9n$ | $-CH(CH_3)CH_2OCH_3$ |
| 1.20 | $-CH_2CH=CHCl$ | H |
| 1.21 | $-C_3H_7n$ | $C(CN)(CH_3)_2$ |
| 1.22 | $-CH(CH_3)_2$ | $-CH_2COOC_2H_5$ |
| 1.23 | $-CH_2CH=CH_2$ | $-CH_2-CON(CH_2CH=CH_2)_2$ |
| 1.24 | $-C_2H_5$ | $-CH_2COOC_3H_7n$ |
| 1.25 | $-CH_2N(CH_3)COCCl_2CH_3$ | H |

Tabelle 1 (Fortsetzung)

| Nr. | $R_a$ | $R_b$ |
|---|---|---|
| 1.26 | $-(CH_2)_3NHCOCHCl_2$ | H |
| 1.27 | $-CH_2CH{=}CH_2$ | $-CH_2PO(OC_3H_7iso)_2$ |
| 1.28 | -NHCOOBenzyl | $-CH_2PO(OC_2H_5)_2$ |
| 1.29 | $-CH(CH_3)_2$ | $-CH(CH_3)_2$ |
| 1.30 | $-C_4H_9n$ | $-C_2H_4CN$ |
| 1.31 | $-CH(CH_3)_2$ | $-C_2H_4CN$ |
| 1.32 | $-CH_3$ | $-C_2H_4CN$ |
| 1.33 | $-CH_2CH{=}CH_2$ | $-CH(CH_3)CH_2OCH_3$ |
| 1.34 | -NHCOO Benzyl | $-CH_2PO(CH_3)OCH(CH_3)_2$ |
| 1.35 | -NHCOO Benzyl | $-CH_2PO(C_2H_5)OCH(CH_3)_2$ |
| 1.36 | $-NHCOOC_2H_5$ | $-CH_2PO(OC_3H_7iso)_2$ |
| 1.37 | $-CH_2CH{=}CH_2$ | $-CH_2OC_2H_4OC_2H_5$ |
| 1.38 | $-NHCOOC_2H_5$ | $-CH_2PO(CH_3)OCH_2CH(CH_3)_2$ |
| 1.39 | $-CH_2CH{=}CH_2$ | $-CH_2CONHCH_2CH{=}CH_2$ |
| 1.40 | $-NHCOOC_2H_5$ | $-CH_2PO(OC_2H_5)_2$ |
| 1.41 | $-CH_2CH{=}CH_2$ | $-CH_2OC_2H_4OCH_3$ |
| 1.42 | $-CH_2CH{=}CH_2$ | $-(C_2H_4O)_2-C_4H_9n$ |
| 1.43 | $-CH_2C{\equiv}CH$ | $-(C_2H_4O)_3-CH_3$ |
| 1.44 | $-CH_2CH{=}CH_2$ | $-(C_2H_4O)_2CH_2CH{=}CH_2$ |
| 1.45 | $-CH_2CH{=}CH_2$ | $-(C_2H_4O)_3CH_2CH{=}CH_2$ |
| 1.46 | -NHCOO Benzyl | $-CH_2PO(CH_3)OC_2H_5$ |
| 1.47 | $-CH_2CH{=}CH_2$ | $-CH_2CH(COCH_3)_2$ |
| 1.48 | $-CH_3$ | -CHO |
| 1.49 | $-(CH_2)_8NHCOCHCl_2$ | H |
| 1.50 | $-(CH_2)_6NHCOCHCl_2$ | H |

Tabelle 1 (Fortsetzung)

| Nr. | $R_a$ | $R_b$ |
|---|---|---|
| 1.51 | H | H |
| 1.52 | $-CH_2CH=CHCH_2N(C_3H_7n)$<br>\|<br>$COCHCl_2$ | $-C_3H_7n$ |
| 1.53 | $-CH(OH)Cl_3$ | H |
| 1.54 | $-CH(CH_3)NHCOCHCl_2$ | H |
| 1.55 | $-CH_3$ | $-CH_2CN$ |
| 1.56 | $-C(CH_3)=C(CH_3)_2$ | $-C_2H_4OC_2H_5$ |
| 1.57 | $-C_2H_4OCH_3$ | H |
| 1.58 | $-C_2H_4OCH(CH_3)_2$ | $-C_2H_4OCH(CH_3)_2$ |
| 1.59 | $-CH_2CH=CH_2$ | O<br>‖<br>$-CH_2-NH-C-CCl_3$ |
| 1.60 | $-C_2H_5$ | $-CH(CH_3)COOCH_3$ |
| 1.61 | $-C_3H_6OCH_3$ | $-CH_2NHCOCH_2Cl$ |
| 1.62 | $-CH_3$ | $-CH_2NHCOCHCl_2$ |
| 1.63 | $-CH_2N(CH_3)COCH_2Cl$ | H |
| 1.64 | $-CH_3$ | $-C_3H_6NHCOCHCl_2$ |
| 1.65 | $-CH_3$ | $-C_2H_4-N(CH_3)COCHCl_2$ |
| 1.66 | $-C_2H_5$ | $-CH_2NHCOCH_2Cl$ |
| 1.67 | $-NHCOOC_4H_9t.$ | $-CH_2PO(OC_2H_5)_2$ |
| 1.68 | $-C(COOCH_3)=C(CH_3)_2$ | H |
| 1.69 | $-(C_2H_4O)_3CH_3$ | H |
| 1.70 | $-CH_3$ | $-(C_2H_4O)_3CH_3$ |
| 1.71 | $-NHCOCHCl_2$ | $-CH_2PO(CH_3)OC_3H_7iso$ |
| 1.72 | 3,5,5-Trimethyl-<br>cyclohex-1-en-yl | $-CH_2CH=CH_2$ |
| 1.73 | 1-Cyclohexylvinyl | $-C_4H_9n$ |

Tabelle 1 (Fortsetzung)

| Nr. | $R_a$ | $R_b$ |
|---|---|---|
| 1.74 | $C_2H_5$ | $-CH_2CN$ |
| 1.75 | $C_3H_7i$ | $-NHCOCH_2Cl$ |
| 1.76 | $C_3H_7i$ | $-CH_2CH_2-NHCOCHCl_2$ |
| 1.77 | $C_2H_5$ | $-CH_2CH_2-NHCOCHCl_2$ |
| 1.78 | 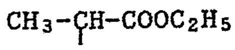 | H |
| 1.79 | H | $-(C_2H_4O)_2C_4H_9-n$ |
| 1.80 | $NHCOOC_2H_5$ | $-CH_2PO(CH_3)OC_2H_5$ |
| 1.81 | $NHCOCH_2CH_2-$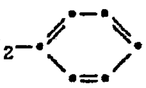 | $-CH_2-PO(OC_2H_5)_2$ |
| 1.82 | $NHCOOC_2H_5$ | $-CH_2PO(OC_2H_5)_2$ |
| 1.83 | $NHCOCH_2CH_2-$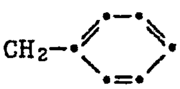 | H |
| 1.84 | $NHCOOC_2H_5$ | $-CH(C_2H_5)PO(C_2H_5)(OC_2H_5)$ |
| 1.85 | $NHCOOC_2H_5$ | $-CH($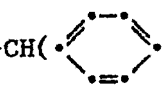$)PO(OC_2H_5)_2$ |
| 1.86 | $NHCOCH_2H_4-$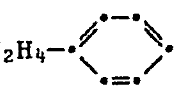 | H |
| 1.87 | $NHCOCH_2-$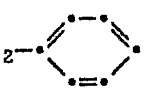 | $-CH_2PO(OC_3H_7-i)_2$ |
| 1.88 | $NH_2$ | $-CH_2PO(OC_2H_5)_2$ |
| 1.89 | H | $-CH_2COOC_2H_5$ |
| 1.90 | H | $-CH_2CONHCH_3$ |
| 1.91 | H | 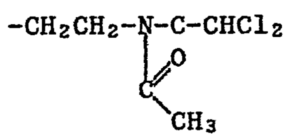 |
| 1.92 | H | 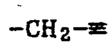 |
| 1.93 | H | 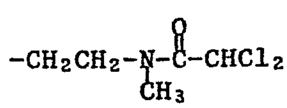 |
| 1.94 | $HC\equiv CCH_2-$ | $HC\equiv CCH_2-$ |

Tabelle 1 (Fortsetzung)

| Nr. | $R_a$ | $R_b$ |
|---|---|---|
| 1.95 | $CH_3$ | 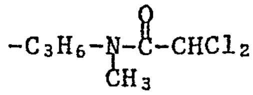 |
| 1.96 | 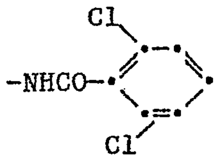 | $-CH_2PO(OC_2H_5)_2$ |
| 1.97 | 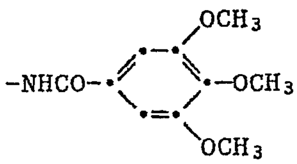 | $-CH_2-PO(OC_2H_5)_2$ |
| 1.98 | $-C_2H_5$ | 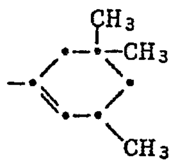 |
| 1.99 | $-CH_2-CH=CH_2$ | 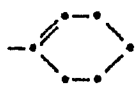 |
| 1.100 | $-CH_2-CH=CH_2$ | 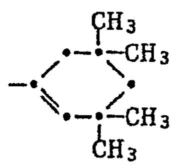 |
| 1.101 | $-C_3H_7-n$ | 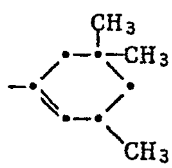 |
| 1.102 | $-NHCOO-C(CH_3CCl$ | $-CH_2PO(OC_2H_5)_2$ |
| 1.103 | $-NHCOOCH_2CCl_3$ | $-CH_2PO(OC_2H_5)_2$ |
| 1.104 | 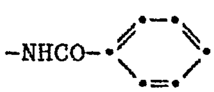 | $-CH_2PO(OC_3H_7-i)_2$ |
| 1.105 | $-CH_2-C\equiv CH$ | 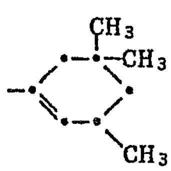 |
| 1.106 | $-CH_2-CH=CH_2$ | $-CH_2OCH_2CH=CH_2$ |

Die Fähigkeit der Verbindungen der Formel II, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 1a zusammengefasst.

Beispiel 1: Tankmischung Vorauflauf in Mais

Plastiktöpfe (oberer Durchmesser 11 cm. Inhalt 500 ccm) werden mit sandig toniger Lehmerde gefüllt. Acht Maissamen der Sorte 'LG-11' werden eingesät und mit Erde bedeckt und anschliessend angegossen. Die Testpflanzen werden im Gewächshaus kultiviert und im 2- bis 3-Blattstadium mit den Prüfsubstanzen behandelt. Die als Safener zu prüfende Substanz wird in Wasser gelöst und zusammen mit dem Herbizid der Formel I in der gegebenen Aufwandmenge auf die Pflanzen gesprüht. 21 Tage nach der Applikation wird die Schutzwirkung des Safeners in Prozent bonitiert. Als Referenz dienen dabei mit Herbizid allein behandelte Pflanzen (keine Schutzwirkung) sowie die vollständig unbehandelte Kontrolle (100 % Wachstum). Die Resultate sind in der Tabelle 1a zusammengefasst.

Tabelle 1a

| Herbizid Aufwandmenge | Antidote No. | Aufwandmenge | relative Schutzwirkung |
|---|---|---|---|
| 400 g/ha | 1.1 | 200 g/ha | 75 % |
| 400 g/ha | 1.1 | 100 g/ha | 60 % |
| 400 g/ha | 1.1 | 50 g/ha | 40 % |
| 200 g/ha | 1.1 | 200 g/ha | 60 % |
| 200 g/ha | 1.1 | 100 g/ha | 60 % |
| 200 g/ha | 1.1 | 50 g/ha | 60 % |
| 200 g/ha | 1.1 | 25 g/ha | 65 % |
| 100 g/ha | 1.1 | 100 g/ha | 30 % |
| 100 g/ha | 1.1 | 50 g/ha | 10 % |
| 100 g/ha | 1.1 | 25 g/ha | 30 % |
| 100 g/ha | 1.1 | 12,5 g/ha | 35 % |
| 400 g/ha | 1.2 | 400 g/ha | 25 % |
| 400 g/ha | 1.2 | 200 g/ha | 35 % |
| 200 g/ha | 1.2 | 200 g/ha | 10 % |
| 400 g/ha | 1.3 | 400 g/ha | 25 % |
| 400 g/ha | 1.3 | 200 g/ha | 20 % |
| 200 g/ha | 1.3 | 200 g/ha | 25 % |
| 200 g/ha | 1.3 | 100 g/ha | 20 % |
| 400 g/ha | 1.4 | 400 g/ha | 50 % |
| 400 g/ha | 1.4 | 200 g/ha | 45 % |
| 200 g/ha | 1.4 | 200 g/ha | 20 % |
| 200 g/ha | 1.4 | 100 g/ha | 35 % |
| 400 g/ha | 1.5 | 400 g/ha | 35 % |
| 400 g/ha | 1.5 | 200 g/ha | 45 % |
| 200 g/ha | 1.5 | 200 g/ha | 40 % |
| 200 g/ha | 1.5 | 100 g/ha | 25 % |
| 400 g/ha | 1.6 | 400 g/ha | 15 % |
| 400 g/ha | 1.6 | 200 g/ha | 35 % |
| 200 g/ha | 1.6 | 200 g/ha | 25 % |
| 200 g/ha | 1.6 | 100 g/ha | 20 % |
| 400 g/ha | 1.7 | 400 g/ha | 10 % |
| 400 g/ha | 1.7 | 200 g/ha | 10 % |
| 200 g/ha | 1.7 | 100 g/ha | 25 % |
| 400 g/ha | 1.8 | 400 g/ha | 30 % |
| 400 g/ha | 1.8 | 200 g/ha | 35 % |
| 200 g/ha | 1.8 | 200 g/ha | 25 % |
| 200 g/ha | 1.8 | 100 g/ha | 25 % |

Tabelle 1a (Fortsetzung)

| Herbizid Aufwandmenge | Antidote No. | Aufwandmenge | relative Schutzwirkung |
|---|---|---|---|
| 400 g/ha | 1.9 | 400 g/ha | 35 % |
| 400 g/ha | 1.9 | 200 g/ha | 40 % |
| 200 g/ha | 1.9 | 200 g/ha | 35 % |
| 200 g/ha | 1.9 | 100 g/ha | 35 % |
| 400 g/ha | 1.10 | 400 g/ha | 65 % |
| 400 g/ha | 1.10 | 200 g/ha | 50 % |
| 200 g/ha | 1.10 | 200 g/ha | 25 % |
| 200 g/ha | 1.10 | 100 g/ha | 35 % |
| 400 g/ha | 1.11 | 400 g/ha | 45 % |
| 400 g/ha | 1.11 | 200 g/ha | 45 % |
| 200 g/ha | 1.11 | 200 g/ha | 35 % |
| 200 g/ha | 1.11 | 100 g/ha | 35 % |
| 400 g/ha | 1.12 | 400 g/ha | 40 % |
| 400 g/ha | 1.12 | 200 g/ha | 30 % |
| 200 g/ha | 1.12 | 200 g/ha | 35 % |
| 200 g/ha | 1.12 | 100 g/ha | 30 % |
| 400 g/ha | 1.13 | 400 g/ha | 5 % |
| 400 g/ha | 1.13 | 200 g/ha | 10 % |
| 200 g/ha | 1.13 | 200 g/ha | 20 % |
| 200 g/ha | 1.13 | 100 g/ha | 20 % |
| 400 g/ha | 1.14 | 400 g/ha | 30 % |
| 400 g/ha | 1.14 | 200 g/ha | 30 % |
| 200 g/ha | 1.14 | 200 g/ha | 30 % |
| 200 g/ha | 1.14 | 100 g/ha | 25 % |
| 400 g/ha | 1.15 | 400 g/ha | 35 % |
| 400 g/ha | 1.15 | 200 g/ha | 45 % |
| 200 g/ha | 1.15 | 200 g/ha | 40 % |
| 200 g/ha | 1.15 | 100 g/ha | 25 % |
| 400 g/ha | 1.17 | 400 g/ha | 35 % |
| 400 g/ha | 1.17 | 200 g/ha | 30 % |
| 200 g/ha | 1.17 | 200 g/ha | 30 % |
| 200 g/ha | 1.17 | 100 g/ha | 25 % |
| 400 g/ha | 1.19 | 400 g/ha | 5 % |
| 400 g/ha | 1.19 | 200 g/ha | 10 % |
| 200 g/ha | 1.19 | 200 g/ha | 25 % |
| 200 g/ha | 1.19 | 100 g/ha | 30 % |
| 400 g/ha | 1.20 | 400 g/ha | 40 % |
| 400 g/ha | 1.20 | 200 g/ha | 45 % |
| 200 g/ha | 1.20 | 200 g/ha | 45 % |
| 200 g/ha | 1.20 | 100 g/ha | 35 % |

Tabelle 1a (Fortsetzung)

| Herbizid Aufwandmenge | Antidote No. | Aufwandmenge | relative Schutzwirkung |
|---|---|---|---|
| 400 g/ha | 1.22 | 400 g/ha | 25 % |
| 400 g/ha | 1.22 | 200 g/ha | 35 % |
| 400 g/ha | 1.23 | 400 g/ha | 10 % |
| 200 g/ha | 1.23 | 200 g/ha | 30 % |
| 200 g/ha | 1.23 | 100 g/ha | 20 % |
| 400 g/ha | 1.24 | 400 g/ha | 10 % |
| 400 g/ha | 1.24 | 200 g/ha | 5 % |
| 200 g/ha | 1.24 | 200 g/ha | 25 % |
| 200 g/ha | 1.24 | 100 g/ha | 25 % |
| 400 g/ha | 1.25 | 400 g/ha | 40 % |
| 400 g/ha | 1.25 | 200 g/ha | 30 % |
| 200 g/ha | 1.25 | 200 g/ha | 10 % |
| 200 g/ha | 1.25 | 100 g/ha | 30 % |
| 400 g/ha | 1.26 | 400 g/ha | 10 % |
| 400 g/ha | 1.26 | 200 g/ha | 40 % |
| 200 g/ha | 1.26 | 200 g/ha | 10 % |
| 200 g/ha | 1.26 | 100 g/ha | 25 % |
| 400 g/ha | 1.27 | 200 g/ha | 20 % |
| 400 g/ha | 1.28 | 400 g/ha | 45 % |
| 400 g/ha | 1.28 | 200 g/ha | 30 % |
| 200 g/ha | 1.28 | 200 g/ha | 35 % |
| 200 g/ha | 1.28 | 100 g/ha | 40 % |
| 400 g/ha | 1.29 | 400 g/ha | 5 % |
| 400 g/ha | 1.29 | 200 g/ha | 25 % |
| 200 g/ha | 1.29 | 200 g/ha | 15 % |
| 200 g/ha | 1.29 | 100 g/ha | 20 % |
| 400 g/ha | 1.30 | 400 g/ha | 40 % |
| 400 g/ha | 1.30 | 200 g/ha | 40 % |
| 200 g/ha | 1.30 | 200 g/ha | 30 % |
| 200 g/ha | 1.30 | 100 g/ha | 35 % |
| 400 g/ha | 1.31 | 400 g/ha | 30 % |
| 400 g/ha | 1.31 | 200 g/ha | 10 % |
| 200 g/ha | 1.31 | 200 g/ha | 20 % |
| 400 g/ha | 1.32 | 400 g/ha | 45 % |
| 400 g/ha | 1.32 | 200 g/ha | 30 % |

Tabelle 1a (Fortsetzung)

| Herbizid Aufwandmenge | Antidote No. | Aufwandmenge | relative Schutzwirkung |
|---|---|---|---|
| 400 g/ha | 1.33 | 400 g/ha | 35 % |
| 400 g/ha | 1.33 | 200 g/ha | 40 % |
| 400 g/ha | 1.33 | 100 g/ha | 10 % |
| 400 g/ha | 1.33 | 50 g/ha | 10 % |
| 200 g/ha | 1.33 | 200 g/ha | 15 % |
| 200 g/ha | 1.33 | 100 g/ha | 10 % |
| 200 g/ha | 1.33 | 50 g/ha | 5 % |
| 200 g/ha | 1.33 | 25 g/ha | 15 % |
| 400 g/ha | 1.34 | 400 g/ha | 20 % |
| 400 g/ha | 1.34 | 200 g/ha | 25 % |
| 200 g/ha | 1.34 | 200 g/ha | 5 % |
| 200 g/ha | 1.34 | 200 g/ha | 15 % |
| 400 g/ha | 1.35 | 400 g/ha | 40 % |
| 400 g/ha | 1.35 | 200 g/ha | 40 % |
| 400 g/ha | 1.35 | 100 g/ha | 15 % |
| 400 g/ha | 1.35 | 50 g/ha | 10 % |
| 200 g/ha | 1.35 | 200 g/ha | 10 % |
| 200 g/ha | 1.35 | 100 g/ha | 20 % |
| 200 g/ha | 1.35 | 50 g/ha | 5 % |
| 400 g/ha | 1.36 | 400 g/ha | 50 % |
| 400 g/ha | 1.36 | 200 g/ha | 70 % |
| 400 g/ha | 1.36 | 100 g/ha | 70 % |
| 400 g/ha | 1.36 | 50 g/ha | 65 % |
| 200 g/ha | 1.36 | 200 g/ha | 45 % |
| 200 g/ha | 1.36 | 100 g/ha | 45 % |
| 200 g/ha | 1.36 | 50 g/ha | 40 % |
| 200 g/ha | 1.36 | 25 g/ha | 45 % |
| 100 g/ha | 1.36 | 100 g/ha | 15 % |
| 100 g/ha | 1.36 | 50 g/ha | 15 % |
| 100 g/ha | 1.36 | 25 g/ha | 30 % |
| 100 g/ha | 1.36 | 12,5 g/ha | 30 % |
| 400 g/ha | 1.38 | 400 g/ha | 40 % |
| 400 g/ha | 1.38 | 200 g/ha | 35 % |
| 200 g/ha | 1.38 | 200 g/ha | 40 % |
| 200 g/ha | 1.38 | 100 g/ha | 50 % |
| 400 g/ha | 1.39 | 400 g/ha | 35 % |
| 400 g/ha | 1.39 | 200 g/ha | 45 % |
| 200 g/ha | 1.39 | 200 g/ha | 30 % |
| 200 g/ha | 1.39 | 100 g/ha | 40 % |

Tabelle 1a (Fortsetzung)

| Herbizid Aufwandmenge | Antidote No. | Aufwandmenge | relative Schutzwirkung |
|---|---|---|---|
| 400 g/ha | 1.40 | 400 g/ha | 35 % |
| 400 g/ha | 1.40 | 200 g/ha | 35 % |
| 200 g/ha | 1.40 | 200 g/ha | 5 % |
| 200 g/ha | 1.40 | 100 g/ha | 25 % |
| 400 g/ha | 1.41 | 400 g/ha | 45 % |
| 400 g/ha | 1.41 | 200 g/ha | 50 % |
| 200 g/ha | 1.41 | 200 g/ha | 40 % |
| 400 g/ha | 1.42 | 400 g/ha | 40 % |
| 400 g/ha | 1.42 | 200 g/ha | 30 % |
| 400 g/ha | 1.42 | 100 g/ha | 10 % |
| 400 g/ha | 1.42 | 50 g/ha | 5 % |
| 200 g/ha | 1.42 | 200 g/ha | 15 % |
| 200 g/ha | 1.42 | 100 g/ha | 15 % |
| 200 g/ha | 1.42 | 50 g/ha | 20 % |
| 200 g/ha | 1.42 | 25 g/ha | 25 % |
| 100 g/ha | 1.42 | 100 g/ha | 25 % |
| 100 g/ha | 1.42 | 50 g/ha | 15 % |
| 100 g/ha | 1.42 | 25 g/ha | 25 % |
| 100 g/ha | 1.42 | 12,5 g/ha | 30 % |
| 400 g/ha | 1.43 | 400 g/ha | 45 % |
| 400 g/ha | 1.43 | 200 g/ha | 45 % |
| 200 g/ha | 1.43 | 200 g/ha | 40 % |
| 200 g/ha | 1.43 | 100 g/ha | 45 % |
| 400 g/ha | 1.44 | 400 g/ha | 35 % |
| 400 g/ha | 1.44 | 200 g/ha | 50 % |
| 200 g/ha | 1.44 | 200 g/ha | 35 % |
| 200 g/ha | 1.44 | 100 g/ha | 55 % |
| 400 g/ha | 1.45 | 400 g/ha | 35 % |
| 400 g/ha | 1.45 | 200 g/ha | 35 % |
| 200 g/ha | 1.45 | 200 g/ha | 50 % |
| 200 g/ha | 1.45 | 100 g/ha | 60 % |
| 400 g/ha | 1.46 | 400 g/ha | 40 % |
| 400 g/ha | 1.46 | 200 g/ha | 55 % |
| 200 g/ha | 1.46 | 200 g/ha | 40 % |
| 200 g/ha | 1.46 | 100 g/ha | 65 % |
| 400 g/ha | 1.47 | 400 g/ha | 35 % |
| 400 g/ha | 1.47 | 200 g/ha | 25 % |
| 200 g/ha | 1.47 | 200 g/ha | 35 % |
| 200 g/ha | 1.47 | 100 g/ha | 25 % |

Tabelle 1a (Fortsetzung)

| Herbizid Aufwandmenge | Antidote No. | Aufwandmenge | relative Schutzwirkung |
|---|---|---|---|
| 400 g/ha | 1.50 | 400 g/ha | 5 % |
| 400 g/ha | 1.51 | 200 g/ha | 5 % |
| 200 g/ha | 1.51 | 100 g/ha | 30 % |
| 400 g/ha | 1.52 | 400 g/ha | 30 % |
| 400 g/ha | 1.52 | 200 g/ha | 35 % |
| 200 g/ha | 1.52 | 200 g/ha | 35 % |
| 200 g/ha | 1.52 | 100 g/ha | 25 % |
| 400 g/ha | 1.53 | 200 g/ha | 5 % |
| 200 g/ha | 1.53 | 200 g/ha | 15 % |
| 200 g/ha | 1.53 | 100 g/ha | 5 % |
| 400 g/ha | 1.54 | 400 g/ha | 50 % |
| 400 g/ha | 1.54 | 200 g/ha | 40 % |
| 200 g/ha | 1.54 | 200 g/ha | 35 % |
| 200 g/ha | 1.54 | 100 g/ha | 40 % |
| 400 g/ha | 1.55 | 400 g/ha | 40 % |
| 400 g/ha | 1.55 | 200 g/ha | 35 % |
| 200 g/ha | 1.55 | 200 g/ha | 15 % |
| 200 g/ha | 1.55 | 100 g/ha | 5 % |
| 400 g/ha | 1.56 | 400 g/ha | 35 % |
| 400 g/ha | 1.56 | 200 g/ha | 30 % |
| 200 g/ha | 1.56 | 200 g/ha | 25 % |
| 200 g/ha | 1.56 | 100 g/ha | 30 % |
| 400 g/ha | 1.57 | 400 g/ha | 35 % |
| 400 g/ha | 1.57 | 200 g/ha | 40 % |
| 200 g/ha | 1.57 | 200 g/ha | 10 % |
| 200 g/ha | 1.57 | 100 g/ha | 10 % |
| 400 g/ha | 1.58 | 200 g/ha | 10 % |
| 200 g/ha | 1.58 | 200 g/ha | 30 % |
| 200 g/ha | 1.58 | 100 g/ha | 10 % |
| 400 g/ha | 1.59 | 400 g/ha | 35 % |
| 400 g/ha | 1.59 | 200 g/ha | 50 % |
| 200 g/ha | 1.59 | 200 g/ha | 30 % |
| 200 g/ha | 1.59 | 100 g/ha | 25 % |
| 400 g/ha | 1.60 | 400 g/ha | 65 % |
| 400 g/ha | 1.60 | 200 g/ha | 60 % |
| 200 g/ha | 1.60 | 200 g/ha | 10 % |
| 200 g/ha | 1.60 | 100 g/ha | 15 % |

Tabelle 1a (Fortsetzung)

| Herbizid Aufwandmenge | Antidote No. | Aufwandmenge | relative Schutzwirkung |
|---|---|---|---|
| 400 g/ha | 1.62 | 400 g/ha | 70 % |
| 400 g/ha | 1.62 | 200 g/ha | 65 % |
| 200 g/ha | 1.62 | 200 g/ha | 30 % |
| 200 g/ha | 1.62 | 100 g/ha | 35 % |
| 400 g/ha | 1.63 | 400 g/ha | 15 % |
| 400 g/ha | 1.63 | 200 g/ha | 25 % |
| 200 g/ha | 1.63 | 200 g/ha | 5 % |
| 400 g/ha | 1.64 | 400 g/ha | 60 % |
| 400 g/ha | 1.64 | 200 g/ha | 55 % |
| 200 g/ha | 1.64 | 200 g/ha | 30 % |
| 200 g/ha | 1.64 | 100 g/ha | 20 % |
| 400 g/ha | 1.65 | 400 g/ha | 70 % |
| 400 g/ha | 1.65 | 200 g/ha | 60 % |
| 200 g/ha | 1.65 | 200 g/ha | 40 % |
| 200 g/ha | 1.65 | 100 g/ha | 35 % |
| 400 g/ha | 1.66 | 400 g/ha | 55 % |
| 400 g/ha | 1.66 | 200 g/ha | 40 % |
| 200 g/ha | 1.66 | 200 g/ha | 45 % |
| 200 g/ha | 1.66 | 100 g/ha | 30 % |
| 400 g/ha | 1.67 | 400 g/ha | 40 % |
| 400 g/ha | 1.67 | 200 g/ha | 10 % |
| 200 g/ha | 1.67 | 200 g/ha | 30 % |
| 200 g/ha | 1.67 | 100 g/ha | 40 % |
| 400 g/ha | 1.68 | 400 g/ha | 30 % |
| 400 g/ha | 1.68 | 200 g/ha | 40 % |
| 200 g/ha | 1.68 | 200 g/ha | 20 % |
| 400 g/ha | 1.69 | 400 g/ha | 25 % |
| 400 g/ha | 1.69 | 200 g/ha | 20 % |
| 200 g/ha | 1.69 | 200 g/ha | 5 % |
| 200 g/ha | 1.69 | 100 g/ha | 10 % |
| 400 g/ha | 1.70 | 400 g/ha | 20 % |
| 400 g/ha | 1.70 | 200 g/ha | 45 % |
| 200 g/ha | 1.70 | 200 g/ha | 30 % |
| 200 g/ha | 1.70 | 100 g/ha | 30 % |
| 400 g/ha | 1.71 | 400 g/ha | 25 % |
| 400 g/ha | 1.71 | 200 g/ha | 10 % |
| 200 g/ha | 1.71 | 200 g/ha | 20 % |
| 200 g/ha | 1.71 | 100 g/ha | 5 % |

Tabelle 1a (Fortsetzung)

| Herbizid Aufwandmenge | Antidote No. | Aufwandmenge | relative Schutzwirkung |
|---|---|---|---|
| 400 g/ha | 1.72 | 400 g/ha | 50 % |
| 400 g/ha | 1.72 | 200 g/ha | 65 % |
| 200 g/ha | 1.72 | 200 g/ha | 25 % |
| 200 g/ha | 1.72 | 100 g/ha | 25 % |
| 400 g/ha | 1.74 | 400 g/ha | 15 % |
| 400 g/ha | 1.74 | 200 g/ha | 30 % |
| 200 g/ha | 1.74 | 200 g/ha | 30 % |
| 200 g/ha | 1.74 | 100 g/ha | 25 % |
| 400 g/ha | 1.75 | 400 g/ha | 30 % |
| 400 g/ha | 1.75 | 200 g/ha | 25 % |
| 200 g/ha | 1.75 | 200 g/ha | 20 % |
| 200 g/ha | 1.75 | 100 g/ha | 15 % |
| 400 g/ha | 1.77 | 400 g/ha | 25 % |
| 200 g/ha | 1.77 | 200 g/ha | 20 % |
| 200 g/ha | 1.77 | 100 g/ha | 25 % |
| 400 g/ha | 1.78 | 400 g/ha | 35 % |
| 400 g/ha | 1.78 | 200 g/ha | 15 % |
| 200 g/ha | 1.78 | 200 g/ha | 15 % |
| 200 g/ha | 1.78 | 100 g/ha | 10 % |
| 400 g/ha | 1.79 | 400 g/ha | 5 % |
| 400 g/ha | 1.79 | 200 g/ha | 30 % |
| 200 g/ha | 1.79 | 200 g/ha | 15 % |
| 200 g/ha | 1.79 | 100 g/ha | 10 % |
| 400 g/ha | 1.88 | 400 g/ha | 13 % |
| 400 g/ha | 1.89 | 400 g/ha | 13 % |
| 400 g/ha | 1.90 | 400 g/ha | 25 % |
| 400 g/ha | 1.90 | 200 g/ha | 25 % |
| 200 g/ha | 1.90 | 200 g/ha | 13 % |
| 200 g/ha | 1.90 | 100 g/ha | 25 % |
| 400 g/ha | 1.93 | 400 g/ha | 13 % |
| 400 g/ha | 1.94 | 400 g/ha | 13 % |
| 400 g/ha | 1.94 | 200 g/ha | 13 % |
| 400 g/ha | 1.95 | 400 g/ha | 13 % |

Eine Untergruppe der Dichloracetamide der Formel II wird durch die Verbindungen der Formel III verkörpert

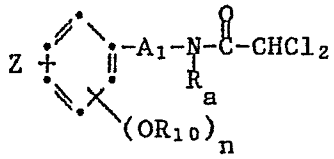

III

worin

$A_1$ eine $C_1$-$C_8$-Alkylenbrücke, die geradkettig oder verzweigt sein kann und die unsubstituiert oder durch Cyan oder $C_1$-$C_4$-Alkoxy oder einen Alkylamido- oder Halogenalkenylamido-Rest substituiert sein kann,

n Null oder eine Zahl von 1 bis 3,

$R_a$ Wasserstoff, $C_1$-$C_8$-Alkyl oder $C_3$-$C_8$-Cycloalkyl, unsubstituiert oder substituiert durch $-PO(R_1)(R_2)$, $-NR_3R_4$, $-(O)_mCOOR_5$, $-(X')_m-CX''R_6$, Cyano, Het, -X'''-Het, $-O-(A-O)_{m'}-R_7$, $-XR_7$, $C_5$-$C_6$-Cycloalkyl, $-CR(OR_8)(OR_9)$ oder Halogen oder $R_a$ ist ein $C_3$-$C_8$-Alkenyl- oder $C_3$-$C_8$-Cycloalkylrest, der unsubstituiert oder durch Halogen, Phenyl, $C_5$-$C_6$-Cycloalkyl oder $C_1$-$C_4$-Alkyl substituiert oder $R_a$ ist ein $C_3$-$C_8$-Alkinylrest, der unsubstituiert oder durch Phenyl substituiert ist, wobei die Phenylkerne unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sind, oder $R_a$ ist ein Alkoxy-iminoalkylrest $-CH(R_r)-C(R_s)=N-OR_t$, $R_r$ und $R_s$ je Wasserstoff oder $C_1$-$C_4$-Alkyl und $R_t$ Wasserstoff, $C_1$-$C_6$-Alkenyl, $C_3$-$C_6$-Alkenyl oder $C_3$-$C_6$-Alkinyl bedeuten,

Z Wasserstoff, $C_1$-$C_4$-Alkyl, Halogen, $C_1$-$C_4$-Halogenalkyl, Nitro, $C_1$-$C_4$-Alkoxy, $C_2$-$C_8$-Halogenalkoxy, der Dioxymethylenrest, ein 1,3-Dioxolan-2-yl- oder 1,3-Dioxan-2-ylrest, der über eine $C_1$-$C_4$-Alkylenbrücke gebunden sein kann,

A eine $C_1$-$C_4$-Alkylenkette

Het einen gegebenenfalls durch $C_1$-$C_4$-Alkyl, $C_1$-$C_4$-Alkoxy, $C_1$-$C_4$-Alkoxycarbonyl oder $C_1$-$C_4$-Alkylthio optimal substituierten 5-6-gliedrigen Heterocyclus mit 1-3 Heteroatomen resp. $S(O)_n$, Gruppen, wobei Sauerstoff und Schwefel nie direkt benachbart im Ring

vorhanden sein können, sondern in einer 1.3-Anwendung vorliegen müssen, wie im 1,3-Dioxolan-2-yl-, 1,3-Dioxan-2-yl oder dem 2,4-Dioxan-1-ylrest und welcher zusätzlich durch $R_n$ in der 1-Stellung und $R'_n$ an einem Ring Kohlenstoffatom substituiert sein kann,

m' Null oder eine Zahl von 1 bis 4, m Null oder 1

R, R', $R_1$ und $R_2$ unabhängig voneinander je $C_1$-$C_4$-Alkyl oder $C_1$-$C_4$-Alkoxy,

$R_3$ Wasserstoff oder $C_1$-$C_4$-Alkyl,

$R_4$ einen Rest -COOR'$_5$ oder -COR'$_6$,

$R_5$ und $R'_5$ unabhängig voneinander je $C_1$-$C_4$-Alkyl, oder $C_1$-$C_4$-Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist,

$R_6$ Wasserstoff, $C_1$-$C_4$-Alkyl, $C_1$-$C_4$-Halogenalkyl, $C_2$-$C_5$-Alkoxyalkyl, Phenyl oder $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist, oder $R_6$ bedeutet einen $C_2$-$C_4$-Alkenyl- oder einen Rest -$NR_{12}$, $R_{13}$,

$R_7$ Wasserstoff, $C_1$-$C_4$-Alkyl, $C_3$-$C_6$-Alkenyl, $C_3$-$C_6$-Alkinyl oder Benzyl,

$R_8$ und $R_9$ unabhängig voneinander je $C_1$-$C_6$-Alkyl oder $C_3$-$C_6$-Alkenyl

$R_{10}$ $C_1$-$C_4$-Alkyl, $C_3$-$C_6$-Alkenyl, $C_2$-$C_6$-Alkoxyalkyl, $C_1$-$C_4$-Halogenalkenyl oder in Zusammenhang mit 2 Sauerstoffatomen auch eine $C_1$-$C_5$-Alkylenbrücke,

$R_{12}$ und $R_{13}$ unabhängig voneinander je Wasserstoff, $C_1$-$C_8$-Alkyl, $C_3$-$C_8$-Alkenyl, Phenyl oder $C_1$-$C_4$-Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist, $R_r$ und $R_s$ je Wasserstoff oder $C_1$-$C_4$-Alkyl und $R_t$ Wasserstoff, $C_1$-$C_6$-Alkyl, $C_3$-$C_6$-Alkenyl oder $C_3$-$C_6$-Alkinyl,

X', X", X'" je Sauerstoff, Schwefel und X Sauerstoff, Schwefel oder -S(O)$_n$,

bedeuten.

Solche Verbindungen sind zum Teil in den veröffentlichten Patentanmeldungen resp. den Patentschriften DE-A 2 218 097, USP 4 124 376, 4 208 203, USP 3 982 923 sowie der schweizerischen Patentanmeldung 2612/84-3 beschrieben. Viele Verbindungen der Formel III sind aus der Literatur bekannt, andere können nach bekannten Methoden hergestellt werden. Folgende Dichloracetamide eignen sich erfindungsgemäss als Antidote zum Sulfonylharnstoff der Formel I besonders gut.

Tabelle 2

| Nr. | $(-O-R_{10})_n$ | Z | $A_1$ | $R_a$ |
|---|---|---|---|---|
| 2.1 | - | - | $-CH_2-$ | $-CH_2-PO(OC_2H_5)_2$ |
| 2.2 | - | 4-Cl | $-CH_2-$ | H |
| 2.3 | $3-OCH_2O-4$ | - | $-CH_2-$ | H |
| 2.4 | - | 2-Cl | $-CH_2-$ | H |
| 2.5 | - | - | $-CH_2-$ | $CH_3$ |
| 2.6 | - | - | $-CH_2-$ | $C_2H_5$ |
| 2.7 | - | - | $-CH_2-$ | Cyclopropyl |
| 2.8 | $4-OCH(CH_3)_2$ | - | $-CH_2-$ | H |
| 2.9 | 4-(1,3-Dioxolan-2-yloxy) | - | $-CH_2-$ | H |
| 2.10 | - | - | -CH(CN)- | H |
| 2.11 | $3,4(OCH_3)_2$ | - | $-CH_2-$ | $-CH(CH_3)_2$ |
| 2.12 | $3,4(OCH_3)_2$ | - | $-CH_2-$ | H |
| 2.13 | - | - | $-C_2H_4-$ | H |
| 2.14 | - | $3,4(CH_3)_2$ | $-CH_2-$ | H |
| 2.15 | - | - | $-C_2H_4CH(CH_3)-$ | H |
| 2.16 | - | - | $-CH_2-$ | $-CH_2CH(OCH_3)_2$ |
| 2.17 | - | - | $-CH_2-$ | $-CH(CN)CH_3$ |
| 2.18 | - | - | $-CH_2-$ | $-C_2H_4OCH_3$ |
| 2.19 | - | 4-Cl | $-CH_2-$ | $-C_2H_4OH$ |
| 2.20 | - | - | $-CH_2-$ | $-C_2H_4OH$ |
| 2.21 | $4-OCH_3$ | - | $-CH_2-$ | $-C_2H_4OH$ |
| 2.22 | $4-OCH_3$ | - | $-CH_2-$ | H |
| 2.23 | $2-OCH_3$ | - | $-CH_2-$ | H |

Tabelle 2 (Fortsetzung)

| Nr. | $(-OR_{10})_n$ | Z | $A_1$ | $R_a$ |
|---|---|---|---|---|
| 2.24 | - | - | $-CH_2-$ | $-CH(CH_3)_2$ |
| 2.25 | 4-$OCH_3$ | - | $-CH_2-$ | $-CH(CH_3)_2$ |
| 2.26 | 4-$OCH_3$ | - | $-C_2H_4-$ | H |
| 2.27 | - | - | $-C_2H_4-$ | H |
| 2.28 | 4-$OCH_3$ | - | $-CH(CH_3)-$ | H |
| 2.29 | - | 2,4$(CH_3)_2$ | $-CH_2-$ | H |
| 2.30 | - | - | $-CH_2-$ | $-CH_2CH(CH_3)-$ |
| 2.31 | - | - | $-CH_2-$ | $-CH_2CN$ |
| 2.32 | 4-$OCH_3$ | - | $-CH_2-$ | $-CH_2CH(OCH_3)_2$ |
| 2.33 | - | - | $-C(CH_3)_2-$ | H |
| 2.34 | - | 3-Cl | $-C(CH_3)_2$ | H |
| 2.35 | - | 4-$CF_3$ | $-CH_2-$ | $-CH(CH_3)_2$ |
| 2.36 | 3,4$(OCH_3)_2$ | - | $-C_2H_4-$ | $-CH(CH_3)_2$ |
| 2.37 | - | - | $-CH_2-$ | $-(C_2H_4O)_2C_4H_9n$ |
| 2.38 | - | - | $-CH_2-$ | $-(C_2H_4O)_3CH_3$ |
| 2.39 | 3,4$(OCH_3)_2$ | - | $-CH_2-$ | $-CH(CH_3)C_2H_5$ |
| 2.40 | 3,4$(OCH_3)_2$ | - | $-CH_2-$ | Tetrahydro-furfuryl |
| 2.41 | 3,4$(OCH_3)_2$ | - | $-CH_2-$ | $-NHCOOC_2H_5$ |
| 2.42 | 3,4,5$(OCH_3)_3$ | - | $-CH_2-$ | $-CH(CH_3)_2$ |
| 2.43 | 3-$OCH_2O$-4 | - | $-CH_2-$ | $-CH(CH_3)_2$ |
| 2.44 | - | - | $-CH_2-$ | $-C_3H_6OCH_3$ |
| 2.45 | - | - | $-C_2H_4-$ | $-CH_2CH(OCH_3)_2$ |

Tabelle 2 (Fortsetzung)

| Nr. | $(-OR_{10})_n$ | Z | $A_1$ | $R_a$ |
|---|---|---|---|---|
| 2.46 | - | - | $-CH_2-$ | $C_3H_6-OC_2H_5$ |
| 2.47 | - | - | $-CH_2-$ | $C_2H_4-OC_2H_5$ |
| 2.48 | - | - | $-C_2H4$ | $-CH_2-CH(OCH_3)_2$ |
| 2.49 | $2,5(OCH_3)_2$ | - | $-CH_2-$ | $-CH(CH_3)_2$ |
| 2.50 | $4-OCH_3$ | - | $-C(CH_3)_2-$ | H |
| 2.51 | - | - | $-CH_2-CH(CH_3)-$ | $-CH(CH_3)_2$ |
| 2.52 | $3,4(OCH_3)_2$ | - | $-CH(CH_3)-$ | H |
| 2.53 | $2,4(OCH_3)_2$ | - | $-CH_2-$ | $-CH(CH_3)_2$ |
| 2.54 | $2,3(OCH_3)_2$ | - | $-CH_2-$ | $-CH(CH_3)_2$ |
| 2.55 | $3-OCH_3$ | - | $-CH_2-$ | $-CH(CH_3)_2$ |
| 2.56 | - | $4-CH_3$ | $-CH_2-$ | $-C_2H_4-OC_2H_5$ |
| 2.57 | - | - | $-CH-$<br>$PO(OC_2H_5)_2$ | H |
| 2.58 | $2,4(OCH_3)_2$ | - | $-CH_2-$ | H |
| 2.59 | $2,4(OCH_3)_2$ | - | $-CH_2-$ | H |
| 2.60 | - | - | $-CH(CH_3)-$ | H |
| 2.61 | - | $2-CH_3$ | $-CH_2-$ | H |
| 2.62 | - | - | $-C_2H_4$ | $CH_3$ |
| 2.63 | - | - | $CH_2$ | 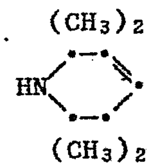 |
| 2.64 | $3,4(OCH_3)_2$ | - | $-CH-NH-$<br>$COCHCl_2$ | H |
| 2.65 | $3-OCH_2O-4$ | - | $C_2H_4$ | $CH(CH_3)_2$ |

Tabelle 2 (Fortsetzung)

| Nr. | Z | $A_1$ | $R_a$ |
|---|---|---|---|
| 2.66 | H | $CH-NHCOCHCl_2$ | H |
| 2.67 | $4-i-C_3H_7$ | $CH-NHCOCHCl_2$ | H |
| 2.68 | $4-CH_3O$ | $CH-NHCOCHCl_2$ | H |
| 2.69 | $3,4-OCH_2CH_2O-$ | $CH-NHCOCHCl_2$ | H |
| 2.70 | $2-CH_3$ | $CH-NHCOCHCl_2$ | H |
| 2.71 | 4-Cl | $CH-NHCOCHCl_2$ | H |
| 2.72 | 3-Cl | $CH-NHCOCHCl_2$ | H |
| 2.73 | $3-NO_2$ | $CH-NHCOCHCl_2$ | H |
| 2.74 | $4-NO_2$ | $CH-NHCOCHCl_2$ | H |
| 2.75 | $2,3-(OCH_3)_2$ | $CH-NHCOCHCl_2$ | H |
| 2.76 | 2-Cl | $CH-NHCOCHCl_2$ | H |
| 2.77 | $2,6-Cl_2$ | $CH-NHCOCHCl_2$ | H |
| 2.78 | $4-CH(NHCOCHCl_2)_2$ | | |

Tabelle 2a

| Nr. | $A_1$ | $R_a$ | phys. Daten |
|---|---|---|---|
| 2.79 | $CH_2$ | $CH(CH_3)_2$ | Oel Beispiel 5, Nr. 2.43 |
| 2.80 | $C_2H_4$ | $CH(CH_3)_2$ | dickes Oel Bsp. 6, Nr. 2.65 |
| 2.81 | $CH_2$ | 1,4-Dioxolan-2-yl-methyl | |
| 2.82 | $CH_2$ | $C_2H_5$ | |
| 2.83 | $CH_2$ | $CH(CH_3)C_2H_5$ | |
| 2.84 | $CH_2$ | $C_3H_7$-n | |
| 2.85 | $CH_2$ | $C_2H_4OCH_3$ | |
| 2.86 | $CH_2$ | $CH_2CH{=}CH_2$ | |
| 2.87 | $C_2H_4$ | $CH_2CH{=}CH_2$ | |
| 2.88 | $CH(CH_3)$ | $CH(CH_3)_2$ | |
| 2.89 | $CH_2$ | $-NH-COOC_2H_5$ | |
| 2.90 | $CH_2$ | Tetrahydro-furan-2-yl | |
| 2.91 | $CH(CCl_3)$ | $CH(CH_3)_2$ | |
| 2.92 | $CH(SCH_3)$ | $CH(CH_3)_2$ | |
| 2.93 | $C(CH_3)_2CH_2$ | $CH(CH_3)_2$ | |
| 2.94 | $CH_2CH(CH_3)$ | $CH(CH_3)_2$ | |
| 2.95 | $CH_2$ | $CH_2CH(CH_3)_2$ | |
| 2.96 | $CH_2$ | $CH_2CH(OCH_3)_2$ | |
| 2.97 | $CH(CF_3)$ | $CH(CH_3)_2$ | |
| 2.98 | $CH(OCH_3)CH_2$ | $CH(CH_3)_2$ | |
| 2.99 | $CH_2$ | $CH(CH_3)CH_2OCH_3$ | |
| 2.100 | $C_2H_4$ | $CH(CH_3)CH_2OCH_3$ | |

Typus

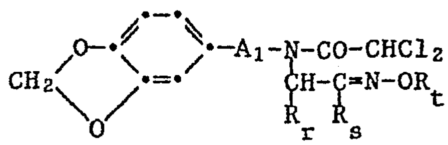

IIIb

| Nr. | $A_1$ | $R_r$ | $R_s$ | $R_t$ |
|---|---|---|---|---|
| 2.101 | $CH_2$ | H | $CH_3$ | $CH_3$ |
| 2.102 | $CH_3-CH_2$ | H | $CH_3$ | $CH_3$ |
| 2.103 | $CH_3$ | $CH_3$ | H | $CH_3$ |
| 2.104 | $CH_3$ | H | H | $C_2H_3$ |
| 2.105 | $CH_3$ | H | H | $CH_3$ |

Die Verbindungen der Tabelle 2a sind neu und bilden ebenfalls einen Gegenstand dieser Erfindung.

Entsprechend der Struktur der Amine der Formeln IIIa und III, die als Zwischenprodukte und Endprodukte verwendet werden, sind verschiedene, an sich bekannte Wege zu deren Herstellung nötig.

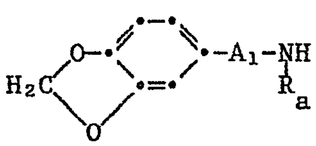

IIIa

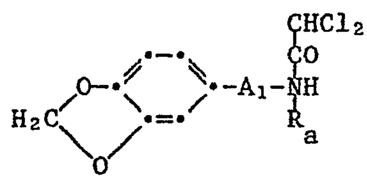

III

Wenn das Brückenglied $A_1$ die Methylengruppe ist, hat man es mit dem Benzylamin zu tun, dessen Herstellung weitgehend bekannt ist und beispielsweise durch Hydrierung von 3,4-Methylendioxy-benzonitril.

Durch katalytische Reduktion von im Phenylkern entsprechend substituierten Acetophenonoximen gelangt man zu α-Methyl-substituierten-Benzylaminen und durch katalytische Hydrierung von entsprechend substituierten α,α,α-Trifluoracetophenonoximen zu in α-Stellung durch die Trifluormethylgruppe substituierten Benzylamine. Weitere in der α-Stellung substituierte Benzylamine lassen

sich durch Anlagerung einer entsprechend substituierten Benzoylverbindung an ein Amin und Erhitzen der Komponenten in Gegenwart von Ameisensäure, Formamid oder Ammoniumformiat in Gegenwart von einer katalytischen Menge einer Lewis Säure (siehe R. Leuckart Ber.22 1409 und 1851).

Durch Abbau von im Phenylkern und in α-Stellung entsprechend substituierter α,α-Phenylessigsäureamiden mittels eines Alkalihypogenites in wässrigem Milieu bei ca. 70°C kann man das Phenylessigsäureamid zum entsprechenden Benzylamin decarboxylieren, (siehe A.W. Hofmann Ber.18 (1885) 2734).

Durch Hydrierung von im Phenylkern in 3,4-Methylendioxy-Stellung substituierten Benzylcyanid lässt sich das entsprechend substituierte primäre Phenyläthylamin herstellen. Sofern das Benzylcyanid in der α-Stellung durch ein oder zwei Alkylreste substituiert ist, entstehen die entsprechend substituierten 2-Aryl-2-alkyl äthylamine bzw. 2-Aryl-2,2-dialkyl-äthylamine, wie es z.B. die folgende Gleichung darlegt:

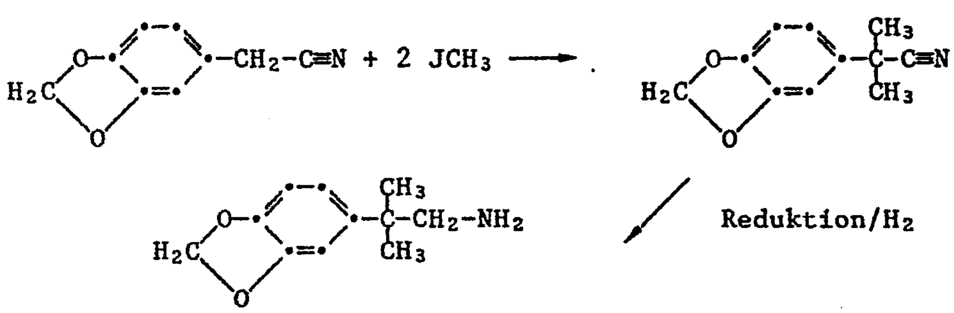


Es können aber auch sekundäre Amine halogenacyliert werden. Diese lassen sich leicht nach an sich bekannten Verfahren z.B. durch Umsetzung des entsprechend substituierten aromatischen Aldehydes mit einem primären Amin und anschliessender Hydrierung herstellen. Durch Kondensation von 3,4-Methylendioxybenzaldehyd und 1-Methyl-2,2,-trifluoräthylamin und anschliessender Hydrierung entsteht das N-(3,4-Methylendioxybenzyl)-N-(1-methyl-2,2,2-trifluoräthyl)-amin gemäss folgendem Schema:

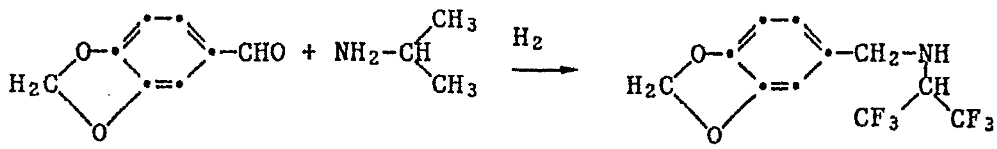


(siehe DE-OS 3 218 201)

Schiff'sche Basen sind geeignete Grundstoffe zur Herstellung weiterer sekundärer Amine. So entsteht z.B. aus 3,4-Methylendioxybenzyliden-butylamin und Isopropylmagnesiumjodid die folgende Gleichung (siehe Naturwissenschaften 48 129 (1961)

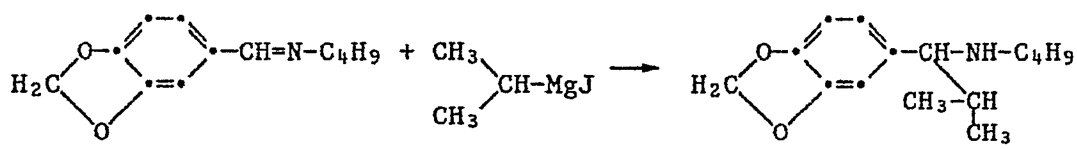

Als weiteres Beispiel für die Herstellung von Aminen der Formel III sei die Anlagerung von Allylmercaptane an Schiff'sche Basen erwähnt, z.B. von 3,4-Methylendioxybenzoliden-allylamin und Methylmercaptan gemäss dem Schema:

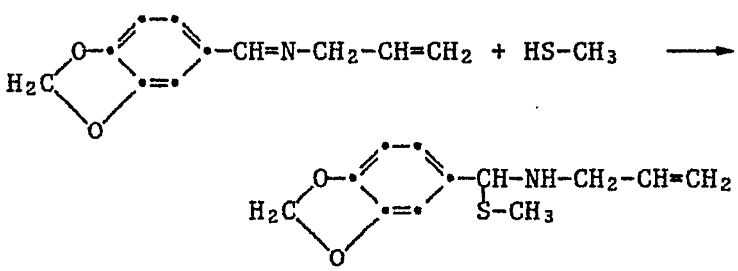

(siehe EP 93 610)

Ebenso erhält man durch Umsetzung von Schiff'schen Basen mit Trichloressigsäure nach der folgenden Gleichung z.B. N-(α-Trichlormethyl-3,4-methylendioxybenzyl)-N-isopropyl-amin

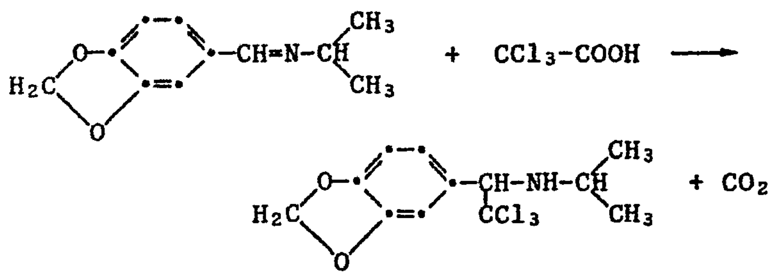

Durch geeignete Reduktion der entsprechend kernsubstituierten Phenylessigsäurealkylamide, z.B. des 3,4-Methylendioxyphenylessigsäure-N-isopropylamides mit Lithiumaluminumhydrid bzw. Boran entstehen weitere Phenylalkylamine der Formel III

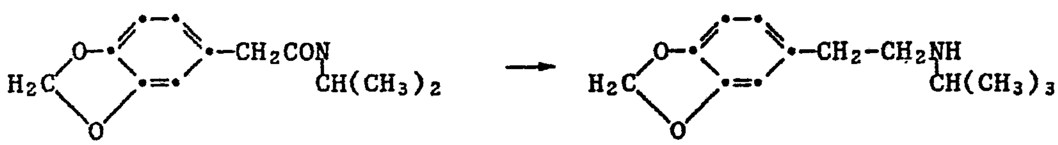

(siehe dazu auch DE-OS 1 959 898)

Verwendet man N(3,4-Methylendioxybenzyl)dichloracetamid und Allylchlorid als Ausgangsstoff, so lässt sich der Verlauf des erfindungsgemässen Verfahrens durch das folgende Formelschema wiedergeben.

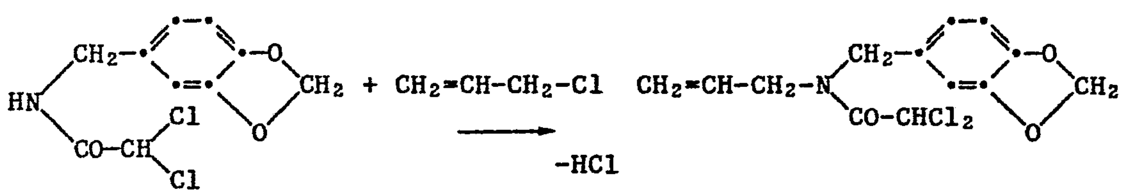


Verwendet man N-Allyl-N-dichloracetamid und 3,4-Methylendioxybenzylchlorid als Ausgangsmaterial, so lässt sich der Verlauf der erfindungsgemässen Verfahren durch das folgende Formelschema wiedergeben:

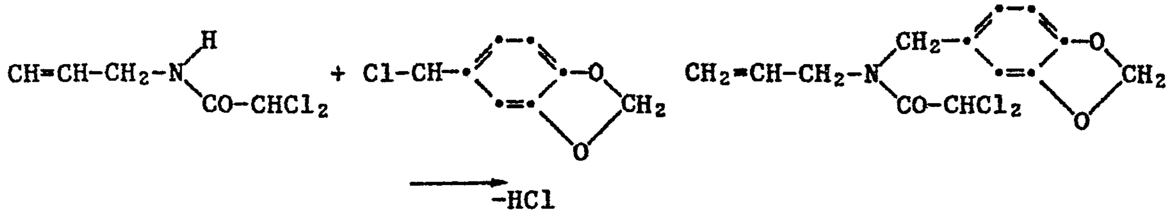

Zur Herstellung des anspruchsgemäss substitutierten N-Benzyl-N-alkoxy-iminoäthyl-dichloracetamide der allgemeinen Formel

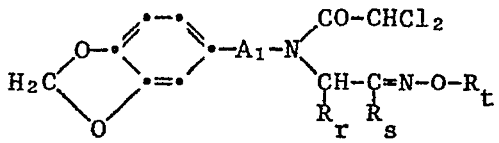

IIIb

wobei die Substituenten $R_r$, $R_s$ und $R_t$ der in der Formel III gegebenen Bedeutung entsprechen,
stehen verschiedene Verfahren zur Verfügung (D-OS 3 004 871)

a) Durch Umsetzung der Säurechloride der Formel

$AcCOCHCl_2$

worin Ac ein Halogenatom oder den Rest $-O-CO-CHCl_2$ darstellt, mit dem Amin der Formel IIIb'

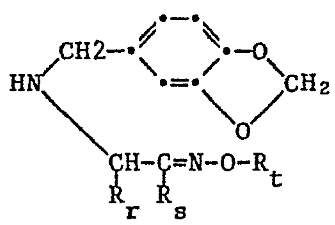

IIIb'

wobei $R_r$, $R_s$ vornehmlich Wasserstoff bedeuten und $R_t$ Methyl, Aethyl und Allyl bedeuten

b) oder Halogenacylamine der Formel

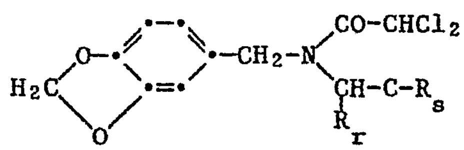

worin $R_s$ Wasserstoff oder Methyl bedeutet

mit $HCl \cdot NH_2-O-R_t$

wobei $R_t$ die angegebene Bedeutung hat in Gegenwart eines säurebindenden Mittels sowie in Gegenwart eines Verdünnungsmittels umsetzt.

Die Aminoderivate der Formel IIIb'

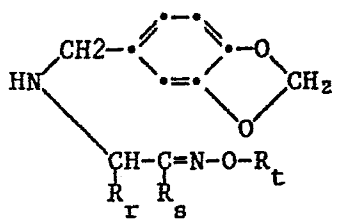

IIIb'

sind neu, lassen sich aber nach bekannten Verfahren herstellen, wie z.B. durch Umsetzung eines Amins der Formel

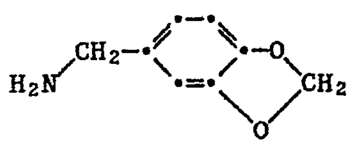

mit einer Iminoverbindung der Formel

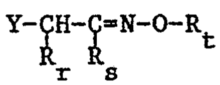

IIIc

wobei $R_r$, $R_s$ und $R_t$ die oben angegebene Bedeutung haben und Y für Halogen, Tosyl oder Mesyl steht.

Verwendet man Dichloressigsäurechlorid und 3,4-Methylendioxybenzyl-2'-Methoxyiminoäthylamin als Ausgangsstoffe, so lässt sich der Verlauf der erfindungsgemässen Verfahrens durch das folgende Formelschema wiedergeben:

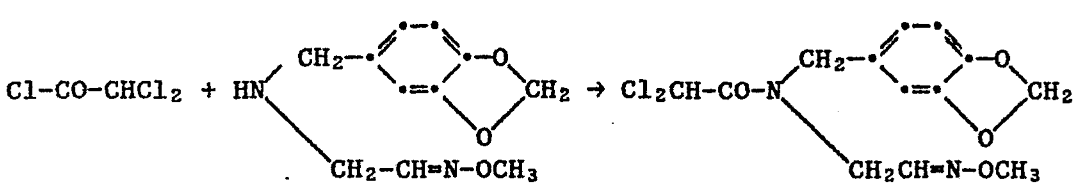

Verwendet man N-2-Oxopropyl-N-3,4-Methylendioxybenzyldichloracetamid und O-Methylhydroxylamin-Chlorhydrid als Ausgangsstoffe, so lässt sich der Verlauf des erfindungsgemässen Verfahrens durch das folgende Formelschema verdeutlichen:

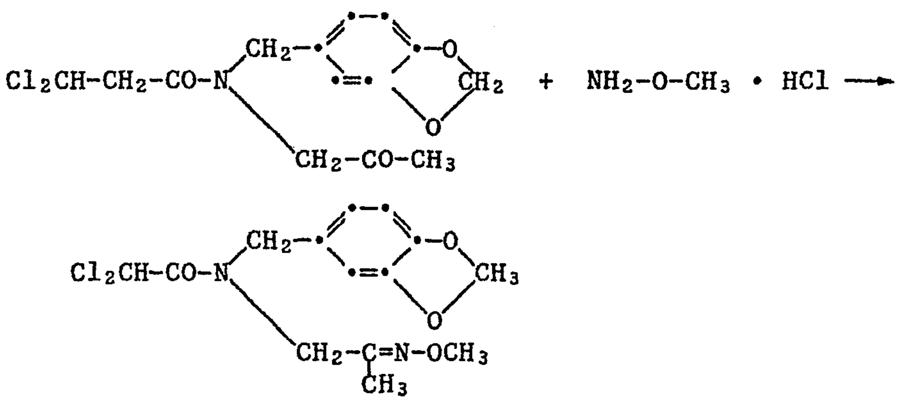


Verbindungen der Formel IIIc lassen sich wie folgt auf einfache Weise herstellen, indem man Verbindungen der Formel, d.h. Carbonylverbindungen

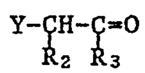

in welcher $R_r$, $R_s$ und Y die oben angegebene Bedeutung haben mit einem Hydroxylamin-Derivat der Formel

$$NH_2 - O - R_t \cdot HCl$$

worin $R_t$ die oben angegebene Bedeutung hat in Gegenwart eines säurebindenden Mittels umsetzt.

Die Halogenacylamine z.B. der Formel

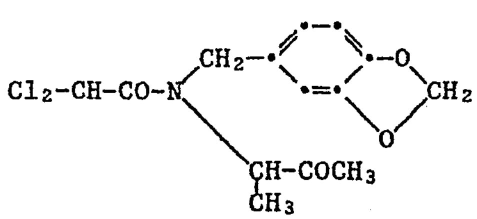

 0190105

lassen sich in einfacher Weise dadurch darstellen, dass man Verbindungen der Formel

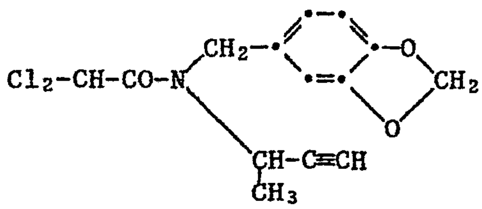

in saurem Medium in Gegenwart eines Katalysators mit Wasser behandelt

bzw. die Halogenacylamine der Formel

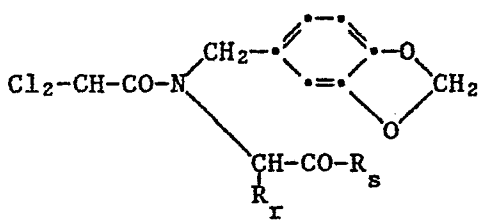

wobei $R_r$ die oben angegebene Bedeutung hat und $R_s$ für Wasserstoff oder Methyl steht

über ihre Acetale

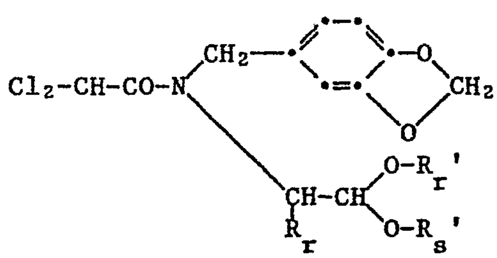

in welchen $R_r$ die oben gegebene Bedeutung hat und für $R_r'$ und $R_s'$ Alkyl steht

und ferner $R_r'$ und $R_s'$ gemeinsam für eine Aethylengruppe steht, in Gegenwart eines sauren Katalysatoren sowie in Gegenwart eines Verdünnungsmittels steht.

Die Fähigkeit der Verbindungen der Formel III, junge Maispflanzen vor der phytotoxischen Wirkung von N-(2-Methoxycarbonylphenyl-sulfonyl)-N'-(4',6'-bis-difluormethoxypyrimidin-2-yl)-harnstoff zu schützen, wurde gemäss Beispiel 1 geprüft. Die Resultate sind in der Tabelle 2b zusammengefasst.

Tabelle 2b

| Herbizid Aufwandmenge | Antidote No. | Aufwandmenge | relative Schutzwirkung |
|---|---|---|---|
| 400 g/ha | 2.1 | 400 g/ha | 55 % |
| 400 g/ha | 2.1 | 200 g/ha | 50 % |
| 200 g/ha | 2.1 | 200 g/ha | 30 % |
| 200 g/ha | 2.1 | 100 g/ha | 35 % |
| 400 g/ha | 2.2 | 400 g/ha | 30 % |
| 400 g/ha | 2.2 | 200 g/ha | 25 % |
| 200 g/ha | 2.2 | 200 g/ha | 10 % |
| 200 g/ha | 2.2 | 100 g/ha | 5 % |
| 400 g/ha | 2.3 | 400 g/ha | 55 % |
| 400 g/ha | 2.3 | 200 g/ha | 50 % |
| 200 g/ha | 2.3 | 100 g/ha | 30 % |
| 400 g/ha | 2.4 | 400 g/ha | 55 % |
| 400 g/ha | 2.4 | 200 g/ha | 25 % |
| 200 g/ha | 2.4 | 200 g/ha | 25 % |
| 200 g/ha | 2.4 | 100 g/ha | 10 % |
| 400 g/ha | 2.5 | 400 g/ha | 70 % |
| 400 g/ha | 2.5 | 200 g/ha | 40 % |
| 200 g/ha | 2.5 | 200 g/ha | 30 % |
| 200 g/ha | 2.5 | 100 g/ha | 40 % |
| 400 g/ha | 2.6 | 400 g/ha | 70 % |
| 400 g/ha | 2.6 | 200 g/ha | 65 % |
| 200 g/ha | 2.6 | 200 g/ha | 50 % |
| 200 g/ha | 2.6 | 100 g/ha | 30 % |
| 400 g/ha | 2.7 | 400 g/ha | 20 % |
| 200 g/ha | 2.7 | 200 g/ha | 20 % |

Tabelle 2b (Fortsetzung)

| Herbizid Aufwandmenge | Antidote No. | Aufwandmenge | relative Schutzwirkung |
|---|---|---|---|
| 400 g/ha | 2.8 | 400 g/ha | 20 % |
| 400 g/ha | 2.8 | 200 g/ha | 15 % |
| 200 g/ha | 2.8 | 200 g/ha | 15 % |
| 200 g/ha | 2.8 | 100 g/ha | 15 % |
| 400 g/ha | 2.9 | 400 g/ha | 35 % |
| 400 g/ha | 2.9 | 200 g/ha | 35 % |
| 200 g/ha | 2.9 | 200 g/ha | 20 % |
| 200 g/ha | 2.9 | 100 g/ha | 20 % |
| 400 g/ha | 2.10 | 400 g/ha | 50 % |
| 400 g/ha | 2.10 | 200 g/ha | 55 % |
| 200 g/ha | 2.10 | 200 g/ha | 45 % |
| 200 g/ha | 2.10 | 100 g/ha | 25 % |
| 400 g/ha | 2.11 | 400 g/ha | 50 % |
| 400 g/ha | 2.11 | 200 g/ha | 355% |
| 400 g/ha | 2.11 | 100 g/ha | 40 % |
| 400 g/ha | 2.11 | 50 g/ha | 45 % |
| 200 g/ha | 2.11 | 200 g/ha | 40 % |
| 200 g/ha | 2.11 | 100 g/ha | 40 % |
| 200 g/ha | 2.11 | 50 g/ha | 40 % |
| 200 g/ha | 2.11 | 25 g/ha | 30 % |
| 100 g/ha | 2.11 | 50 g/ha | 10 % |
| 100 g/ha | 2.11 | 25 g/ha | 20 % |
| 100 g/ha | 2.11 | 12,5 g/ha | 25 % |
| 400 g/ha | 2.12 | 400 g/ha | 45 % |
| 400 g/ha | 2.12 | 200 g/ha | 30 % |
| 200 g/ha | 2.12 | 200 g/ha | 20 % |
| 200 g/ha | 2.12 | 100 g/ha | 15 % |
| 400 g/ha | 2.13 | 400 g/ha | 40 % |
| 400 g/ha | 2.13 | 200 g/ha | 35 % |
| 200 g/ha | 2.13 | 200 g/ha | 25 % |
| 200 g/ha | 2.13 | 100 g/ha | 5 % |
| 400 g/ha | 2.14 | 400 g/ha | 40 % |
| 400 g/ha | 2.14 | 200 g/ha | 40 % |
| 200 g/ha | 2.14 | 200 g/ha | 30 % |
| 400 g/ha | 2.15 | 400 g/ha | 45 % |
| 400 g/ha | 2.15 | 200 g/ha | 10 % |
| 200 g/ha | 2.15 | 200 g/ha | 30 % |
| 200 g/ha | 2.15 | 100 g/ha | 35 % |

Tabelle 2b (Fortsetzung)

| Herbizid Aufwandmenge | Antidote No. | Aufwandmenge | relative Schutzwirkung |
|---|---|---|---|
| 400 g/ha | 2.16 | 400 g/ha | 50 % |
| 400 g/ha | 2.16 | 200 g/ha | 50 % |
| 200 g/ha | 2.16 | 200 g/ha | 30 % |
| 200 g/ha | 2.16 | 100 g/ha | 35 % |
| 400 g/ha | 2.17 | 400 g/ha | 55 % |
| 400 g/ha | 2.17 | 200 g/ha | 75 % |
| 200 g/ha | 2.17 | 200 g/ha | 60 % |
| 200 g/ha | 2.17 | 100 g/ha | 60 % |
| 400 g/ha | 2.18 | 400 g/ha | 40 % |
| 400 g/ha | 2.18 | 200 g/ha | 45 % |
| 200 g/ha | 2.18 | 200 g/ha | 30 % |
| 200 g/ha | 2.18 | 100 g/ha | 25 % |
| 400 g/ha | 2.19 | 400 g/ha | 20 % |
| 400 g/ha | 2.19 | 200 g/ha | 10 % |
| 200 g/ha | 2.19 | 200 g/ha | 5 % |
| 200 g/ha | 2.19 | 100 g/ha | 15 % |
| 400 g/ha | 2.20 | 400 g/ha | 10 % |
| 400 g/ha | 2.20 | 200 g/ha | 15 % |
| 200 g/ha | 2.20 | 200 g/ha | 15 % |
| 200 g/ha | 2.20 | 100 g/ha | 5 % |
| 400 g/ha | 2.21 | 400 g/ha | 35 % |
| 400 g/ha | 2.21 | 200 g/ha | 20 % |
| 200 g/ha | 2.21 | 100 g/ha | 25 % |
| 400 g/ha | 2.22 | 400 g/ha | 25 % |
| 400 g/ha | 2.22 | 200 g/ha | 35 % |
| 200 g/ha | 2.22 | 200 g/ha | 10 % |
| 200 g/ha | 2.22 | 100 g/ha | 5 % |
| 400 g/ha | 2.23 | 400 g/ha | 40 % |
| 400 g/ha | 2.23 | 200 g/ha | 35 % |
| 200 g/ha | 2.23 | 200 g/ha | 30 % |
| 200 g/ha | 2.23 | 100 g/ha | 15 % |
| 400 g/ha | 2.24 | 400 g/ha | 20 % |
| 400 g/ha | 2.24 | 200 g/ha | 25 % |
| 200 g/ha | 2.24 | 200 g/ha | 5 % |
| 400 g/ha | 2.25 | 400 g/ha | 50 % |
| 400 g/ha | 2.25 | 200 g/ha | 40 % |
| 200 g/ha | 2.25 | 200 g/ha | 45 % |
| 200 g/ha | 2.25 | 100 g/ha | 15 % |

Tabelle 2b (Fortsetzung)

| Herbizid Aufwandmenge | Antidote No. | Aufwandmenge | relative Schutzwirkung |
|---|---|---|---|
| 400 g/ha | 2.26 | 400 g/ha | 65 % |
| 400 g/ha | 2.26 | 200 g/ha | 15 % |
| 200 g/ha | 2.26 | 200 g/ha | 10 % |
| 200 g/ha | 2.26 | 100 g/ha | 15 % |
| 400 g/ha | 2.27 | 400 g/ha | 40 % |
| 400 g/ha | 2.27 | 200 g/ha | 35 % |
| 200 g/ha | 2.27 | 200 g/ha | 25 % |
| 200 g/ha | 2.27 | 100 g/ha | 5 % |
| 400 g/ha | 2.28 | 400 g/ha | 45 % |
| 200 g/ha | 2.28 | 200 g/ha | 15 % |
| 200 g/ha | 2.28 | 100 g/ha | 25 % |
| 400 g/ha | 2.29 | 400 g/ha | 45 % |
| 400 g/ha | 2.29 | 200 g/ha | 40 % |
| 200 g/ha | 2.29 | 200 g/ha | 35 % |
| 200 g/ha | 2.29 | 100 g/ha | 45 % |
| 400 g/ha | 2.30 | 400 g/ha | 55 % |
| 400 g/ha | 2.30 | 200 g/ha | 30 % |
| 200 g/ha | 2.30 | 200 g/ha | 30 % |
| 200 g/ha | 2.30 | 100 g/ha | 40 % |
| 400 g/ha | 2.31 | 400 g/ha | 20 % |
| 400 g/ha | 2.31 | 200 g/ha | 30 % |
| 200 g/ha | 2.31 | 200 g/ha | 40 % |
| 200 g/ha | 2.31 | 100 g/ha | 35 % |
| 400 g/ha | 2.32 | 400 g/ha | 45 % |
| 400 g/ha | 2.32 | 200 g/ha | 40 % |
| 200 g/ha | 2.32 | 200 g/ha | 35 % |
| 200 g/ha | 2.32 | 100 g/ha | 35 % |
| 400 g/ha | 2.33 | 400 g/ha | 50 % |
| 400 g/ha | 2.33 | 200 g/ha | 45 % |
| 200 g/ha | 2.33 | 200 g/ha | 35 % |
| 200 g/ha | 2.33 | 100 g/ha | 40 % |
| 400 g/ha | 2.34 | 400 g/ha | 40 % |
| 400 g/ha | 2.34 | 200 g/ha | 45 % |
| 200 g/ha | 2.34 | 200 g/ha | 25 % |
| 200 g/ha | 2.34 | 100 g/ha | 30 % |
| 400 g/ha | 2.35 | 400 g/ha | 10 % |
| 400 g/ha | 2.35 | 200 g/ha | 5 % |
| 200 g/ha | 2.35 | 200 g/ha | 25 % |
| 200 g/ha | 2.35 | 100 g/ha | 25 % |

Tabelle 2b (Fortsetzung)

| Herbizid Aufwandmenge | Antidote No. | Aufwandmenge | relative Schutzwirkung |
|---|---|---|---|
| 400 g/ha | 2.36 | 400 g/ha | 20 % |
| 400 g/ha | 2.36 | 200 g/ha | 25 % |
| 200 g/ha | 2.36 | 200 g/ha | 35 % |
| 200 g/ha | 2.36 | 100 g/ha | 30 % |
| 400 g/ha | 2.38 | 400 g/ha | 45 % |
| 400 g/ha | 2.38 | 200 g/ha | 40 % |
| 400 g/ha | 2.39 | 400 g/ha | 45 % |
| 400 g/ha | 2.39 | 200 g/ha | 35 % |
| 200 g/ha | 2.39 | 200 g/ha | 40 % |
| 200 g/ha | 2.39 | 100 g/ha | 30 % |
| 400 g/ha | 2.40 | 400 g/ha | 30 % |
| 400 g/ha | 2.40 | 200 g/ha | 20 % |
| 200 g/ha | 2.40 | 200 g/ha | 30 % |
| 200 g/ha | 2.40 | 100 g/ha | 30 % |
| 400 g/ha | 2.41 | 400 g/ha | 50 % |
| 400 g/ha | 2.41 | 200 g/ha | 50 % |
| 200 g/ha | 2.41 | 200 g/ha | 45 % |
| 200 g/ha | 2.41 | 100 g/ha | 45 % |
| 400 g/ha | 2.42 | 400 g/ha | 35 % |
| 400 g/ha | 2.42 | 200 g/ha | 50 % |
| 200 g/ha | 2.42 | 200 g/ha | 45 % |
| 200 g/ha | 2.42 | 100 g/ha | 30 % |
| 400 g/ha | 2.43 | 400 g/ha | 50 % |
| 400 g/ha | 2.43 | 200 g/ha | 55 % |
| 200 g/ha | 2.43 | 200 g/ha | 35 % |
| 200 g/ha | 2.43 | 100 g/ha | 30 % |
| 400 g/ha | 2.44 | 400 g/ha | 25 % |
| 400 g/ha | 2.44 | 200 g/ha | 50 % |
| 200 g/ha | 2.44 | 200 g/ha | 35 % |
| 200 g/ha | 2.44 | 100 g/ha | 50 % |
| 400 g/ha | 2.45 | 400 g/ha | 35 % |
| 400 g/ha | 2.45 | 200 g/ha | 40 % |
| 200 g/ha | 2.45 | 200 g/ha | 45 % |
| 200 g/ha | 2.45 | 100 g/ha | 45 % |
| 400 g/ha | 2.58 | 400 g/ha | 5 % |
| 200 g/ha | 2.58 | 100 g/ha | 5 % |

Tabelle 2b (Fortsetzung)

| Herbizid Aufwandmenge | Antidote No. | Aufwandmenge | relative Schutzwirkung |
|---|---|---|---|
| 400 g/ha | 2.59 | 400 g/ha | 25 % |
| 400 g/ha | 2.59 | 200 g/ha | 15 % |
| 200 g/ha | 2.59 | 200 g/ha | 15 % |
| 200 g/ha | 2.59 | 100 g/ha | 20 % |
| 400 g/ha | 2.60 | 400 g/ha | 15 % |
| 400 g/ha | 2.60 | 200 g/ha | 10 % |
| 200 g/ha | 2.60 | 200 g/ha | 15 % |
| 200 g/ha | 2.60 | 100 g/ha | 25 % |
| 400 g/ha | 2.61 | 400 g/ha | 38 % |
| 400 g/ha | 2.61 | 200 g/ha | 13 % |
| 200 g/ha | 2.61 | 200 g/ha | 13 % |
| 200 g/ha | 2.61 | 100 g/ha | 13 % |
| 400 g/ha | 2.64 | 400 g/ha | 13 % |
| 400 g/ha | 2.201 | 400 g/ha | 55 % |
| 400 g/ha | 2.201 | 200 g/ha | 60 % |
| 200 g/ha | 2.201 | 200 g/ha | 25 % |
| 200 g/ha | 2.201 | 100 g/ha | 35 % |
| 400 g/ha | 2.202 | 400 g/ha | 45 % |
| 400 g/ha | 2.202 | 200 g/ha | 55 % |
| 200 g/ha | 2.202 | 200 g/ha | 40 % |
| 200 g/ha | 2.202 | 100 g/ha | 40 % |
| 400 g/ha | 2.203 | 400 g/ha | 70 % |
| 400 g/ha | 2.203 | 200 g/ha | 50 % |
| 200 g/ha | 2.203 | 200 g/ha | 20 % |
| 200 g/ha | 2.203 | 100 g/ha | 10 % |
| 400 g/ha | 2.205 | 400 g/ha | 50 % |
| 400 g/ha | 2.205 | 200 g/ha | 55 % |
| 200 g/ha | 2.205 | 200 g/ha | 45 % |
| 200 g/ha | 2.205 | 100 g/ha | 30 % |
| 400 g/ha | 2.206 | 400 g/ha | 35 % |
| 400 g/ha | 2.206 | 200 g/ha | 35 % |
| 200 g/ha | 2.206 | 200 g/ha | 25 % |
| 200 g/ha | 2.206 | 100 g/ha | 20 % |
| 400 g/ha | 2.208 | 400 g/ha | 10 % |
| 400 g/ha | 2.208 | 200 g/ha | 25 % |
| 200 g/ha | 2.208 | 200 g/ha | 30 % |
| 200 g/ha | 2.208 | 100 g/ha | 35 % |

Tabelle 2b (Fortsetzung)

| Herbizid Aufwandmenge | Antidote No. | Aufwandmenge | relative Schutzwirkung |
|---|---|---|---|
| 400 g/ha | 2.209 | 400 g/ha | 40 % |
| 400 g/ha | 2.209 | 200 g/ha | 55 % |
| 200 g/ha | 2.209 | 200 g/ha | 35 % |
| 200 g/ha | 2.209 | 100 g/ha | 50 % |
| 400 g/ha | 2.210 | 400 g/ha | 45 % |
| 400 g/ha | 2.210 | 200 g/ha | 55 % |
| 200 g/ha | 2.210 | 200 g/ha | 40 % |
| 200 g/ha | 2.210 | 100 g/ha | 50 % |
| 400 g/ha | 2.211 | 400 g/ha | 50 % |
| 400 g/ha | 2.211 | 200 g/ha | 50 % |
| 200 g/ha | 2.211 | 200 g/ha | 40 % |
| 200 g/ha | 2.211 | 100 g/ha | 40 % |
| 400 g/ha | 2.212 | 200 g/ha | 20 % |
| 200 g/ha | 2.212 | 200 g/ha | 35 % |
| 200 g/ha | 2.212 | 100 g/ha | 40 % |
| 400 g/ha | 2.213 | 200 g/ha | 20 % |
| 200 g/ha | 2.213 | 200 g/ha | 20 % |
| 200 g/ha | 2.213 | 100 g/ha | 30 % |

Eine Untergruppe der Dichloracetamide der Formel II wird durch die Verbindungen der Formel IV verkörpert

$$Het-(\overset{R_{11}}{\overset{|}{C}}H)_n-\underset{\underset{R_a}{|}}{N}-COCHCl_2 \qquad (IV)$$

worin Het einen gegebenenfalls durch $C_1$-$C_4$-Alkyl, $C_1$-$C_4$-Alkoxy, $C_1$-$C_4$-Alkoxycarbonyl oder $C_1$-$C_4$-Alkylthio optimal substituierten 5-6 gliedrigen Heterocyclus mit 1-3 Heteroatomen resp. $S(O)_{n'}$ Gruppen, wobei Sauerstoff und Schwefel nie direkt benachbart im Ring vorhanden sein können, sondern in einer 1,3-Anordnung vorliegen müssen, wie im 1,3-Dioxolan-2-yl-, 1,3-Dioxan-2-yl oder dem 2,4-Dioxan-1-ylrest und welcher zusätzlich durch $R_n$ in der 1-Stellung und $R_{n'}$ an einen Ringkohlenstoff substituiert sein kann,
$R_a$ Wasserstoff $C_1$-$C_8$-Alkyl oder $C_3$-$C_8$-Cycloalkyl unsubstituiert oder substituiert durch $-PO(R_1)(R_2)$, $-NR_3,R_4$, $-(O)_mCOOR_5$, $-(X')_mCXR''_6$, $-O-(A-O-)_m-R_7$, $-XR_7$, Cyano, -X"'-Het, Het, $-C_5$-$C_6$-Cycloalkyl, $-CR(OR_8)(OR_9)$ oder Halogen; oder $R_a$ ist ein $C_3$-$C_8$-Alkenyl- oder $C_3$-$C_8$-Cycloalkenylrest, der unsubstituiert oder substituiert durch Halogen, Phenyl, $C_5$-$C_6$-Cycloalkyl oder $C_1$-$C_4$-Alkyl ist oder $R_a$ ist ein $C_3$-$C_8$-Alkinylrest der unsubstituiert oder durch Phenyl substituiert ist, wobei die Phenylkerne unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sind, oder $R_a$ ist ein Alkoxyiminoalkylrest $-CH(R_r)-CH(R_s)=N-O-R_t$, $R_r$ und $R_s$ je Wasserstoff oder $C_1$-$C_4$-Alkyl und $R_t$ Wasserstoff, $C_1$-$C_6$-Alkyl, $C_3$-$C_6$-Alkenyl oder $C_3$-$C_6$-Alkinyl,
A eine $C_1$-$C_4$-Alkylenkette,
m Null oder eins
m' Null oder eine Zahl von 1 bis 4,
n' Null bis 2,
n Null bis 3,
R, R', $R_1$ und $R_2$ unabhängig voneinander je $C_1$-$C_4$-Alkyl oder $C_1$-$C_4$-Alkoxy,
$R_3$ Wasserstoff, $C_1$-$C_4$-Alkyl oder $C_1$-$C_8$-Cycloalkyl

$R_4$ Wasserstoff, $C_1$-$C_4$-Alkyl, Phenyl, durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl oder einen Rest -COOR'$_5$ oder -COR'$_6$

$R_5$ und $R'_5$ unabhängig voneinander je $C_1$-$C_4$-Alkyl oder $C_1$-$C_4$-Aralkyl wobei der Phenylkern unsubstituiert oder durch Halogen $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist.

$R_6$ und $R'_6$ unabhängig voneinander je Wasserstoff, $C_1$-$C_4$-Alkyl, $C_1$-$C_4$-Halogenalkyl, $C_{2-8}$-Alkoxyalkyl, Phenyl oder $C_1$-$C_4$-Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist; oder $R_6$ und $R'_6$ bedeuten einen $C_2$-$C_4$-Alkenyl- oder einen Rest -N($R_{12}$)($R_{13}$)

$R_7$ Wasserstoff, $C_1$-$C_4$-Alkyl, $C_3$-$C_6$-Alkenyl, $C_3$-$C_6$-Alkinyl oder Phenyl($C_1$-$C_4$)alkyl, wobei die Phenylkerne durch Halogen, $C_1$-$C_4$-Alkyl, $C_1$-$C_4$-Alkoxy oder $C_1$-$C_4$-Halogenalkoxy substituiert sind

$R_8$ und $R_9$ unabhängig voneinander je $C_1$-$C_6$-Alkyl oder $C_3$-$C_6$-Alkenyl,

$R_{11}$ Wasserstoff oder $C_1$-$C_4$-Alkyl,

$R_{12}$ und $R_{13}$ unabhängig voneinander je Wasserstoff, $C_1$-$C_8$-Alkyl, $C_3$-$C_6$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert,

$R_{12}$ und $R_{13}$ unabhängig voneinander je Wasserstoff, $C_1$-$C_8$-Alkyl, $C_3$-$C_8$-Cycloalkyl, $C_3$-$C_8$-Alkenyl, Phenyl oder $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan, Nitro und

$R_{12}$ und $R_{13}$ zusammen eine $C_1$-$C_7$-Alkylenkette, die durch Sauerstoff, Schwefel-NH- oder -N($C_1$-$C_4$)alkyl-unterbrochen sein kann,

$R_n$ Wasserstoff, $C_1$-$C_4$-Alkyl oder Phenyl welches unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist, $R_r$, $R_s$ je Wasserstoff oder $C_1$-$C_4$-Alkyl und $R_t$ Wasserstoff, $C_1$-$C_6$-Alkyl, $C_3$-$C_6$-Alkenyl oder $C_3$-$C_6$-Alkinyl,

X', X", X"' Sauerstoff, Schwefel, X Sauerstoff, Schwefel $-S(O)_n$, bedeuten.

Solche Verbindungen sind zum Teil in den veröffentlichten Patentanmeldungen resp. den Patentschriften EP-A 31 686, EP-A 49 760, EP-A 68 709, USP 4 336 058 und 4 406 686 bekannt geworden oder sie können nach bekannten Methoden hergestellt werden. Folgende Dichloracetamide eignen sich erfindungsgemäss als Antidote zum Sulfonylharnstoff der Formel I besonders gut.

Tabelle 3

| Nr. | Het | $R_{11}$ \| $-(CH_2)_n$ | $R_9$ |
|---|---|---|---|
| 3.1 | 1,3-Dioxolan-2-yl | $-CH_2CH_2-$ | $-CH_2CH=CH_2$ |
| 3.2 | 1H-Indol-3-yl | $-CH_2CH_2$ | H |
| 3.3 | 2-Furyl | $-CH_2-$ | $-CH_2COOCH_3$ |
| 3.4 | 2-Pyridyl | $-CH_2-$ | H |
| 3.5 | 4-Pyridyl | $-CH_2-$ | $-CH_2CH_2OH$ |
| 3.6 | 3-Pyridyl | $-CH_2-$ | $-CH_2CH_2OH$ |
| 3.7 | 3-Pyridyl | $-CH_2-$ | H |
| 3.8 | 4-Pyridyl | $-CH_2-$ | H |
| 3.9 | 2-Pyridyl | $-CH_2-$ | $CH_3$ |
| 3.10 | 1,3-Dioxolan-2-yl | $-CH_2-$ | $-CH_2-CH=CH_2$ |
| 3.11 | 2-Furyl | $-CH_2-$ | H |
| 3.12 | 2-Furyl | $-CH_2-$ | $-CH(CH_3)_2$ |
| 3.13 | 2-Furyl | $-CH_2-$ | $-C_2H_5$ |
| 3.14 | 2-Furyl | $-CH_2-$ | $-nC_3H_7$ |
| 3.15 | 2-Furyl | $-CH_2$ | $-C(CH_3)_3$ |
| 3.16 | 2-Furyl | $-CH_2-$ | 2-Furyl |
| 3.17 | 2-Furyl | $-CH_2-$ | 2-Tetrahydro-furfuryl |
| 3.18 | 3-Tetrahydro-furfuryl | $-CH_2-$ | H |
| 3.19 | 2-Methyl-1,3-dioxolan-2-yl | $-CH_2$ | H |

Die Fähigkeit der Verbindungen der Formel III, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 3a zusammengefasst.

Tabelle 3a

| Herbizid Aufwandmenge | Antidote No. | Aufwandmenge | relative Schutzwirkung |
|---|---|---|---|
| 400 g/ha | 3.1 | 400 g/ha | 25 % |
| 400 g/ha | 3.1 | 200 g/ha | 15 % |
| 200 g/ha | 3.1 | 200 g/ha | 20 % |
| 200 g/ha | 3.1 | 100 g/ha | 30 % |
| 400 g/ha | 3.2 | 200 g/ha | 5 % |
| 200 g/ha | 3.2 | 100 g/ha | 10 % |
| 400 g/ha | 3.3 | 400 g/ha | 20 % |
| 400 g/ha | 3.3 | 200 g/ha | 10 % |
| 400 g/ha | 3.8 | 400 g/ha | 45 % |
| 400 g/ha | 3.8 | 200 g/ha | 40 % |
| 200 g/ha | 3.8 | 200 g/ha | 15 % |
| 200 g/ha | 3.8 | 100 g/ha | 20 % |
| 400 g/ha | 3.9 | 400 g/ha | 45 % |
| 400 g/ha | 3.9 | 200 g/ha | 40 % |
| 200 g/ha | 3.9 | 200 g/ha | 35 % |
| 200 g/ha | 3.9 | 100 g/ha | 25 % |
| 400 g/ha | 3.10 | 400 g/ha | 55 % |
| 400 g/ha | 3.10 | 200 g/ha | 65 % |
| 200 g/ha | 3.10 | 200 g/ha | 50 % |
| 200 g/ha | 3.10 | 100 g/ha | 30 % |
| 400 g/ha | 3.11 | 400 g/ha | 45 % |
| 400 g/ha | 3.11 | 200 g/ha | 35 % |
| 200 g/ha | 3.11 | 200 g/ha | 20 % |
| 200 g/ha | 3.11 | 100 g/ha | 20 % |
| 400 g/ha | 3.12 | 400 g/ha | 35 % |
| 400 g/ha | 3.12 | 200 g/ha | 30 % |
| 200 g/ha | 3.12 | 200 g/ha | 10 % |
| 400 g/ha | 3.13 | 400 g/ha | 60 % |
| 400 g/ha | 3.13 | 200 g/ha | 55 % |
| 200 g/ha | 3.13 | 200 g/ha | 25 % |
| 200 g/ha | 3.13 | 100 g/ha | 40 % |
| 400 g/ha | 3.14 | 400 g/ha | 40 % |
| 400 g/ha | 3.14 | 200 g/ha | 40 % |
| 200 g/ha | 3.14 | 200 g/ha | 30 % |
| 200 g/ha | 3.14 | 100 g/ha | 30 % |
| 400 g/ha | 3.15 | 400 g/ha | 45 % |
| 400 g/ha | 3.15 | 200 g/ha | 15 % |
| 200 g/ha | 3.15 | 200 g/ha | 40 % |
| 200 g/ha | 3.15 | 100 g/ha | 35 % |

Tabelle 3a (Fortsetzung)

| Herbizid Aufwandmenge | Antidote No. | Aufwandmenge | relative Schutzwirkung |
|---|---|---|---|
| 400 g/ha | 3.16 | 400 g/ha | 45 % |
| 400 g/ha | 3.16 | 200 g/ha | 50 % |
| 200 g/ha | 3.16 | 200 g/ha | 40 % |
| 200 g/ha | 3.16 | 100 g/ha | 40 % |
| 400 g/ha | 3.17 | 400 g/ha | 35 % |
| 400 g/ha | 3.17 | 200 g/ha | 50 % |
| 200 g/ha | 3.17 | 200 g/ha | 30 % |
| 200 g/ha | 3.17 | 100 g/ha | 45 % |
| 400 g/ha | 3.18 | 400 g/ha | 25 % |

Eine Untergruppe der Dichloracetamide der Formel II wird durch die Verbindungen der Formel V verkörpert

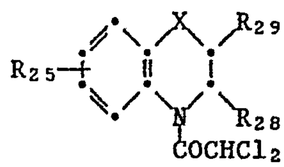

(V)

worin

$R_{25}$ Wasserstoff, $C_1$-$C_4$-Alkyl, unsubstituiert oder substituiert durch Hydroxy, $C_1$-$C_4$-Alkoxy oder Halogen,

$R_{28}$ Wasserstoff, $C_1$-$C_4$-Alkyl unsubstituiert oder substituiert durch Halogen, Cyan oder einen Rest $-COA_3$; $C_2$-$C_4$-Alkenyl, $C_2$-$C_4$-Alkinyl, $C_2$-$C_4$-Alkoxyalkyl, Cyano oder einen Rest -COA;

$R_{29}$ Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

A $C_1$-$C_4$-Alkyl

$A_3$ $C_1$-$C_4$-Alkyl, $C_2$-$C_4$-Alkenyl oder einen Rest $-OR'''_5$ oder $-N(R'''_{12})(R'''_{13})$,

$R'''_5$ $C_1$-$C_4$-Alkyl, $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyano oder Nitro substituiert ist,

$R'''_{12}$ und $R'''_{13}$ unabhängig voneinander je Wasserstoff, $C_1$-$C_8$-Alkyl, $C_2$-$C_8$-Alkenyl, Phenyl oder $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist und X Sauerstoff, Schwefel, SO oder $SO_2$, bedeuten.

Solche Verbindungen sind zum Teil in den US Patentanmeldungen Ser. No. 560 465 und 560 466 vom 12. Dezember 1983 beschrieben oder sie können nach bekannten Methoden hergestellt werden. Folgende Dichloracetamide eignen sich erfindungsgemäss als Antidote zum Sulfonylharnstoff der Formel I besonders gut.

Tabelle 4

| Nr. | $R_{25}$ | X | $R_{28}$ | $R_{29}$ |
|---|---|---|---|---|
| 4.1 | H | O | $CH_3$ | H |
| 4.2 | 6-$CH_3$ | O | $CH_3$ | H |
| 4.3 | H | O | H | H |
| 4.4 | 6-$CH_3$, 8-$CH_3$ | O | H | H |
| 4.5 | 5-$CH_3$ | O | $CH_3$ | H |
| 4.6 | H | S | H | H |
| 4.7 | Cl | O | $CH_3$ | H |
| 4.8 | 7-$CH_3$ | O | $CH_3$ | H |
| 4.9 | 6-$CH_3$, 6-$CH_3$ | O | $CH_3$ | H |
| 4.10 | 6-Cl, 8-Cl | O | $CH_3$ | |
| 4.11 | H | O | $CH_3$ | $CH_3$ |
| 4.12 | H | O | H | $OC_2H_5$ |
| 4.13 | H | S | $CH_3$ | |
| 4.14 | | S⟶O | H | |
| 4.15 | H | $S(O)_2$ | H | H |
| 4.16 | 5-$CH_3$ | O | $CH_3$ | $CH_3$ |
| 4.17 | H | S⟶O (trans) | $CH_3$ | H |
| 4.18 | H | S⟶O (cis) | $CH_3$ | H |
| 4.19 | H | O | H | $COOCH_3$ |
| 4.20 | H | O | H | CN |
| 4.21 | 6-$CH_3$ | O | H | $CH_3$ |

Tabelle 4a

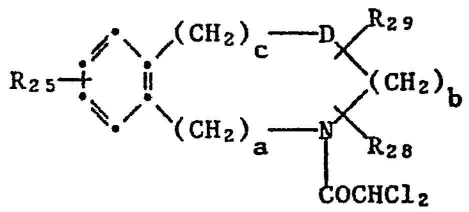

(Va)

| Nr. | $R_{25}$ | $R_{28}$ | $R_{29}$ | D | a | b | c |
|---|---|---|---|---|---|---|---|
| 4.100 | H | H | H | O | O | 2 | 1 |
| 4.101 | H | H | 2-i-$C_3H_7$ | S | O | 1 | O |
| 4.102 | H | H | H | S | O | 1 | O |
| 4.103 | H | H | 2-$CH_3$ | S | O | 1 | O |
| 4.104 | H | H | 2-$CH_3$ | O | O | 1 | 1 |
| 4.105 | H | H | H | S | O | 3 | O |
| 4.106 | H | $CH_3$ | H-C=O | NH | O | 2 | O |
| 4.107 | H | H=O | H-C=CO | NM | O | 2 | O |

Die Fähigkeit der Verbindungen der Formel V, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 4b zusammengefasst.

Tabelle 4b

| Herbizid Aufwandmenge | Antidote No. | Aufwandmenge | relative Schutzwirkung |
|---|---|---|---|
| 400 g/ha | 4.1 | 400 g/ha | 55 % |
| 400 g/ha | 4.1 | 200 g/ha | 55 % |
| 400 g/ha | 4.1 | 100 g/ha | 55 % |
| 400 g/ha | 4.1 | 50 g/ha | 40 % |
| 200 g/ha | 4.1 | 200 g/ha | 65 % |
| 200 g/ha | 4.1 | 100 g/ha | 65 % |
| 200 g/ha | 4.1 | 50 g/ha | 60 % |
| 200 g/ha | 4.1 | 25 g/ha | 60 % |
| 100 g/ha | 4.1 | 100 g/ha | 25 % |
| 100 g/ha | 4.1 | 50 g/ha | 30 % |
| 100 g/ha | 4.1 | 25 g/ha | 30 % |
| 100 g/ha | 4.1 | 12.5 g/ha | 30 % |
| 400 g/ha | 4.2 | 400 g/ha | 60 % |
| 400 g/ha | 4.2 | 200 g/ha | 70 % |
| 400 g/ha | 4.2 | 100 g/ha | 70 % |
| 400 g/ha | 4.2 | 50 g/ha | 50 % |
| 200 g/ha | 4.2 | 200 g/ha | 40 % |
| 200 g/ha | 4.2 | 100 g/ha | 50 % |
| 200 g/ha | 4.2 | 50 g/ha | 45 % |
| 200 g/ha | 4.2 | 25 g/ha | 25 % |
| 100 g/ha | 4.2 | 100 g/ha | 20 % |
| 100 g/ha | 4.2 | 50 g/ha | 25 % |
| 100 g/ha | 4.2 | 25 g/ha | 25 % |
| 100 g/ha | 4.2 | 12.5 g/ha | 20 % |
| 400 g/ha | 4.3 | 400 g/ha | 10 % |
| 400 g/ha | 4.3 | 200 g/ha | 15 % |
| 200 g/ha | 4.3 | 200 g/ha | 20 % |
| 400 g/ha | 4.4 | 400 g/ha | 50 % |
| 400 g/ha | 4.4 | 200 g/ha | 25 % |
| 200 g/ha | 4.4 | 200 g/ha | 30 % |
| 200 g/ha | 4.4 | 100 g/ha | 25 % |
| 400 g/ha | 4.6 | 400 g/ha | 70 % |
| 400 g/ha | 4.6 | 200 g/ha | 65 % |
| 400 g/ha | 4.6 | 100 g/ha | 30 % |
| 400 g/ha | 4.6 | 50 g/ha | 35 % |
| 200 g/ha | 4.6 | 200 g/ha | 10 % |
| 200 g/ha | 4.6 | 100 g/ha | 15 % |
| 200 g/ha | 4.6 | 50 g/ha | 25 % |
| 200 g/ha | 4.6 | 25 g/ha | 40 % |

Tabelle 4b (Fortsetzung)

| Herbizid Aufwandmenge | Antidote No. | Aufwandmenge | relative Schutzwirkung |
|---|---|---|---|
| 100 g/ha | 4.6 | 100 g/ha | 20 % |
| 100 g/ha | 4.6 | 50 g/ha | 10 % |
| 100 g/ha | 4.6 | 25 g/ha | 20 % |
| 100 g/ha | 4.6 | 12.5 g/ha | 20 % |
| 400 g/ha | 4.7 | 400 g/ha | 45 % |
| 400 g/ha | 4.7 | 200 g/ha | 35 % |
| 200 g/ha | 4.7 | 200 g/ha | 30 % |
| 200 g/ha | 4.7 | 100 g/ha | 30 % |
| 400 g/ha | 4.8 | 400 g/ha | 55 % |
| 400 g/ha | 4.8 | 200 g/ha | 75 % |
| 200 g/ha | 4.8 | 200 g/ha | 60 % |
| 200 g/ha | 4.8 | 100 g/ha | 65 % |
| 400 g/ha | 4.9 | 400 g/ha | 30 % |
| 400 g/ha | 4.9 | 200 g/ha | 45 % |
| 200 g/ha | 4.9 | 200 g/ha | 30 % |
| 200 g/ha | 4.9 | 100 g/ha | 35 % |
| 400 g/ha | 4.10 | 200 g/ha | 40 % |
| 200 g/ha | 4.10 | 100 g/ha | 20 % |
| 400 g/ha | 4.11 | 400 g/ha | 30 % |
| 400 g/ha | 4.11 | 200 g/ha | 40 % |
| 200 g/ha | 4.11 | 200 g/ha | 35 % |
| 200 g/ha | 4.11 | 100 g/ha | 35 % |
| 400 g/ha | 4.12 | 200 g/ha | 30 % |
| 200 g/ha | 4.12 | 200 g/ha | 15 % |
| 200 g/ha | 4.12 | 100 g/ha | 30 % |
| 400 g/ha | 4.13 | 400 g/ha | 35 % |
| 400 g/ha | 4.13 | 200 g/ha | 35 % |
| 200 g/ha | 4.13 | 200 g/ha | 25 % |
| 200 g/ha | 4.13 | 100 g/ha | 25 % |
| 400 g/ha | 4.14 | 400 g/ha | 30 % |
| 400 g/ha | 4.14 | 200 g/ha | 35 % |
| 200 g/ha | 4.14 | 200 g/ha | 40 % |
| 200 g/ha | 4.14 | 100 g/ha | 30 % |
| 400 g/ha | 4.16 | 400 g/ha | 25 % |
| 400 g/ha | 4.16 | 200 g/ha | 25 % |
| 200 g/ha | 4.16 | 200 g/ha | 30 % |
| 200 g/ha | 4.16 | 100 g/ha | 30 % |

Tabelle 4b (Fortsetzung)

| Herbizid Aufwandmenge | Antidote No. | Aufwandmenge | relative Schutzwirkung |
|---|---|---|---|
| 400 g/ha | 4.17 | 400 g/ha | 25 % |
| 400 g/ha | 4.17 | 200 g/ha | 45 % |
| 200 g/ha | 4.17 | 200 g/ha | 5 % |
| 200 g/ha | 4.17 | 100 g/ha | 30 % |
| 400 g/ha | 4.18 | 400 g/ha | 25 % |
| 400 g/ha | 4.18 | 200 g/ha | 15 % |
| 200 g/ha | 4.18 | 200 g/ha | 25 % |
| 200 g/ha | 4.18 | 100 g/ha | 25 % |
| 400 g/ha | 4.19 | 200 g/ha | 13 % |
| 400 g/ha | 4.100 | 400 g/ha | 60 % |
| 400 g/ha | 4.100 | 200 g/ha | 45 % |
| 200 g/ha | 4.100 | 200 g/ha | 15 % |
| 200 g/ha | 4.100 | 100 g/ha | 25 % |
| 400 g/ha | 4.101 | 400 g/ha | 50 % |
| 400 g/ha | 4.101 | 200 g/ha | 15 % |
| 200 g/ha | 4.101 | 200 g/ha | 20 % |
| 200 g/ha | 4.101 | 100 g/ha | 20 % |
| 400 g/ha | 4.102 | 400 g/ha | 65 % |
| 400 g/ha | 4.102 | 200 g/ha | 50 % |
| 200 g/ha | 4.102 | 200 g/ha | 15 % |
| 400 g/ha | 4.103 | 400 g/ha | 75 % |
| 400 g/ha | 4.103 | 200 g/ha | 65 % |
| 200 g/ha | 4.103 | 200 g/ha | 30 % |
| 200 g/ha | 4.103 | 100 g/ha | 25 % |
| 400 g/ha | 4.104 | 400 g/ha | 50 % |
| 400 g/ha | 4.104 | 200 g/ha | 25 % |
| 200 g/ha | 4.104 | 200 g/ha | 20 % |
| 200 g/ha | 4.104 | 100 g/ha | 20 % |
| 400 g/ha | 4.105 | 400 g/ha | 10 % |
| 400 g/ha | 4.105 | 200 g/ha | 10 % |
| 200 g/ha | 4.105 | 200 g/ha | 35 % |
| 200 g/ha | 4.105 | 100 g/ha | 20 % |
| 400 g/ha | 4.107 | 400 g/ha | 15 % |
| 400 g/ha | 4.107 | 200 g/ha | 45 % |
| 200 g/ha | 4.107 | 200 g/ha | 25 % |
| 200 g/ha | 4.107 | 100 g/ha | 30 % |

Eine Untergruppe der Dichloracetamide der Formel II wird durch die Verbindungen der Formel VI verkörpert

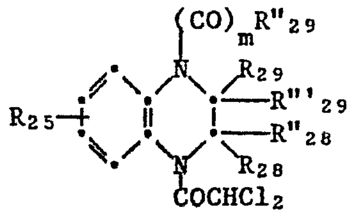

VI

worin

m Null oder Zahl 1

$R_{25}$ Wasserstoff, $C_1$-$C_4$-Alkyl, unsubstituiert oder substituiert durch Hydroxy, $C_1$-$C_4$-Alkoxy oder Halogen,

$R_{28}$ und $R'''_{29}$ Wasserstoff, $C_1$-$C_4$-Alkyl unsubstituiert oder substituiert durch Halogen, Cyan oder einen Rest $-COA_3$; $C_2$-$C_4$-Alkenyl, $C_2$-$C_4$-Alkinyl, Cyano oder einen Rest $-COA_2$;

$R_{29}$, $R''_{29}$ und $R''_{28}$ sind unabhängig voneinander je Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

$R_{28}$ und $R''_{28}$ oder $R_{29}$ und $R'''_{29}$ zusammen mit dem Kohlenstoffatom auch die Oxogruppe $>C=O$

$A_2$ $C_1$-$C_4$-Alkyl, $C_2$-$C_4$-Alkenyl, $-N(R'''_{12})(R'''_{13})$,

$A_3$ $C_1$-$C_4$-Alkyl, $C_2$-$C_4$-Alkenyl oder einen Rest $-COR''_5$ oder $-N(R''_{12})(R''_{13})$,

$R''_5$ $C_1$-$C_4$-Alkyl, $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyano oder Nitro substituiert ist,

$R'''_{12}$ und $R'''_{13}$ unabhängig voneinander je Wasserstoff, $C_1$-$C_8$-Alkyl, $C_3$-$C_8$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist

bedeuten.

Solche Verbindungen sind zum Teil in der schweizerischen Patentanmeldung Nr. 3 701/84-1 beschrieben oder sie können nach bekannten Methoden hergestellt werden. Folgende Dichloracetamide eignen sich erfindungsgemäss als Antidote zum Sulfonylharnstoff der Formel I besonders gut.

Tabelle 5

| Nr. | $R_{25}$ | $R_{28}$ | $R''_{28}$ | $R_{29}$ | $R'''_{29}$ | $R''_{29}$ | m |
|---|---|---|---|---|---|---|---|
| 5.1 | H | $CH_3$ | | H | | $CHCl_2$ | 1 |
| 5.2 | H | H | | H | | $CHCl_2$ | 1 |
| 5.3 | H | $CH_3$ | $CH_3$ | | =O | H | 0 |
| 5.4 | H | $CH_3$ | $CH_3$ | | =O | $C_2H_5$ | 1 |
| 5.5 | 6,7-$(CH_3)_2$ | $CH_3$ | $CH_3$ | | =O | $-CHCl_2$ | 1 |
| 5.6 | H | $CH_3$ | $CH_3$ | | =O | H | 1 |

 

Die Fähigkeit der Verbindungen der Formel VI, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 5a zusammengefasst.

Tabelle 5a

| Herbizid Aufwandmenge | Antidote No. | Aufwandmenge | relative Schutzwirkung |
|---|---|---|---|
| 400 g/ha | 5.1 | 400 g/ha | 70 % |
| 400 g/ha | 5.1 | 200 g/ha | 45 % |
| 200 g/ha | 5.1 | 100 g/ha | 25 % |
| 400 g/ha | 5.2 | 400 g/ha | 35 % |
| 400 g/ha | 5.2 | 200 g/ha | 35 % |
| 200 g/ha | 5.2 | 200 g/ha | 35 % |
| 200 g/ha | 5.2 | 100 g/ha | 35 % |
| 400 g/ha | 5.3 | 200 g/ha | 30 % |
| 200 g/ha | 5.3 | 200 g/ha | 20 % |
| 200 g/ha | 5.3 | 100 g/ha | 5 % |
| 400 g/ha | 5.4 | 400 g/ha | 35 % |
| 400 g/ha | 5.4 | 200 g/ha | 35 % |
| 200 g/ha | 5.4 | 200 g/ha | 25 % |
| 200 g/ha | 5.4 | 100 g/ha | 5 % |
| 400 g/ha | 5.5 | 400 g/ha | 15 % |
| 400 g/ha | 5.5 | 200 g/ha | 15 % |
| 200 g/ha | 5.5 | 200 g/ha | 30 % |
| 200 g/ha | 5.5 | 100 g/ha | 15 % |
| 400 g/ha | 5.6 | 400 g/ha | 30 % |
| 400 g/ha | 5.6 | 200 g/ha | 30 % |
| 200 g/ha | 5.6 | 200 g/ha | 25 % |
| 200 g/ha | 5.6 | 100 g/ha | 30 % |

Eine Untergruppe der Dichloracetamide der Formel II wird durch die Verbindungen der Formel VII verkörpert

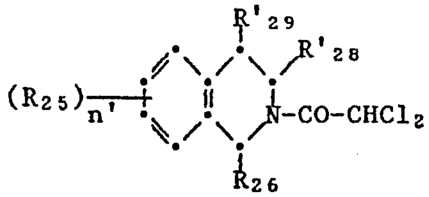

VII

worin n Null bis 2, n' Null bis 3,

$R_{25}$ Wasserstoff, $C_1$-$C_4$-Alkyl, unsubstituiert oder substituiert durch Hydroxy, $C_1$-$C_4$-Alkoxy oder Halogen, $C_1$-$C_4$-Alkyl-$S(O)_n$-, Phenyl-$S(O)_n$- durch Halogen, Methyl, Methoxy substituiertes Phenyl-$S(O)_n$-

$R_{26}$ Wasserstoff, $C_1$-$C_6$-Alkyl, $C_3$-$C_6$-Alkenyl $C_3$-$C_6$-Cycloalkyl, Phenyl oder $C_1$-$C_4$-Aralkyl bedeutet wobei der Phenylkern unsubstituiert oder durch Halogen, $C_1$-$C_4$-Alkyl, $C_1$-$C_6$-Halogenalkyl, $C_1$-$C_6$-Alkoxy, $C_1$-$C_6$-Halogenalkoxy, Methylthio, Cyan oder Nitro substituiert ist,

$R'_{28}$ Wasserstoff, $C_1$-$C_6$-Alkyl unsubstituiert oder substituiert durch Halogen, Cyan oder einen Rest $-COA_3$;

$C_3$-$C_6$-Alkenyl, $C_3$-$C_6$-Alkinyl, $C_2$-$C_6$-Alkoxyalkyl, Cyano oder $-COA_2$,

$R'_{29}$ Wasserstoff, $C_1$-$C_6$-Alkyl, $C_1$-$C_6$-Halogenalkyl, $C_3$-$C_6$-Alkenyl, $C_3$-$C_6$-Alkinyl oder $C_2$-$C_6$-Alkoxyalkyl,

$A_2$ $C_1$-$C_4$-Alkyl, $C_2$-$C_4$-Alkenyl, $-NR''_{12}R''_{13}$

$A_3$ $C_1$-$C_4$-Alkyl, $C_2$-$C_4$-Alkenyl oder einen Rest $-OR''_5$ oder $-NR''_{13}R''_{14}$,

$R''_5$ $C_1$-$C_4$-Alkyl, $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyano oder Nitro, substituiert ist,

$R''_{13}$ und $R''_{14}$ unabhängig voneinander je Wasserstoff, $C_1$-$C_8$-Alkyl, $C_3$-$C_8$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sein kann,

bedeuten.

Die Verbindung 6.1 der Tabelle 6 ist in der Patentschrift EP-A-6542 bekannt geworden. Andere Beispiele der Tabelle 6 sind neu und bilden ebenfalls einen Gegenstand dieser Erfindung.Sie können nach bekannten Methoden aus Aminen der Formel VIIa

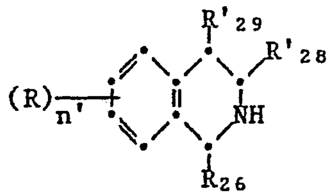

VIIa

und

Ac-$COCHCl_2$

wobei Ac Halogen oder $-O-\overset{O}{\overset{\|}{C}}-CHCl_2$, bedeutet, hergestellt werden.

Die Amine der Formel VIIa sind ihrerseits teilweise bekannt oder können nach bekannten Methoden hergestellt werden, die in Fachschriften wie z.B. Chem. Ber. <u>91</u>, 1515 (1958); Y.org.Chem. <u>16</u>, 1917 (1961) beschrieben wurden.

Folgende Dichloracetamide der Tabelle 6 eignen sich erfindungsgemäss als Antidote zum Sulfonylharnstoff der Formel I besonders gut.

<u>Tabelle 6</u>

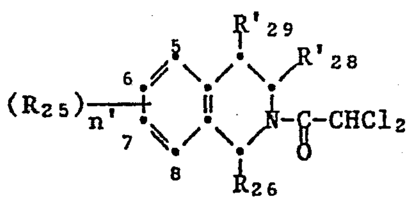

VII

| Nr. | $R_{25}$ | $R_{26}$ | $R'_{28}$ | $R'_{29}$ | Phys. Daten (Smp) |
|---|---|---|---|---|---|
| 6.1 | H | H | H | H | |
| 6.2 | | $CH_3$ | H | H | 74-77°C |
| 6.3 | H | $C_2H_5$ | H | H | Oel |
| 6.4 | H | $i-C_3H_7$ | H | H | Oel |
| 6.5 | H | $CH_3$ | $CH_3$ | H | |
| 6.6 | $6-OCH_3$<br>$7-OCH_3$ | H | H | H | 122-123°C |
| 6.7 | 7-Cl,8-Cl | H | H | H | |
| 6.8 | H | H | $C_2H_5$ | H | |
| 6.9 | H | H | H | $C_2H_5$ | |

Die Fähigkeit der Verbindungen der Formel VII, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 6a zusammengefasst.

Tabelle 6a

| Herbizid Aufwandmenge | Antidote No. | Aufwandmenge | relative Schutzwirkung |
|---|---|---|---|
| 400 g/ha | 6.1 | 400 g/ha | 45 % |
| 400 g/ha | 6.1 | 200 g/ha | 35 % |
| 400 g/ha | 6.1 | 100 g/ha | 30 % |
| 200 g/ha | 6.1 | 200 g/ha | 45 % |
| 200 g/ha | 6.1 | 100 g/ha | 35 % |
| 200 g/ha | 6.1 | 50 g/ha | 30 % |
| 200 g/ha | 6.1 | 25 g/ha | 25 % |
| 100 g/ha | 6.1 | 100 g/ha | 10 % |
| 100 g/ha | 6.1 | 50 g/ha | 5 % |
| 100 g/ha | 6.1 | 25 g/ha | 10 % |
| 100 g/ha | 6.1 | 12.5 g/ha | 20 % |
| 400 g/ha | 6.2 | 400 g/ha | 35 % |
| 400 g/ha | 6.2 | 200 g/ha | 40 % |
| 200 g/ha | 6.2 | 200 g/ha | 25 % |
| 200 g/ha | 6.2 | 100 g/ha | 25 % |
| 400 g/ha | 6.3 | 400 g/ha | 35 % |
| 400 g/ha | 6.3 | 200 g/ha | 45 % |
| 200 g/ha | 6.3 | 200 g/ha | 35 % |
| 200 g/ha | 6.3 | 100 g/ha | 35 % |
| 400 g/ha | 6.4 | 400 g/ha | 10 % |
| 400 g/ha | 6.4 | 200 g/ha | 20 % |
| 200 g/ha | 6.4 | 200 g/ha | 25 % |
| 200 g/ha | 6.4 | 100 g/ha | 25 % |
| 400 g/ha | 6.5 | 400 g/ha | 40 % |
| 200 g/ha | 6.5 | 200 g/ha | 25 % |
| 200 g/ha | 6.5 | 100 g/ha | 20 % |

Eine Untergruppe der Dichloracetamide der Formel II wird durch die Verbindungen der Formel VIII verkörpert

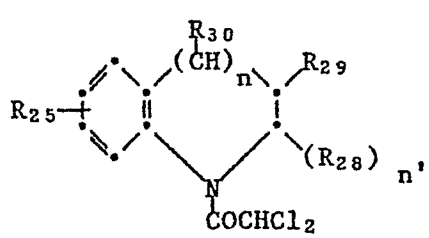

(VIII)

worin

n' Null oder eins oder zwei

n Null oder eine Zahl von 1 bis 3

$R_{25}$ Wasserstoff, $C_1$-$C_4$-Alkyl, unsubstituiert oder substituiert durch Hydroxy, $C_1$-$C_4$-Alkyl oder Halogen,

$R_{28}$ Wasserstoff, $C_1$-$C_4$-Alkyl, unsubstituiert oder substituiert durch Halogen, Cyan oder einen Rest $-COA_3$; $C_2$-$C_4$-Alkenyl oder $C_2$-$C_4$-Alkinyl;

$R_{29}$ Wasserstoff, $C_1$-$C_4$-Halogenalkyl, $C_2$-$C_4$-Alkenyl, $C_2$-$C_4$-Alkinyl oder $C_2$-$C_4$-Alkoxyalkyl;

$R_{30}$ dasselbe wie $R_{28}$ aber unabhängig davon,

$A_3$ $C_1$-$C_4$-Alkyl, $C_2$-$C_4$-Alkenyl, einen Rest $-OR''_5$ oder $-NR''_{12}$, $R''_{13}$,

$R'''_5$ $C_1$-$C_4$-Alkyl, $C_1$-$C_4$-Aralky, wobei der Phenylkern unsubstituiert oder durch $C_1$-$C_4$-Alkyl, $C_1$-$C_4$-Alkoxy, Halogen, $C_1$-$C_4$-Halogenalkyl Cyan oder Nitro substituiert sein kann; wenn n Null ist kann $R_5$ auch Wasserstoff bedeuten,

$R''_{12}$ und $R''_{13}$ unabhängig voneinander je Wasserstoff, $C_1$-$C_8$-Alkyl, $C_3$-$C_6$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sein kann,

bedeuten.

Solche Verbindungen sind zum Teil in den veröffentlichten Patentanmeldungen resp. Patentschriften EP-A 6 540 und EP-A 23 306 bekannt geworden oder sie können nach bekannten Methoden hergestellt werden. Folgende Dichloracetamide eignen sich erfindungsgemäss als Antidote zum Sulfonylharnstoff der Formel I besonders gut:

Tabelle 7

| Nr. | $R_{28}$ | $R_{29}$ | $R_{25}$ | m | $R_{30}$ | n |
|---|---|---|---|---|---|---|
| 7.1 | $CH_3$ | H | H | 2 | $CH_3$ | 1 |
| 7.2 | $CH_3$ | $CH_3$ | H | 1 | - | 0 |
| 7.3 | H | H | H | 2 | H | 1 |
| 7.4 | $CH_3$ | H | H | 1 | H | 1 |
| 7.5 | $CH_3$ | H | H | 1 | - | 0 |
| 7.6 | $CH_3$ | H | 6-$CH_3$ | 1 | H | 1 |
| 7.7 | H | H | 6-$CH_2$-$SO_2$- 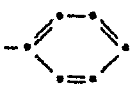 -Cl | 1 | H | 0 |
| 7.8 | H | H | 6-$CH_2$-$SO_2$- 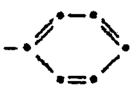 -Cl | 1 | H | 1 |
| 7.9 | H | H | 5-Cl | 1 | H | 0 |
| 7.10 | H | H | H | 1 | H | 0 |

Die Fähigkeit der Verbindungen der Formel VIII, junge Maispflanzen vor der phytotoxischen Wirkung von N-(2-Methoxycarbonylphenyl-sulfonyl)-N'-(4',6'-bis-difluormethoxypyrimidin-2-yl)-harnstoff zu schützen, wurde gemäss Beispiel 1 geprüft. Die Resultate sind in der Tabelle 7a zusammengefasst.

Tabelle 7a

| Herbizid Aufwandmenge | Antidote No. | Aufwandmenge | relative Schutzwirkung |
|---|---|---|---|
| 400 g/ha | 7.3 | 400 g/ha | 45 % |
| 400 g/ha | 7.3 | 200 g/ha | 45 % |
| 200 g/ha | 7.3 | 200 g/ha | 35 % |
| 200 g/ha | 7.3 | 100 g/ha | 40 % |
| 400 g/ha | 7.4 | 400 g/ha | 35 % |
| 400 g/ha | 7.4 | 200 g/ha | 25 % |
| 200 g/ha | 7.4 | 200 g/ha | 20 % |
| 200 g/ha | 7.4 | 100 g/ha | 25 % |
| 400 g/ha | 7.5 | 400 g/ha | 55 % |
| 400 g/ha | 7.5 | 200 g/ha | 30 % |
| 200 g/ha | 7.5 | 200 g/ha | 30 % |
| 200 g/ha | 7.5 | 100 g/ha | 40 % |
| 400 g/ha | 7.7 | 200 g/ha | 15 % |
| 200 g/ha | 7.7 | 200 g/ha | 20 % |
| 200 g/ha | 7.7 | 100 g/ha | 10 % |
| 400 g/ha | 7.8 | 400 g/ha | 10 % |
| 400 g/ha | 7.8 | 200 g/ha | 15 % |
| 200 g/ha | 7.8 | 200 g/ha | 20 % |
| 200 g/ha | 7.8 | 100 g/ha | 30 % |
| 400 g/ha | 7.9 | 400 g/ha | 25 % |
| 400 g/ha | 7.10 | 400 g/ha | 38 % |
| 400 g/ha | 7.10 | 200 g/ha | 13 % |
| 200 g/ha | 7.10 | 200 g/ha | 13 % |
| 200 g/ha | 7.10 | 100 g/ha | 13 % |

Eine Untergruppe der Dichloracetamide der Formel II wird durch die Verbindungen der Formel IX verkörpert

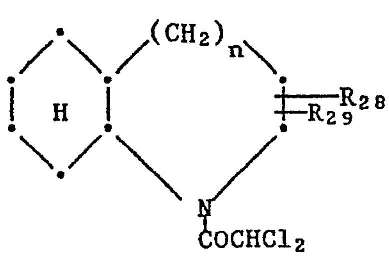

(IX)

worin n Null eins, zwei, oder drei

$R_{28}$ Wasserstoff, $C_1$-$C_4$-Alkyl unsubstituiert oder substituiert durch Halogen, Cyan oder einen Rest -CO-$A_3$,
$C_2$-$C_4$-Alkenyl oder $C_2$-$C_4$-Alkinyl, $C_2$-$C_4$-Alkoxyalkyl oder Cyan;

$R_{29}$ Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl;

$A_3$ $C_1$-$C_4$-Alkyl, $C_2$-$C_4$-Alkenyl, einen Rest -OR"$_5$ oder -NR"$_{12}$, R"$_{13}$,

R"$_5$ $C_1$-$C_4$-Alkyl, $C_1$-$C_4$-Aralkyl wobei der Phenylkern unsubstituiert oder durch $C_1$-$C_4$-Alkyl, $C_1$-$C_4$-Alkoxy, Halogen, $C_1$-$C_4$-Halogenalkyl, Cyan oder Nitro substituiert sein kann; wenn m Null ist, kann $R_5$ auch Wasserstoff sein;

R"$_{12}$ und R"$_{13}$ unabhängig voneinander je Wasserstoff, $C_1$-$C_8$-Alkyl, $C_3$-$C_6$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sein kann,
bedeuten.

Solche Verbindungen sind zum Teil aus der veröffentlichten Patentanmeldung DE-A 2,828,265 bekannt geworden oder sie können nach bekannten Methoden hergestellt werden. Folgende Dichloracetamide eignen sich erfindungsgemäss als Antidote zum Sulfonylharnstoff der Formel I besonders gut.

Tabelle 8

| Nr. | $R_{28}$ | $R_{29}$ | m |
|---|---|---|---|
| 8.1 | 2-$CH_3$ | 3-$CH_3$ | 0 |
| 8.2 | 2-$CH_3$ | H | 1 |

Die Fähigkeit der Verbindungen 8.1 und 8.2 junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 8a zusammengefasst.

Tabelle 8a

| Herbizid Aufwandmenge | Antidote No. | Aufwandmenge | relative Schutzwirkung |
|---|---|---|---|
| 400 g/ha | 8.1 | 400 g/ha | 30 % |
| 400 g/ha | 8.2 | 400 g/ha | 45 % |
| 400 g/ha | 8.2 | 200 g/ha | 50 % |

Eine Untergruppe der Dichloracetamide der Formel II wird durch die Verbindungen der Formel X verkörpert

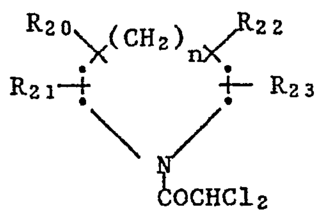

(X)

n Null eins, zwei, oder drei

R"$_{20}$ einen Rest -COOR'$_5$, COR'$_6$ der auch über eine $C_1$-$C_4$-Alkylenbrücke gebunden sein kann,

$R_{21}$, $R_{22}$ und $R_{23}$ unabhängig voneinander je Wasserstoff oder $C_1$-$C_4$-Alkyl

R'$_5$ einen $C_1$-$C_4$-Alkylrest; einen $C_1$-$C_4$-Aralkylrest der im Phenylkern unsubstituiert oder durch Halogen, $C_1$-$C_4$-Alkyl, $C_1$-$C_4$-Alkoxy, $C_1$-$C_4$-Halogenalkyl, Cyan oder Nitro substituiert ist; wenn m Null ist, kann R'$_5$ auch Wasserstoff sein,

R'$_6$ Wasserstoff, einen $C_1$-$C_4$-Alkyl, $C_1$-$C_4$-Halogenalkyl- oder $C_2$-$C_8$-Alkoxyalkylrest; Phenyl oder $C_1$-$C_4$-Aralkyl wobei der Phenylrest unsubstituiert oder durch Halogen, Cyan, $C_1$-$C_4$-Alkyl, $C_1$-$C_4$-Halogenalkyl substituiert sein kann; einen $C_2$-$C_4$-Alkenylrest oder ein Aminorest -$NR_{12}R_{13}$, wobei

$R_{12}$ und $R_{13}$ unabhängig voneinander je Wasserstoff, $C_1$-$C_8$-Alkyl, $C_3$-$C_6$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sein kann,

bedeuten.

Solche Verbindungen sind zum Teil aus den veröffentlichten Patentanmeldungen resp. Patentschriften DE-A-2,828,331, EP-A-23 308 sowie USP 4 134 070 bekannt geworden. Folgende Dichloracetamide eignen sich erfindungsgemäss als Antidote zum Sulfonylharnstoff der Formel I besonders gut:

Tabelle 9

| Nr. | $R_{20}$ | $R_{21}$ | $R_{22}$ | $R_{23}$ | m |
|---|---|---|---|---|---|
| 9.1 | 2-$CH_3$ | - | - | 6-$CH_3$ | 1 |
| 9.2 | 2-$CH_3$ | 2-$CH_3$ | 6-$CH_3$ | 6-$CH_3$ | 1 |
| 9.3 | - | 3-$CH_3$ | 3-$CH_3$ | - | 1 |
| 9.4 | 2-$CH_3$ | 4-$CH_3$ | - | 6-$CH_3$ | 1 |
| 9.5 | 2-$CH_3$ | - | 5-$C_2H_5$ | - | 1 |
| 9.6 | - | 3-$CH_3$ | - | - | 1 |
| 9.7 | - | - | 4-$CH_3$ | - | 1 |
| 9.8 | - | 3-$CH_3$ | 5-$CH_3$ | - | 1 |
| 9.9 | 2-$C_2H_5$ | - | - | - | 1 |
| 9.10 | 2-$CH_3$ | - | - | - | 1 |
| 9.11 | 3-$COOC_2H_5$ | - | - | - | 1 |
| 9.12 | - | - | - | - | 2 |
| 9.13 | - | - | - | - | 3 |
| 9.14 | 3-$CH_3$ | 3-$CH_3$ | 5-$CH_3$ | - | 2 |

Die Fähigkeit der Verbindungen der Formel X, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 9a zusammengefasst.

Tabelle 9a

| Herbizid Aufwandmenge | Antidote No. | Aufwandmenge | relative Schutzwirkung |
|---|---|---|---|
| 400 g/ha | 9.1 | 400 g/ha | 20 % |
| 400 g/ha | 9.1 | 200 g/ha | 35 % |
| 200 g/ha | 9.1 | 200 g/ha | 5 % |
| 200 g/ha | 9.1 | 100 g/ha | 25 % |
| 400 g/ha | 9.2 | 400 g/ha | 10 % |
| 400 g/ha | 9.2 | 200 g/ha | 5 % |
| 200 g/ha | 9.2 | 200 g/ha | 30 % |
| 200 g/ha | 9.2 | 100 g/ha | 20 % |
| 400 g/ha | 9.3 | 400 g/ha | 40 % |
| 400 g/ha | 9.3 | 200 g/ha | 35 % |
| 200 g/ha | 9.3 | 200 g/ha | 25 % |
| 200 g/ha | 9.3 | 100 g/ha | 30 % |
| 400 g/ha | 9.4 | 400 g/ha | 60 % |
| 400 g/ha | 9.4 | 200 g/ha | 60 % |
| 200 g/ha | 9.4 | 200 g/ha | 20 % |
| 200 g/ha | 9.4 | 100 g/ha | 15 % |
| 400 g/ha | 9.5 | 400 g/ha | 50 % |
| 400 g/ha | 9.5 | 200 g/ha | 45 % |
| 200 g/ha | 9.5 | 200 g/ha | 5 % |
| 200 g/ha | 9.5 | 100 g/ha | 5 % |
| 400 g/ha | 9.6 | 400 g/ha | 35 % |
| 400 g/ha | 9.6 | 200 g/ha | 40 % |
| 200 g/ha | 9.6 | 200 g/ha | 35 % |
| 200 g/ha | 9.6 | 100 g/ha | 30 % |
| 400 g/ha | 9.7 | 400 g/ha | 45 % |
| 400 g/ha | 9.7 | 200 g/ha | 45 % |
| 200 g/ha | 9.7 | 200 g/ha | 30 % |
| 200 g/ha | 9.7 | 100 g/ha | 15 % |
| 400 g/ha | 9.8 | 400 g/ha | 10 % |
| 400 g/ha | 9.8 | 200 g/ha | 45 % |
| 200 g/ha | 9.8 | 200 g/ha | 5 % |
| 200 g/ha | 9.8 | 100 g/ha | 25 % |
| 400 g/ha | 9.9 | 400 g/ha | 55 % |
| 400 g/ha | 9.9 | 200 g/ha | 60 % |
| 200 g/ha | 9.9 | 200 g/ha | 25 % |
| 200 g/ha | 9.9 | 100 g/ha | 10 % |
| 400 g/ha | 9.10 | 400 g/ha | 50 % |
| 400 g/ha | 9.10 | 200 g/ha | 55 % |
| 200 g/ha | 9.10 | 200 g/ha | 10 % |
| 200 g/ha | 9.10 | 100 g/ha | 25 % |

Tabelle 9a (Fortsetzung)

| Herbizid Aufwandmenge | Antidote No. | Aufwandmenge | relative Schutzwirkung |
|---|---|---|---|
| 400 g/ha | 9.12 | 400 g/ha | 55 % |
| 400 g/ha | 9.12 | 200 g/ha | 45 % |
| 200 g/ha | 9.12 | 200 g/ha | 30 % |
| 200 g/ha | 9.12 | 100 g/ha | 30 % |
| 400 g/ha | 9.13 | 400 g/ha | 35 % |
| 400 g/ha | 9.13 | 200 g/ha | 40 % |
| 200 g/ha | 9.13 | 200 g/ha | 45 % |
| 200 g/ha | 9.13 | 100 g/ha | 30 % |
| 400 g/ha | 9.14 | 400 g/ha | 40 % |
| 400 g/ha | 9.14 | 200 g/ha | 30 % |
| 200 g/ha | 9.14 | 200 g/ha | 20 % |
| 200 g/ha | 9.14 | 100 g/ha | 20 % |

Eine Untergruppe der Dichloracetamide der Formel II wird durch die Verbindungen der Formel XI verkörpert

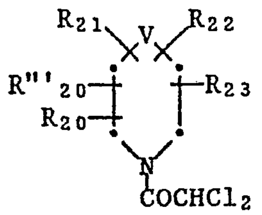

(XI)

worin

$R_{20}$ Wasserstoff oder $C_1$-$C_4$-Alkyl

$R'''_{20}$ Phenyl, durch Halogen, $C_1$-$C_4$-Alkyl, $C_1$-$C_4$-Alkoxy, $C_1$-$C_4$-Alkylthio substituiertes Phenyl,

$R_{21}$ Wasserstoff oder $C_1$-$C_4$-Alkyl

$R_{22}$ Wasserstoff oder $C_1$-$C_4$-Alkyl

$R_{23}$ Wasserstoff oder $C_1$-$C_4$-Alkyl bedeutet,

V Sauerstoff, $>CO$ , $>C(OR''_{25})OR''_{26}$, $>C(R''_{25})R''_{26}$

oder $S(O)_{n'}$

n' Null 1, oder 2

$R''_{25}$ eine $C_4$-$C_6$-Alkylenbrücke, die geradkettig oder verzweigt sein kann

$R''_{26}$ dasselbe wie $R_{25}$ bedeuten.

Diese Verbindungen sind aus den veröffentlichten Patentanmeldungen resp. der Patentschriften EP-A 23308, EP-A 65 189, DE 2'938'155 bekannt oder sie können nach bekannten Methoden hergestellt werden. Folgende Dichloracetamide eignen sich erfindungsgemäss als Antidote zum Sulfonylharnstoff der Formel I besonders gut:

Tabelle 10

| Nr. | $R_{20}$ | $R_{21}$ | $R'''_{20}$ | $R_{22}$ | $R_{23}$ | V |
|---|---|---|---|---|---|---|
| 10.1 | H | H | H | H | H | O |
| 10.2 | H | 3-$CH_3$ | H | 5-$CH_3$ | H | O |
| 10.3 | 2-$CH_3$ | 2-$CH_3$ | H | 6-$CH_3$ | 6-$CH_3$ | CO |
| 10.4 | 2-$CH_3$ | 2-$CH_3$ | H | 6-$CH_3$ | 6-$CH_3$ | $SO_2$ |
| 10.5 | H | H | H | H | H | 1,4-Dioxolan-2-yl |
| 10.6 | H | H | H | H | H | CO |
| 10.7 | 2,3-$(CH_2)_4$ | - | H | H | H | O |
| 10.8 | H | H | 2-Phenyl | H | H | O |
| 10.9 | 3-$CH_3$ | H | 2-Phenyl | H | H | O |
| 10.10 | H | H | | H | H | $C(OCH_3)_2$ |
| 10.11 | H | H | | H | H | $C(OC_2H_5)_2$ |

Die Fähigkeit der Verbindungen der Formel XI, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 10a zusammengefasst.

Tabelle 10a

| Herbizid Aufwandmenge | Antidote No. | Aufwandmenge | relative Schutzwirkung |
|---|---|---|---|
| 400 g/ha | 10.1 | 400 g/ha | 65 % |
| 400 g/ha | 10.1 | 200 g/ha | 40 % |
| 200 g/ha | 10.1 | 200 g/ha | 20 % |
| 200 g/ha | 10.1 | 100 g/ha | 15 % |
| 400 g/ha | 10.2 | 400 g/ha | 10 % |
| 400 g/ha | 10.2 | 200 g/ha | 5 % |
| 200 g/ha | 10.2 | 200 g/ha | 30 % |
| 200 g/ha | 10.2 | 100 g/ha | 20 % |
| 400 g/ha | 10.3 | 200 g/ha | 5 % |
| 200 g/ha | 10.3 | 200 g/ha | 25 % |
| 400 g/ha | 10.4 | 400 g/ha | 25 % |
| 400 g/ha | 10.4 | 200 g/ha | 10 % |
| 200 g/ha | 10.4 | 100 g/ha | 5 % |
| 400 g/ha | 10.5 | 400 g/ha | 20 % |
| 400 g/ha | 10.5 | 200 g/ha | 35 % |
| 200 g/ha | 10.5 | 200 g/ha | 20 % |
| 200 g/ha | 10.5 | 100 g/ha | 5 % |
| 400 g/ha | 10.6 | 400 g/ha | 35 % |
| 400 g/ha | 10.6 | 200 g/ha | 40 % |
| 200 g/ha | 10.6 | 100 g/ha | 15 % |
| 400 g/ha | 10.7 | 400 g/ha | 40 % |
| 400 g/ha | 10.7 | 200 g/ha | 15 % |
| 400 g/ha | 10.8 | 400 g/ha | 20 % |
| 400 g/ha | 10.8 | 200 g/ha | 40 % |
| 200 g/ha | 10.8 | 200 g/ha | 20 % |
| 200 g/ha | 10.8 | 100 g/ha | 35 % |
| 400 g/ha | 10.9 | 400 g/ha | 5 % |
| 400 g/ha | 10.9 | 200 g/ha | 30 % |
| 200 g/ha | 10.9 | 200 g/ha | 10 % |
| 200 g/ha | 10.9 | 100 g/ha | 10 % |

Eine Untergruppe der Dichloracetamide der Formel II wird durch die Verbindungen der Formel XII verkörpert

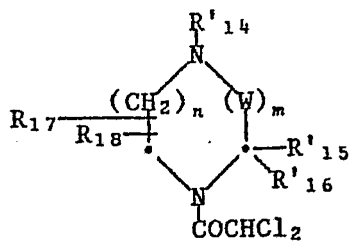

(XII)

worin

m Null oder 1

n Null, 1, 2 oder 3

$R''_6$ Wasserstoff, $C_1$-$C_4$-Alkyl, durch Halogen, $C_1$-$C_4$-Alkoxy substituiertes $C_1$-$C_4$-Alkyl, $NR_{12}R_{13}$, $C_2$-$C_4$-Alkenyl, Phenyl, $C_1$-$C_4$-Aralkyl wobei der Phenylkern durch Halogen, $C_1$-$C_4$-Alkyl, $C_1$-$C_4$-Alkoxy, $C_1$-$C_4$-Alkylthio substituiert sein kann; $C_1$-$C_8$-Alkoxy,

$R_{12}$ Wasserstoff, $C_1$-$C_8$-Alkyl, $C_3$-$C_6$-Alkenyl,

$R_{13}$ Wasserstoff, $C_1$-$C_8$-Alkyl, $C_3$-$C_6$-Alkenyl, Phenyl, $C_1$-$C_4$-Aralkyl wobei Phenyl durch Halogen, $C_1$-$C_4$-Alkyl, $C_1$-$C_4$-Alkoxy und Alkylthio substituiert sein kann,

$R'_{14}$ Wasserstoff oder $C_1$-$C_4$-Alkyl, Phenyl, $C_1$-$C_4$-Aralkyl wobei der Phenylkern durch Halogen, $C_1$-$C_4$-Alkyl, $C_1$-$C_4$-Alkoxy und Alkylthio substituiert sein kann; -CO-$R''_6$

$R'_{15}$ Wasserstoff oder $C_1$-$C_4$-Alkyl

$R'_{16}$ Wasserstoff oder $C_1$-$C_4$-Alkyl

$R_{17}$ Wasserstoff, $C_1$-$C_4$-Alkyl

$R_{18}$ Wasserstoff, $C_1$-$C_4$-Alkyl

$R_{17}$ und $R_{18}$ zusammen ein $C_3$-$C_5$-Alkylenkette

W einen Rest $>CO$, $>C(OR_{24})_2$ oder $>CR_{25}R_{26}$,

$R_{24}$ $C_1$-$C_4$-Alkyl,

$R_{25}$ Wasserstoff, $C_1$-$C_4$-Alkyl unsubstituiert oder substituiert durch Hydroxy, $C_1$-$C_4$-Alkoxy oder Halogen,

$R_{26}$ Wasserstoff, $C_1$-$C_4$-Alkyl, $C_5$-$C_8$-Cycloalkyl, Phenyl oder $C_1$-$C_4$-Aralkyl wobei der Phenylkern unsubstituiert oder substituiert ist durch Halogen Nitro, Cyan, $C_1$-$C_4$-Alkyl, $C_1$-$C_4$-Alkoxy,

Methylthio, $C_1$-$C_4$-Halogenalkoxy, Carboxy, $C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-Alkylcarbamoyl, $C_1$-$C_4$-Alkylsulfonyl, $C_1$-$C_4$-Alkylsulfonyl, Sulfamoyl oder Di-$C_1$-$C_4$-Alkylsulfamoyl bedeuten.

Diese Verbindungen sind aus der Patentschrift EP-A 23287, EP-A 23305 bekannt oder sie können nach bekannten Methoden hergestellt werden. Folgende Dichloracetamide eignen sich erfindungsgemäss als Antidote zum Sulfonylharnstoff der Formel I besonders gut:

Tabelle 11

| Nr. | $R'_{15}$ | $R'_{16}$ | m | W | n | $R'_{14}$ | $R_{17}$ | $R_{18}$ |
|---|---|---|---|---|---|---|---|---|
| 11.1 | H | H | 1 | $CH_2$ | 1 | $COCHCl_2$ | H | H |
| 11.2 | H | H | 1 | $CH_2$ | 1 | $CH_3$ | H | H |
| 11.3 | H | H | 1 | $CH_2$ | 1 | Phenyl | H | H |
| 11.4 | H | H | 0 | - | 3 | $COCHCl_2$ | H | H |
| 11.5 | H | H | 1 | $CH_2$ | 1 | 2-Methoxy-phenyl | H | H |
| 11.6 | H | H | 1 | $CH_2$ | 1 | $CON(C_2H_5)_2$ | H | H |
| 11.7 | H | H | 1 | $CH_2$ | 1 | 4-Fluor-phenyl | H | H |
| 11.8 | H | H | 1 | $CH_2$ | 1 | 3,4-Dichlor-phenyl | H | H |
| 11.9 | H | H | 1 | $CH_2$ | 1 | 3-Chlorphenyl | H | H |
| 11.10 | H | H | 1 | $CH_2$ | 1 | Benzyl | H | H |
| 11.11 | H | H | 1 | $CH_2$ | 1 | 4-Chlorphenyl | H | H |
| 11.12 | H | H | 1 | $CHCH_3$ | 1 | 3-Methoxy-phenyl | H | H |
| 11.13 | H | H | 1 | $CHCH_3$ | 1 | 4-Methoxy-phenyl | H | H |
| 11.14 | H | H | 1 | $CHCH_3$ | 1 | 3-$CF_3$-phenyl | H | H |
| 11.15 | H | H | 1 | CH | 1 | $COOCH_3$ | H | H |
| 11.16 | H | H | 1 | $CH_2$ | 1 | 2-Pyridyl | H | H |
| 11.17 | H | H | 1 | $CH_2$ | 1 | 3-Pyridyl | H | H |
| 11.18 | H | H | 1 | CH | 1 | Benzoyl | H | H |
| 11.19 | H | H | 1 | $CH_2$ | 1 | Phenylethyl | H | H |
| 11.20 | H | H | 1 | $CH_2$ | 1 | 3-Chlorphenyl-ethyl | H | H |
| 11.21 | H | H | 1 | $CH_2$ | 1 | 4-Chlorphenyl-ethyl | H | H |

Tabelle 11 (Fortsetzung)

| Nr. | $R_a$ | | | | $R_b$ | | | |
|---|---|---|---|---|---|---|---|---|
| 11.22 | H | H | 1 | $CH_2$ | 1 | 4-Chlorphenyl-propyl | H | H |
| 11.23 | H | H | 1 | $CH_2$ | 1 | Phenylpropyl | H | H |
| 11.24 | H | H | 1 | $CH_2$ | 1 | 4-Methoxy-phenylethyl | H | H |
| 11.25 | H | H | 1 | $CH_2$ | 1 | Bornan-1-yl-oxycarbonyl | H | H |
| 11.26 | H | H | 1 | $CH_2$ | 1 | $COCF_2$ | H | H |

| Nr. | $R'_{15}$ | $R'_{16}$ | m | W | n | $R'_{14}$ | $R_{17}$ | $R_{18}$ |
|---|---|---|---|---|---|---|---|---|
| 11.27 | H | H | 1 | CO | 0 | H | H | $2\text{-}CH(CH_3)_2$ |
| 11.28 | $CH_3$ | $CH_3$ | 1 | CO | 1 | H | H | |
| 11.29 | $CH_3$ | H | 1 | CO | 1 | H | $5\text{-}CH_3$ | $6\text{-}CH_3$ |
| 11.30 | $CH_3$ | H | 1 | CO | 1 | H | H | H |
| 11.31 | $CH_3$ | H | 1 | CO | 1 | H | $5,6\text{-}(CH_2)_4$ | |
| 11.32 | $CH_3$ | H | 1 | CO | 1 | H | H | $6\text{-}CH_3$ |
| 11.33 | H | H | 1 | CO | 3 | $COCHCl_2$ | H | H |

Die Fähigkeit der Verbindungen der Formel XII, junge Maispflanzen vor der phytotoxischen Wirkung von N-(2-Methoxycarbonylphenyl-sulfonyl)-N'-(4',6'-bis-difluormethoxypyrimidin-2-yl)-harnstoff zu schützen, wurde gemäss Beispiel 1 geprüft. Die Resultate sind in der Tabelle 11b zusammengefasst.

Tabelle 11b

| Herbizid Aufwandmenge | Antidote No. | Aufwandmenge | relative Schutzwirkung |
|---|---|---|---|
| 400 g/ha | 11.1 | 400 g/ha | 55 % |
| 400 g/ha | 11.1 | 200 g/ha | 55 % |
| 200 g/ha | 11.1 | 200 g/ha | 20 % |
| 200 g/ha | 11.1 | 100 g/ha | 30 % |
| 400 g/ha | 11.2 | 400 g/ha | 40 % |
| 400 g/ha | 11.2 | 200 g/ha | 5 % |
| 200 g/ha | 11.2 | 100 g/ha | 15 % |
| 400 g/ha | 11.3 | 400 g/ha | 45 % |
| 400 g/ha | 11.3 | 200 g/ha | 80 % |
| 200 g/ha | 11.3 | 200 g/ha | 30 % |
| 400 g/ha | 11.4 | 400 g/ha | 65 % |
| 400 g/ha | 11.4 | 200 g/ha | 65 % |
| 200 g/ha | 11.4 | 200 g/ha | 40 % |
| 200 g/ha | 11.4 | 100 g/ha | 40 % |
| 400 g/ha | 11.5 | 400 g/ha | 60 % |
| 400 g/ha | 11.5 | 200 g/ha | 55 % |
| 200 g/ha | 11.5 | 200 g/ha | 35 % |
| 200 g/ha | 11.5 | 100 g/ha | 40 % |
| 400 g/ha | 11.6 | 400 g/ha | 50 % |
| 400 g/ha | 11.6 | 200 g/ha | 60 % |
| 200 g/ha | 11.6 | 200 g/ha | 15 % |
| 200 g/ha | 11.6 | 100 g/ha | 20 % |
| 400 g/ha | 11.7 | 400 g/ha | 40 % |
| 400 g/ha | 11.7 | 200 g/ha | 10 % |
| 200 g/ha | 11.7 | 200 g/ha | 35 % |
| 200 g/ha | 11.7 | 100 g/ha | 45 % |
| 400 g/ha | 11.8 | 400 g/ha | 10 % |
| 400 g/ha | 11.8 | 200 g/ha | 10 % |
| 200 g/ha | 11.8 | 200 g/ha | 5 % |
| 200 g/ha | 11.8 | 100 g/ha | 5 % |
| 400 g/ha | 11.9 | 400 g/ha | 35 % |
| 400 g/ha | 11.9 | 200 g/ha | 35 % |
| 200 g/ha | 11.9 | 200 g/ha | 15 % |
| 200 g/ha | 11.9 | 100 g/ha | 40 % |
| 400 g/ha | 11.10 | 400 g/ha | 30 % |
| 400 g/ha | 11.10 | 200 g/ha | 35 % |
| 200 g/ha | 11.10 | 200 g/ha | 25 % |
| 200 g/ha | 11.10 | 100 g/ha | 35 % |

Tabelle 11b (Fortsetzung)

| Herbizid Aufwandmenge | Antidote No. | Aufwandmenge | relative Schutzwirkung |
|---|---|---|---|
| 400 g/ha | 11.11 | 400 g/ha | 10 % |
| 400 g/ha | 11.11 | 200 g/ha | 5 % |
| 200 g/ha | 11.11 | 200 g/ha | 5 % |
| 200 g/ha | 11.11 | 100 g/ha | 10 % |
| 400 g/ha | 11.12 | 400 g/ha | 30 % |
| 400 g/ha | 11.12 | 200 g/ha | 20 % |
| 200 g/ha | 11.12 | 200 g/ha | 10 % |
| 200 g/ha | 11.12 | 100 g/ha | 30 % |
| 200 g/ha | 11.13 | 200 g/ha | 25 % |
| 200 g/ha | 11.13 | 100 g/ha | 15 % |
| 400 g/ha | 11.14 | 400 g/ha | 15 % |
| 400 g/ha | 11.14 | 200 g/ha | 40 % |
| 200 g/ha | 11.14 | 200 g/ha | 15 % |
| 200 g/ha | 11.14 | 100 g/ha | 30 % |
| 400 g/ha | 11.15 | 200 g/ha | 38 % |
| 200 g/ha | 11.15 | 200 g/ha | 13 % |
| 400 g/ha | 11.16 | 400 g/ha | 13 % |
| 400 g/ha | 11.17 | 400 g/ha | 25 % |
| 400 g/ha | 11.19 | 400 g/ha | 25 % |
| 400 g/ha | 11.19 | 400 g/ha | 13 % |
| 400 g/ha | 11.26 | 400 g/ha | 25 % |

**Eine Untergruppe der Dichloracetamide der Formel II wird durch die Verbindungen der Formel XIII verkörpert**

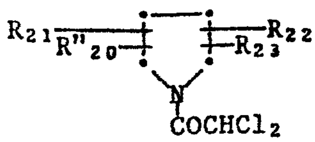

(XIII)

worin

$R''_{20}$ einen Rest $-COOR'_5$, $-COR'_6$ oder einen $C_1$-$C_4$-Alkylrest, der durch $-COOR'_5$ oder $-COR'_6$ substituiert ist,

$R_{21}$ Wasserstoff oder $C_1$-$C_4$-Alkyl,

$R_{22}$ Wasserstoff oder $C_1$-$C_4$-Alkyl,

$R_{23}$ Wasserstoff oder $C_1$-$C_4$-Alkyl,

$R'_5$ $C_1$-$C_4$-Alkyl, unsubstituiert oder ein $C_1$-$C_4$-Aralkylrest dessen Phenylkern unsubstituiert oder substituiert durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxy, $C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-alkylcarbamoyl, $C_1$-$C_4$-Alkylsulfonyl, $C_1$-$C_4$-Alkylsulfunyl, Sulfamoyl oder Di-$C_1$-$C_4$-alkylsulfamoyl,

$R'_6$ Wasserstoff, $C_1$-$C_4$-Alkyl, unsubstituiert oder substituiert durch Halogen oder $C_1$-$C_4$-Alkoxy; Phenyl oder $C_1$-$C_4$-Aralkyl, wobei der Phenylkern unsubstituiert oder durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-alkoxycarbamoyl, $C_1$-$C_4$-Alkylsulfonyl, $C_1$-$C_4$-Alkylsulfonyl, Sulfamoyl oder Di-$C_1$-$C_4$-alkylsulfamoyl, einen Rest $-NR_{12}R_{13}$ oder $C_2$-$C_4$-Alkenyl,

$R_{12}$ Wasserstoff, $C_1$-$C_8$-Alkyl, $C_3$-$C_6$-Alkenyl, Phenyl oder $C_1$-$C_4$-Aralkyl wobei der Phenylkern unsubstituiert oder wie oben angegeben substituiert sein kann,

$R_{13}$ dasselbe wie $R_{12}$

bedeuten.

Viele dieser Verbindungen sind aus den veröffentlichten Patentanmeldungen resp. Patentschriften USP 4'021'224 und USP 4'208'203 bekannt oder können gemäss diesen offenbarten Methoden hergestellt werden. Folgende Dichloracetamide eignen sich erfindungsgemäss als Antidote zum Sulfonylharnstoff der Formel I besonders gut:

Tabelle 12

| Nr. | $R''_{20}$ | $R_{21}$ | $R_{22}$ | $R_{23}$ |
|---|---|---|---|---|
| 12.1 | H | H | H | |
| 12.2 | 2-$COOCH_3$ | H | H | |
| 12.3 | 2-Benzyl-carbamoyl | H | H | |

Die Fähigkeit der Verbindungen der Formel XIII, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 12a zusammengefasst.

Tabelle 12a

| Herbizid Aufwandmenge | Antidote No. | Aufwandmenge | relative Schutzwirkung |
|---|---|---|---|
| 400 g/ha | 12.1 | 400 g/ha | 60 % |
| 400 g/ha | 12.1 | 200 g/ha | 65 % |
| 200 g/ha | 12.1 | 200 g/ha | 20 % |
| 200 g/ha | 12.1 | 100 g/ha | 30 % |
| 400 g/ha | 12.3 | 400 g/ha | 70 % |
| 400 g/ha | 12.3 | 200 g/ha | 65 % |
| 200 g/ha | 12.3 | 200 g/ha | 15 % |
| 200 g/ha | 12.3 | 100 g/ha | 15 % |

Eine Untergruppe der Dichloracetamide der Formel II wird durch die Verbindungen der Formel XIV verkörpert

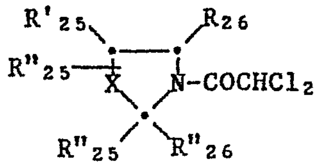

(XIV)

worin

$R'_{25}$ Wasserstoff, $C_1$-$C_4$-Alkyl, unsubstituiert oder substituiert durch Hydroxy, $C_1$-$C_4$-Alkoxy, Halogen, $C_1$-$C_4$-Alkyl-$S(O)_{n'}$-, Phenyl-S(O)n'-

$R''_{25}$ dasselbe wie $R'_{25}$

$R'_{26}$ Wasserstoff, $C_1$-$C_4$-Alkyl unsubstituiert oder substituiert durch Reste $-S(O)_{n'}R_{27}$, $-OR'_{27}$, $C_5$-$C_8$-Cycloalkyl oder durch Phenyl, welches unsubstituiert oder substituiert ist durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-alkylcarbamoyl, $C_1$-$C_4$-Alkylsulfonyl, $C_1$-$C_4$-Alkylsulfuryl, Sulfamoyl oder Di-$C_1$-$C_4$-alkylsulfamoyl,

$R''_{26}$ dasselbe wie $R'_{26}$ oder Phenyl, $C_1$-$C_4$-Aralkyl wobei die Phenylkerne durch Halogen, $C_1$-$C_4$-Alkyl, $C_1$-$C_4$-Alkoxy, $C_1$-$C_4$-Alkylthio, $C_1$-$C_4$-Halogenalkyl substituiert sein können

$R_{27}$ $C_1$-$C_4$-Alkyl, unsubstituiert oder substituiert durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl,

n' null, eins oder zwei

X Sauerstoff, Schwefel oder S(O)n',

$C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-alkylcarbamoyl, $C_1$-$C_4$-Alkylsulfonyl, $C_1$-$C_4$-Alkylsulfonyl, Sulfamoyl oder Di-$C_1$-$C_4$-alkylsulfamoyl,

$R'_{27}$ Wasserstoff oder dasselbe wie $R_{27}$

bedeuten.

Folgende Dichloracetamide eignen sich erfindungsgemäss als Antidote zum Sulfonylharnstoff der Formel I besonders gut.

Tabelle 13

| Nr. | $R'_{25}$ | $R_{26}$ | X | $R''_{25}$ | $R''_{26}$ |
|---|---|---|---|---|---|
| 13.1 | H | H | O | $CH_3$ | $CH_3$ |
| 13.2 | H | H | O | $CCl_3$ | H |
| 13.3 | H | H | O | Cyclo-hexyl | $CH_3$ |
| 13.4 | $CH_3$ | H | O | H | H |
| 13.5 | H | H | O | $C_2H_5$ | $C_2H_5$ |
| 13.6 | H | H | O | H | 2-Furyl |

| Nr. | $R'_{25}$ | $R_{26}$ | X | $R''_{25}$ | $R''_{26}$ |
|---|---|---|---|---|---|
| 13.7 | 5-$CH_3$ | H | O | $CH_3$ | $CH_3$ |
| 13.8 | H | H | O | H | $CH_2OCH_3$ |
| 13.9 | 4-$CH_3$ | $CH_2OH$ | O | $CH_3$ | $CH_3$ |
| 13.10 | H | H | O | $CH_3$ | $C_2H_5$ |
| 13.11 | 5-n-$C_3H_7$ | H | O | $CH_3$ | $CH_3$ |
| 13.12 | 5-$CH_2SO_2C_2H_5$ | H | O | $CH_3$ | $CH_3$ |
| 13.13 | H | H | S | $CH_3$ | $CH_3$ |
| 13.14 | H | H | O | $CH_3$ | i-$C_3H_7$ |
| 13.15 | H | H | O | $CH_3$ | Benzyl |
| 13.16 | H | H | O | $CH_3$ | 4-Methoxy-benzyl |
| 13.17 | H | H | O | $CH_3$ | n-$C_3H_7$ |

Die Fähigkeit der Verbindungen der Formel XIV, junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 13b zusammengefasst.

Tabelle 13b

| Herbizid Aufwandmenge | Antidote No. | Aufwandmenge | relative Schutzwirkung |
|---|---|---|---|
| 400 g/ha | 13.1 | 400 g/ha | 45 % |
| 400 g/ha | 13.1 | 200 g/ha | 25 % |
| 200 g/ha | 13.1 | 200 g/ha | 10 % |
| 200 g/ha | 13.1 | 100 g/ha | 30 % |
| 400 g/ha | 13.2 | 400 g/ha | 45 % |
| 400 g/ha | 13.2 | 200 g/ha | 30 % |
| 200 g/ha | 13.2 | 200 g/ha | 25 % |
| 200 g/ha | 13.2 | 100 g/ha | 35 % |
| 400 g/ha | 13.4 | 400 g/ha | 25 % |
| 400 g/ha | 13.4 | 200 g/ha | 15 % |
| 200 g/ha | 13.4 | 200 g/ha | 25 % |
| 200 g/ha | 13.4 | 100 g/ha | 30 % |
| 400 g/ha | 13.5 | 400 g/ha | 25 % |
| 400 g/ha | 13.6 | 400 g/ha | 25 % |

Eine Untergruppe der Dichloracetamide der Formel II wird durch die Verbindungen der Formel XV verkörpert

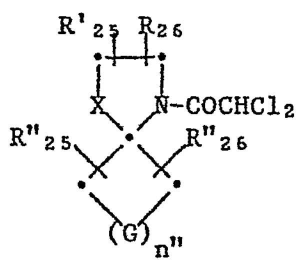

(XV)

worin

G Kohlenstoff, Sauerstoff, Schwefel, n" 4 bis 7,

$R'_{25}$ Wasserstoff, $C_1$-$C_4$-Alkyl, unsubstituiert oder substituiert durch Hydroxy, $C_1$-$C_4$-Alkoxy oder Halogen,

$R''_{25}$ Wasserstoff, $C_1$-$C_4$-Alkyl, unsubstituiert oder substituiert durch Hydroxy, $C_1$-$C_4$-Alkyl oder Halogen,

$R_{26}$ und $R''_{26}$ Wasserstoff, $C_1$-$C_4$-Alkyl unsubstituiert oder substituiert durch Reste $-S(O)_{n'}R_{27}$, $-OR'_{27}$ oder durch Phenyl, welches unsubstituiert oder substituiert ist durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-alkylcarbamoyl, $C_1$-$C_4$-Alkylsulfonyl, $C_1$-$C_4$-Alkylsulfuryl, Sulfamoyl oder Di-$C_1$-$C_4$-alkylsulfamoyl,

X Sauerstoff, oder ein Rest $S(O)_{n'}$

n' Null, 1, oder 2

n" 4, 5, 6 oder 7

$R_{27}$ $C_1$-$C_4$-Alkyl, unsubstituiert oder substituiert durch Phenyl, welches seinerseits unsubstituiert oder substituiert ist durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxycarbonyl, $C_1$-$C_4$-Alkylsulfonyl, $C_1$-$C_4$-Alkylsulfuryl, Sulfamoyl oder $C_1$-$C_4$-Dialkylsulfamoyl,

$R'_{27}$ Wasserstoff oder dasselbe wie $R_{27}$

bedeuten.

Viele Verbindungen der Formeln XIV und XV sind aus den veröffentlichten Patentanmeldungen BE-A 874,361, BE-A 841,320, BE-A 884,875, BE-A 874,361, EP-A 74,255, DE-A 2,350,800, DE-A 2 421 195, FR-A 2 300 084, USP 3 959 304, USP 4 186 130, USP 4 236 011, USP 4 243 811, USP 4 249 931, USP 4 268 677, WP 8 100 406 und USP 4 319 031 bekannt oder können gemäss diesen offenbarten Methoden hergestellt werden. Folgende Dichloracetamide eignen sich erfindungsgemäss als Antidote zum Sulfonylharnstoff der Formel I besonders gut:

Tabelle 14

| Nr. | $R'_{25}$ | $R_{26}$ | G | X | n" | $R''_{25}$ | $R''_{26}$ |
|---|---|---|---|---|---|---|---|
| 14.1 | H | H | $CH_2$ | O | 5 | H | H |
| 14.2 | H | H | $CH_2$ | O | 5 | 10-$CH_3$ | 10-$CH_3$ |
| 14.3 | H | H | $CH_2$ | O | 5 | 10-$CH_3$ | H |
| 14.4 | H | H | $CH_2$ | O | 6 | H | H |
| 14.5 | H | H | $CH_2$ | O | 4 | H | H |
| 14.6 | H | H | $CH_2$ | O | 7 | H | H |
| 14.7 | H | H | 9-O | O | 5 | H | H |
| 14.8 | H | H | $CH_2$ | S | 5 | H | H |
| 14.9 | H | 3-$C_2H_5$ | $CH_2$ | O | 5 | H | H |
| 14.10 | H | 3-Phenyl | $CH_3$ | O | 5 | H | H |

Die Fähigkeit der Verbindung 14.1 junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 14a zusammengefasst.

Tabelle 14a

| Herbizid Aufwandmenge | Antidote No. | Aufwandmenge | relative Schutzwirkung |
|---|---|---|---|
| 400 g/ha | 14.1 | 400 g/ha | 70 % |
| 400 g/ha | 14.1 | 200 g/ha | 75 % |
| 200 g/ha | 14.1 | 200 g/ha | 25 % |
| 200 g/ha | 14.1 | 100 g/ha | 20 % |

Eine Untergruppe der Dichloracetamide der Formel II wird durch die Verbindungen der Formel XVI verkörpert

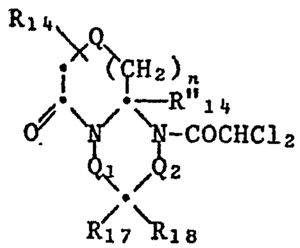

(XVI)

worin

n Null, 1, 2 oder 3,

Q einen Rest $-(CH_2)_p$

$Q_1$ einen Rest $-(CHR_{19})o^1$

$Q_2$ einen Rest $-(CHR'_{19})o^2$

$R_{14}$ Wasserstoff oder $C_1$-$C_4$-Alkyl

$R''_{14}$ Wasserstoff, $C_1$-$C_4$-Alkyl einen Rest $-COR''_6$ oder Phenyl, welches unsubstituiert oder substituiert ist durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxycarbonyl, $C_1$-$C_4$-Halogenalkoxycarbonyl, Carbamoyl, $C_1$-$C_4$-Alkylcarbamoyl, Di-$C_1$-$C_4$-Alkylcarbamoyl, $C_1$-$C_4$-Alkylsulfonyl, $C_1$-$C_4$-Alkylsulfuryl, Sulfamoyl, $C_1$-$C_4$-Alkylsulfamoyl oder Di-$C_1$-$C_4$-alkylsulfamoyl,

$R_{17}$ Wasserstoff oder $C_1$-$C_4$-Alkyl

$R_{18}$ Wasserstoff oder $C_1$-$C_4$-Alkyl

$R_{19}$ Wasserstoff oder $C_1$-$C_4$-Alkyl

$R'_{19}$ Wasserstoff oder $C_1$-$C_4$-Alkyl

$o^1$ Null oder 1,

$o^2$ Null oder 1 und

p Null oder 1

$R''_6$ Wasserstoff, $C_1$-$C_4$-Alkyl, unsubstituiert oder durch Halogen oder $C_1$-$C_4$-Alkoxy substituiert,

bedeuten.

Viele dieser Verbindungen sind aus den offengelegten Patentanmeldungen EP-A 65 724 und DE-A 2 948 535 bekannt oder sie können nach bekannten Methoden hergestellt werden. Folgende Dichloracetamide eignen sich erfindungsgemäss als Antidote zum Sulfonylharnstoff der Formel I besonders gut.

Tabelle 15

| Nr. | Q | $R''_{14}$ | $Q_1$ | $Q_2$ | n | $R_{14}$ | $R_{17}$ | $R_{18}$ |
|---|---|---|---|---|---|---|---|---|
| 15.1 | $CH_2$ | $CH_3$ | $CH_2$ | $CH_2$ | 0 | H | $CH_3$ | $CH_3$ |
| 15.2 | $CH_2$ | $CH_3$ | $CH_2$ | $CH_2$ | 0 | H | H | H |
| 15.3 | $CH_2$ | $CH_3$ | - | $CH_2$ | 0 | H | H | H |
| 15.4 | $CH_2$ | $CH_3$ | - | $CH_2$ | 1 | H | H | H |

Die Fähigkeit der Verbindung 15.1 junge Maispflanzen vor der phytotoxischen Wirkung von N-(2-Methoxycarbonylphenylsulfonyl)-N'-(4',6'-bis-difluormethoxypyrimidin-2-yl)-harnstoff zu schützen, wurde gemäss Beispiel 1 geprüft. Die Resultate sind in der Tabelle 15a zusammengefasst.

Tabelle 15a

| Herbizid Aufwandmenge | Antidote No. | Aufwandmenge | relative Schutzwirkung |
|---|---|---|---|
| 400 g/ha | 15.1 | 400 g/ha | 70 % |
| 400 g/ha | 15.1 | 200 g/ha | 70 % |
| 400 g/ha | 15.1 | 100 g/ha | 65 % |
| 400 g/ha | 15.1 | 50 g/ha | 75 % |
| 200 g/ha | 15.1 | 200 g/ha | 40 % |
| 200 g/ha | 15.1 | 100 g/ha | 45 % |
| 200 g/ha | 15.1 | 50 g/ha | 45 % |
| 200 g/ha | 15.1 | 25 g/ha | 45 % |
| 100 g/ha | 15.1 | 100 g/ha | 25 % |
| 100 g/ha | 15.1 | 50 g/ha | 20 % |
| 100 g/ha | 15.1 | 25 g/ha | 15 % |

Ganz besonders gute Wirkung zur Erhöhung der Toleranz von Kulturen gegen die herbizide Wirkung von N-(2-Methoxycarbonylphenylsulfonyl)-N'-(4,6-bis-difluoromethoxy-pyrimidin-2-yl)harnstoff zeigten folgende Verbindungen:

- N,N-Diallyl-dichloracetamid
- N-Allyl-N-(2-methoxy-1-methyläthyl)-dichloracetamid
- N-Benzyloxycarbonyl-N'-dichloracetyl-hydrazinomethyl-äthyl-phosphonsäure-isopropylester.
- N-Aethoxycarbonyl-N'-dichloracetyl-hydrazinomethyl-phosphonige-säure-diisopropylester,
- N-Allyl-N-n-butoxyäthoxyäthyl-dichloracetamid,
- N-(3,4-Dimethoxybenzyl)-N-isopropyl-dichloracetamid,
- 4-Dichloracetyl-2,3-dihydro-3-methyl-1,4-benzoxazin,
- 4-Dichloracetyl-2,3-dihydro-3,6-dimethyl-1,4-benzoxazin,
- 4-Dichloracetyl-2,3-dihydro-3-methyl-1,4-benzothiazin,
- N-Dichloracetyl-tetrahydro-isochinolin sowie
- 5-Dichloracetyl-3,3,6-trimethyl-9-oxo-1,5-diazabicyclo[4.3.0]-nonan
- N-Methyl-N-(dichloracetamidomethyl)-dichloracetamid,
- N-Methyl-N-(dichloracetamido-(N'-methyl)-methyl)-dichloracetamid,
- N-(1-Cyclohexyläthen-1-yl)-N-n-Butyl-dichloracetamid,
- N-Methyl-N(dichloracetamido(N'-methyl)äthyl)-dichloracetamid,
- N-Benzyl-N-methyl-dichloracetamid,
- N-Benzyl-N-äthyl-dichloracetamid,
- N-Benzyl-N(1-cyano-äth-1-yl)-dichloracetamid,
- N-(1,3-Dioxolan-2-ylmethyl)-N-allyl-dichloracetamid,
- N-Pyrrolidinyl-dichloracetamid
- 5,5-Spiro-cyclohexyl-N-oxazolidinyl-dichloracetamid,
- 2,2-Dimethyl-N-oxazolidinyl-dichloracetamid,
- 2,6-Dimethyl-N-morpholinyl-dichloracetamid,
- 4-Dichloracetyl-2,3-dihydro-3,7-dimethyl-1,4-benzoxazin,
- α,α-bis(dichloracetamido)-toluol,
- N,N'-(bis)Dichloracetyl-piperazin
- N-Phenyl-N'-dichloracetyl-piperazin

- N-Dichloracetyl-tetrahydrochinolin
- N-Dichloracetyl-2-methyl-indolin
- N-Dichloracetyl-2-methyl-benzthiazolidin

Die Erfindung betrifft die selektiv-herbiziden Mittel, sowie Verfahren zur pre- und post-emergenten Unkrautbekämpfung.

Die Verbindungen der Formel I und II-XVI werden in unveränderter Form oder vorzugsweise als Mittel zusammen mit den in der Formulierungstechnik üblichen Hilfsmitteln eingesetzt und werden daher z.B. zu Emulsionskonzentraten, direkt verprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I sowie das Gegenmittel und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie

N-Methyl-2-pyrrolidon, Dimethylsulfoxid oder Dimethylformamid, sowie gegegebenenfalls epoxidiertes Pflanzenöl wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Oxtylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.
M. and J. Ash, "Encyclopedia of Surfactants" Vol. I-III,
Chemical Publishing Co., Inc. New York, 1981.
H. Stache "Tensid-Taschenbuch", C. Hanser Verlag, München und
Wien 1982

Die Wirkstoffzubereitungen enthalten in der Regel 0,1 bis 99 %, insbesondere 0,1 bis 80 %, Wirkstoff der Formel I, 1 bis 99 % eines festen oder flüssigen Zusatzstoffes und 0 bis 25 %, insbesonders 0,1 bis 25 %, eines Tensides.

Insbesondere setzen sich bevorzugte Formulierungen folgendermassen zusammen: (% = Gewichtsprozent)

<u>Emulgierbare Konzentrate</u>

| | |
|---|---|
| Wirkstoffe der Formeln I und II | 1 bis 50 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 %. |

<u>Stäube</u>

| | |
|---|---|
| Aktiver Wirkstoff: | 0,1 bis 10 %, vorzugsweise 0,1 bis 1 % |
| festes Trägermaterial: | 99,9 bis 90 %, vorzugsweise 99,9 bis 99 %. |

<u>Suspensions-Konzentrat</u>

| | |
|---|---|
| Wirkstoffgemisch: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

<u>Benetzbare Pulver</u>

| | |
|---|---|
| Wirkstoffgemisch : | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzgusweise 15 bis 90 %. |

<u>Granulate</u>

| | |
|---|---|
| Wirkstoffgemisch: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg/ha, vorzugsweise 0,025 bis 5 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Herstellung von Dichloracetamiden der Formel II und deren Verarbeitung zu erfindungsgemässen Mitteln Temperaturen sind in Celsiusgraden angegeben, Drucke im millibar und Teile oder Prozentangaben beziehen sich auf das Gewicht.

Beispiel 2: Herstellung von N-(2-Methoxycarbonylphenyl-sulfonyl)-N'-[4,6-bis-(difluormethoxy)-pyrimidin-2-yl]-harnstoff der Formel I.

a) 50,5 g (1,0 Mol) Ammoniumchlorid werden zusammen mit 42,0 g (0,5 Mol) Cyanguanidin auf 175°C erhitzt. Es entsteht eine klare farblose Schmelze. Nach Einsetzen der Reaktion erwärmt sich das Reaktionsgemisch durch die freiwerdende Reaktionswärme auf 206°C. Danach wird für 3,5 Stunden bei 175°C gerührt, auf 80°C abgekühlt, mit 320 ml Methanol verdünnt und innerhalb von 5 Minuten mit 172,3 g 30 %iger methanolischer Natriummethylatlösung (0,97 Mol) versetzt. Die entstehende Suspension wird für 30 Minuten am Rückfluss gekocht und anschliessend innerhalb von 5 Minuten mit 173,0 g (1,08 Mol) Malonsäurediäthylester versetzt. Diese Suspension wird für weitere 6 Stunden zum Rückfluss erhitzt und dann mit 1,25 l Wasser verdünnt. Durch Ansäuren der klaren orangefarbigen Lösung mit Eisessig bis auf einen pH-Wert von 4,5 wird das Produkt ausgefällt. Der Niederschlag wird abgetrennt, mit Wasser gewaschen und bei 60°C über Phosphorpentoxid im Vakuum getrocknet. Man erhält so 35,3 g (25,0 %) 2-Amino-4,6-dihydroxypyrimidin, Smp. > 290°C.

b) Ein Gemisch aus 31,2 g (0,25 Mol) 2-Amino-4,6-dihydroxypyrimidin, 340 g 30 %iger wässeriger Natriumhydroxidlösung (2,5 Mol) und 600 ml Dioxan wird auf 75°C erhitzt. In diese Emulsion leitet man für 1,5 Stunde gasförmiges Difluorchlormethan ein. Die organische Phase wird abgetrennt und eingedampft. Der Rückstand wird mit Eiswasser gewaschen und getrocknet. Man erhält so das gewünschte 2-Amino-4,6-bis-(difluormethoxy)-pyrimidin, Smp. 74-75°C.

c) Eine Lösung von 3,62 g (0,0158 Mol) 2-Methoxycarbonylphenylsulfonylisocyanat und 2,63 g (0,0116 Mol) 2-Amino-4,6-bis-(difluormethoxy)pyrimidin in 50 ml Dioxan wird 2 Stunden bei 60-70°C

gerührt. Durch Filtration, Eindampfe und Verreiben des Rückstandes in Aether erhält man 3,9 g (73,7 % d.Th.) N-(2-Methoxycarbonyl-pheny-sulfony)-N'-[4,6-bis-(difluormethoxy)-pyrimidin-2-yl]-harnstoff, Smp. 186-188°C.

Beispiele für die Herstellung von Dicloracetamide der Formel II:

Beispiel 3: Herstellung von N-(3,4-Dimethoxybenzyl)-N-isopropyl-dichloracetanilid

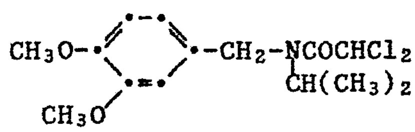

In einem 750 ml Vierhalskolben gibt man 126 g N-(3,4-Dimethoxybenzyl)-N-isopropyl-amin (dargestellt durch hydrierende Kondensation von 3,4-Dimethoxy-benzaldehyd mit Isopropylamin, Kp. 77,5°-78,5°/0,03 mbar) und 250 ml Toluol und rührt bis sich das Amin gelöst hat. Dazu gibt man 120 g 20 %ige Natronlauge und rührt unter Kühlen des Kolbens in Alkohol/$CO_2$-Bad bis die Temperatur der Reaktionslösung -10° bis -15°C beträgt. Dann tropft man langsam in die gerührte Aminlösung eine solche von 89 g Dichloressigsäurechlorid in 100 ml Toluol. Dabei fällt sofort ein weisser Niederschlag aus.

Nachdem alles zugetropft ist, was ca. 1 1/2 Stunden in Anspruch nimmt, wird das Kühlbad weggenommen und das Reaktionsgemisch weitergerührt, bis es Raumtemperatur erreicht. Dann wird auf Eis/Wasser gegossen und die organische Phase im Scheidetrichter mit Toluol extrahiert. Die Toluolphasen werden gesammelt, je zweimal mit 1 N Salzsäure und Wasser gewaschen, getrocknet und eingedampft. Es verbleiben 176 g (91,7 % der Theorie) Titelprodukt als helles zähes Oel mit Brechungsindex $n_D^{22}$ 1.5495, welches schliesslich erstarrt. Smp.69-72°.

Beispiel 4: Herstellung von 5-Dichloracetyl-3,3,6-trimethyl-9-oxo-1,5-diazabicyclo[4,3,0]-nonan

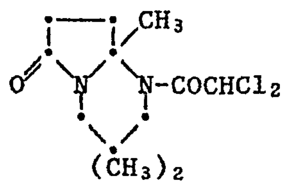

Zu einer Lösung von 18,8 g (0,1 Mol) 3,3,6-Trimethyl-9-oxo-1,5-diazabcylo[4.3.0]nonan in 100 ml Toluol gibt man 10,5 g (0,105 Mol) Triäthylamin. Anschliessend kühlt man auf -10°C ab und tropft bei -10 bis -5°C 14,8 g (0,1 Mol) Dichloracetylchlorid zu. Nach 4-stündige Nachrühren wird das ausgefallene Hydrochlorid des Triäthylamins abfiltriert. Aus de Filtrat scheiden sich nach dem Abdampfen des Toluols Kristalle ab, die aus Toluol/Aceton = 1 : 3 umkristallisiert werden können. Man erhält 21,1 g 5-Dichloracetyl-3,3,6-trimethyl-9-oxo-1,5-diazabicyclo[4.3.0]nonan vom Schmelzpunkt 182-185°.

Beispiel 5: Herstellung von N-(3,4-Methylendioxybenzyl)-N-iso-propyl-dichloracetanilid

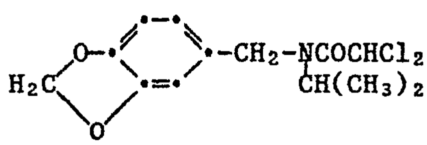

In einem 350 ml Vierhalskolben gibt man 19,3 g N-(3,4-Methylendioxybenzyl)-N-isopropyl-amin (dargestellt durch hydrierende Kondensation von 3,4-Methylendioxybenzaldehyd mit Isopropylamin, Kp. 60°-64°/0.03 mbar) und 60 ml Toluol und rührt bis sich das Amin gelöst hat. Dazu gibt man 20 g 20%ige Natronlauge und rührt unter Kühlen des Kolbens in Alkohol/$CO_2$-Bad bis die Temperatur der Reaktionslösung -10° bis -15°C beträgt. Dann tropft man langsam in die gerührte Aminlösung eine solche von 14,8 g Dichloressigsäurechlorid in 10 ml Toluol. Dabei fällt sofort ein weisser Niederschlag aus.

Nachdem alles zugetropft ist, was ca. 1 1/2 Stunde in Anspruch nimmt, wird das Kühlbad weggenommen und das Reaktionsgemisch weitergerührt, bis es Raumtemperatur erreicht. Dann wird auf Eis/Wasser gegossen und die organische Phase im Scheidetrichter mit Toluol extrahiert. Die Toluolphasen werden gesammelt, je zweimal mit 1 N Salzsäure und Wasser gewaschen, getrocknet und eingedampft. Es verbleiben 20,6 g Titelprodukt als helles zähes Oel.

Beispiel 6: Herstellung von N-(3,4-Dimethoxyphenyläthyl)-N-isopropyl-dichloracetamid.

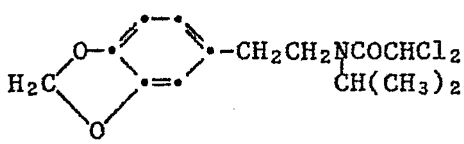

In einem Sulfierkolben wird eine Lösung von 22,3 g N-(3,4-phenyläthyl)-N-isopropyl-amin vorgelegt. Dazu gibt man unter Rühren 20 ml 20%ige wässrige Natronlauge. Dann wird das Reaktionsgemisch mit einem Kühlbad auf eine Temperatur von -10° bis -15° gekühlt, zu dem man unter Rühren langsam eine Lösung der berechneten Menge Dichloracetylchlorid in 10 ml Toluol tropft. Nachdem alles zugegeben ist, nimmt man das Kühlbad weg und rührt während 2 Stunden bei Raumtemperatur weiter. Dann wird das Reaktionsgemisch auf Eis/Wasser gegossen, mit Toluol extrahiert und die gesammelten organischen Phasen je einmal mit verdünnter Natronlauge, verdünnter Salzsäure und zweimal mit Wasser gewaschen, über Natriumsulfat getrocknet und am Rotationsverdampfer eingedampft. Man erhält so 28,4 g Titelprodukt als dickes Oel.

In analoger Weise zu diesen Beispielen werden die Verbindungen folgender Formel hergestellt:

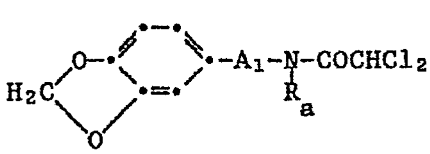

Beispiel 7: Herstellung von N-Dichloracetyl-1-methyl-tetrahydro-isochinolin

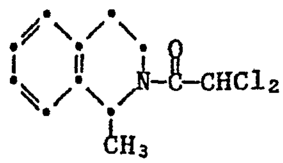

In einer Mischung von 14,7 g 1-Methyl-tetraisochinolin in 60 ml Toluol und 4 g Natriumhydroxyd in 20 ml Wasser werden bei -10° bis 0° 14,7 g Acetylchlorid in 70 ml Toluol zugetropft. Die Reaktion wird während 30 Minuten weitergerührt und mit 100 ml Wasser verdünnt.

Die organische Phase wird mit wässrigem NaOH, Wasser, HCl gewaschen, über Natriumsulfat getrocknet und eingedampft. Man erhält 23,8 g eines Oels, das sichwährend dem Stehen verfestigt. Smp. 74-77°C.

| | | | |
|---|---|---|---|
| % C ber. 55,8 | % H ber. 5,08 | % N ber. 5,43 | % Cl ber. 27,47 |
| % C gef. 55,8 | % H gef. 5,1 | % N gef. 5,3 | % Cl gef. 27,5 |

Beispiel 8

Formulierungsbeispiele für Wirkstoffe der Formel II oder Mischungen

| a) Spritzpulver | a) | b) | c) |
|---|---|---|---|
| Wirkstoff der Formeln II-XVI oder Mischung mit dem Herbizid der Formel I | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) <u>Emulsions-Konzentrat</u> | a) | b) |
|---|---|---|
| Wirkstoff der Formeln II-XVI oder Mischung mit dem Herbizid der Formel I | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) <u>Stäubemittel</u> | a) | b) |
|---|---|---|
| Wirkstoff der Formel II oder Mischung mit dem Herbizid der Formel I | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) <u>Extruder Granulat</u> | a) | b) |
|---|---|---|
| Wirkstoff der Formel II oder Mischung mit dem Herbizid der Formel I | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff der Formel II oder Mischung mit dem Herbizid der Formel I | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff der Formel II oder Mischung mit dem Herbizid der Formel I | 40 % | 5 % |
| Aethylenglykol | 10 % | 10 % |
| Nonylphenolpolyäthylenglykoläther (15 MOl AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff der Formel II oder Mischung mit dem Herbizid der Formel I | 5 % |
| Isopropylamin | 1 % |
| Octylphenolpolyäthylenglykoläther (78 Mol AeO) | 3 % |
| Wasser | 91 % |

Patentansprüche

1. Selektives herbizides Mittel, dadurch gekennzeichnet, dass es neben inerten Zuschlagstoffen eine herbizid wirksame Menge von N-(2-Methoxycarbonylphenylsulfonyl)-N'-(4,6-bis-difluormethoxy-pyrimidin-2-yl)harnstoff der Formel I

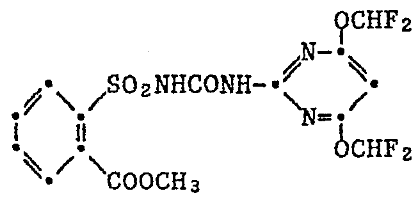

(I)

und zur Erhöhung der Toleranz der Kultur eine nicht-phytotoxische Menge eines Dichloracetamides der Formel II enthält

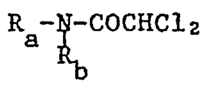

(II) .

worin $R_a$ und $R_b$ unabhängig voneinander je Wasserstoff, einen unsubstituierten oder substituierten alicyclischen Kohlenwasserstoffrest oder eines davon einen Aminrest oder beide zusammen mit dem Stickstoffatom, an das sie gebunden sind auch einen 5-12 gliedrigen heterocyclischen Rest bedeuten.

2. Mittel gemäss Anspruch 1, dadurch gekennzeichnet, dass es zur Erhöhung der Toleranz der Kultur eine nicht phytotoxische Menge eines Dichloracetamides der Formel II enthält, worin $R_a$ und $R_b$ unabhängig voneinander je Wasserstoff, einen Alkyl-, Alkenyl- , einen Cycloalkyl-, Cycloalkenyl-Rest welcher unsubstituiert oder durch Halogen, Cyano; Hydroxy; Alkylcarbonyloxy; Alkenylcarbonyloxy; Alkinylcarbonyloxy; Alkoxyalkylcarbonyloxy; Alkylcarbonylthio; Alkoxycarbonyloxy; Formyloxy; Benzoyloxy; subst. Benzoyloxy; Haloalkylcarboxy; Alkyloxy; Alkenyloxy; Alkinyloxy; Phenyloxy; subst. Phenyloxy; Heterocyclyloxy- und Heterocyclylthio; subst. Heterocyclyloxy- und Heterocyclylthio; Alkylthio; Alkenylthio; Phenylthio; subst. Phenylthio; Alkoxyalkoxy; Alkoxyalkoxyalkoxy; Alkenyloxyalkoxy; Tetraalkoxy; Alkylthioalkoxy; Carbamoyloxy; N-mono- und di-Alkylcarbamoyloxy; N-Cycloalkyl- und N,N-Alkylencarabamoyloxy; Carbamoylthio; Heterocyclyl- und substituiertes Heterocyclylaminocarbonyloxy; N-mono- und di-Alkylcarbamoylthio; Thiocarbamoylthio; N-mono- und di-Alkylthiocarbamoylthio; N-Cycloalkyl- und N,N-Alkylenthiocarbamoylthio; Alkyl- und Alkenylsulfinyl; Alkyl- und Alkenylsulfonyl; Alkyl- und Alkenylsulfonyloxy; Phenyl- und substituiertes Phenylsulfonyloxy; Phenylsulfinyl und -sulfonyl; substituiertes Phenylsulfinyl und -sulfonyl; Heterocyclyl- und substituiertes Heterocyclylsulfonyl; Sulfamoyl; N-Alkyl-, -N-Cycloalkyl-, N-Phenyl-, substituiertes N-Phenylsulfamoyl, Heterocyclyl- und substituiertes Heterocyclylsulfamoyl; N-Alkenyl-, N,N-di-Alkyl-, N,N-di-Alkenyl, N,N-Alkyl, Alkenyl-Sulfamoyl; Aminooxy; Mono- und di-Alkylaminooxy; Alkylcarbonylaminooxy; Phenyl-und substituiertes Phenylcarbonylaminooxy; Alkanaliminooxy; Benzaldehyd- und substituiertes Benzaldehydiminooxy; Dialkylketoniminooxy; Phenylalkylketoniminooxy; Dialkylphosphoryl; Phenyl-, Alkyl-Phosphoryl; Amino; Anilino; substituiertes Anilino; Heterocyclylamino- und substituiertes Heterocyclylamino; Mono- und di-Alkylamino; -Alkenylamino; Alkinylamino; di-Alkenylamino; di-Alkinylamino; N,N-Alkyl, Alkenylamino; N,N,N-trialkylammonium; Alkylamido; Alkenylamido; Alkinylamido; Alkyl-N-alkylamido; Benz-und substituiertes Benzamido; Phenylalkylamido; Phenylalkyl-N-alkylamido; Benz- und substituiertes Benz-N-Alkylamido; Heterocyclylamido- und substituiertes Heterocyclylamido; Haloalkyl- und Alkoxyalkylamido; Haloalkyl- und Alkoxy-N-alkylamido; Alkoxycarbonylamino; Alkoxycarbonyl-N-alkylamino; Alkenyloxycarbonylamino-und N-Alkylamino; Hydroxy- und Alkoxyamino; Hydroxy-und Alkoxy-N-Alkylamino; Alkenyloxyamino; Phenyl- und Alkylaminocarbonylamino; N-Phenyl-N-Alkylaminocarbonylamino; N,N-Alkylenaminocarbonylamino; N-Phenyl-N-Alkylaminocarbonyl-N-alkylamino; N-Phenylaminocarbonyl-N'-alkylamino; N,N'-di- und tri-Alkylaminocarbonylamino; Heterocyclyl- und substituiertes Heterocyclylaminocarbonylamino; N-Heterocyclyl- und substituiertes Heterocylyl-N,N'-mono- und di-alkylaminocarbonylamino; Alkyl- und Alkenylsulfonylamino; Alkyl- und Alkenylsulfonyl-N-alkylamino; Phenyl- und substituiertes Phenylsulfonylamino; Phenyl- und substituiertes Phenylsulfonyl-N-alkylamino; Heterocyclyl- und substituiertes Heterocyclylsulfonylamino; Alkanal-imino; Benzaldehyd- und substituiertes Benzaldehydimino; Phenyl- und substituiertes Phenylalkylketonimino; Dialkylketonimino; Guanidino; N,N',N''-mono-di-tri- und tetra-Alkylguanidino; Amidino; N,N'-mono-und Dialkylamidino, Phenyl- und substituiertes Phenylamidino; Phenyl- und substituiertes Phenyl-N,N'-mono-und di-alkylamidino; Di-alkylphosphonyl; Alkyl-o-alkylphosphinyl; Alkyl- und Alkenyloxycarbonyl; Phenylalkyloxycarbonyl; Alkyl- und Alkenylthiocarbonyl; Alkoxyalkoxycarbonyl; Haloalkyloxycarbonyl; Aminocarbonyl; mono- und di-Alkylaminocarbonyl; mono und di-Alkenylaminocarbonyl; mono- und di-Alkinylaminocarbonyl; Phenyl-und substituiertes Phenylalkylaminocarbonyl; Anilino- und substituiertes Anilinocarbonyl; N-Phenyl-N-alkylaminocarbonyl; N,N-Alkylenaminocarbonyl; Cycloalkylamino-und cycloalkyl-N-alkylaminocarbonyl; Hydroxy- und Alkoxyaminocarbonyl; Haloalkyl-und Alkoxyalkylaminocarbonyl; Formyl; Alkyl- und Alkenyl-und Alkinylcarbonyl; Phenyl- und substituiertes Phenylalkylcarbonyl; Phenyl- und substituiertes Phenylalkenyl-arbonyl; Benzoyl; substituiertes Benzoyl; Heterocyclylcarbonyl; substituiertes Heterocyclylcarbonyl; Phenyl; Naphthyl; substituiertes Phenyl-und Naphthyl; Heterocyclische Reste; substituierte heterocyclische Reste; substituiert sein können, weiter bedeuten $R_a$ und $R_b$ unabhängig voneinander Alkinyl; durch Phenyl, substituiertes Phenyl, Halogenalkyl, Alkylthio- und Alkenylthioalkyl, Phenylthio- und substituiertes Phenylthioalkyl, Alkoxy-und Phenoxyalkyl, Alkoxycarbonyl, Aminocarbonyl, mono- und di-Alkylaminocarbonyl, Anilino- und substituiertes Anilinocarbonyl, N-Anilino- und substituiertes Anilino-N-Alkylaminocarbonyl, N,N-Alkylenaminocarbonyl, Heterocyclyl- und substituiertes Heterocyclylaminocarbonyl, Aminoalkyl, mono- und di-Alkyl- und Alkenylaminoalkyl, N,N-Alkylen-aminoalkyl, Anilino-und substituiertes Anilinoalkyl, Phenyl- und substituiertes Phenylalkylaminoalkyl, Heterocyclylamino- und substituiertes Heterocyclylaminoalkyl, Heterocyclyl- und substi-tuiertes Heterocyclylaminoalkylaminoalkyl, substituierter Alkinyl-rest; Phenyl- und Naphthylreste, heterocyclische Reste und benzannellierte Heterocyclen können ihrerseits ein- oder mehrfach durch Halogen; Nitro; Cyano; Pseudohalogen; Alkyl- und Alkenyloxy;

Alkyl-und Alkenylthio; Alkyl; Alkenyl; Alkinyl; Phenylalkinyl; Alkylsulfinyl und -sulfonyl; Hydroxycarbonyl; Alkoxy- und Benzyloxycarbonyl; Alkenyloxycarbonyl; Alkylendioxy; Aminocarbonyl; mono- und di-Alkylaminocarbonyl; Amino; Ammonio; mono- und di-Alkylamino; Alkan- und Alkencarbonyl; Phenyloxy; durch Halogen, Halogenalkyl, Nitro, Alkoxy, Alkylthio, Alkyl, substituiertes Phenyloxy; Sulfo; Di-Alkylphosphoryl-und phosphonyl; Sulfamoyl; mono- und di-Alkylsulfamoyl; mono- und di-und tri-Halogenalkyl; Alkylsulfinyl- und -sulfonyl; substituiert sein, einer der Reste $R_a$ und $R_b$ kann auch Amino; mono- oder di-Alkyl und Alkenylamino; mono- oder di-Alkinylamino; substituiertes oder unsubstituiertes Phenylalkylamino; substituiertes oder unsubstituiertes N-Phenylalkyl-N-alkylamino; substituiertes oder unsubstituiertes Heterocyclylamino; substituiertes oder unsubstituiertes N-Heterocyclyl-N'-Alkylamino; Alkanalimino, substituiertes oder unsubstituierte Benzaldehydimino, Dialkylketonimino, substituiertes oder unsubstituiertes Phenylalkylketonimino; Alkyloxycarbonylamino-und N-Alkylamino; substituiertes oder unsubstituiertes Phenylalkoxycarbonylamino- und N-Alkylamino; durch 1-3-Alkyl substituiertes Ureido; durch 1-2 Alkyl substituiertes Phenylureido; durch 1-2 Alkyl substituiertes Heterocyclylureido; Alkyl-, Alkenyl- und Alkinylamido und N-Alkylamido; substituiertes und unsubstituiertes Phenylalkylamido; substituiertes und unsubstituiertes Phenyl- und Heterocyclylamido; substituiertes und unsubstituiertes Phenyl- und Heterocyclyl-N-Alkylamido; Alkyloxy- und Alkylthiothionoamino bedeuten, wobei die Alkylreste wie oben angegeben, substituiert sein können; ferner können die Reste $R_a$ und $R_b$ können zusammen mit dem Stickstoffatom an das sie gebunden sind auch einen gesättigten oder gesättigten 5 bis 12-gliedrigen Heterocyclus bilden, der noch ein zwei oder drei weitere Heteroatome oder eine Sulfinyl- resp. Sulfinylgruppe enthält durch ein oder zwei Carbonylgruppen unterbrochen sein kann, und welcher benzannelliert, unsubstituiert oder substituiert sein kann.

3. Mittel gemäss Anspruch 1, dadurch gekennzeichnet dass es zur Erhöhung der Toleranz der Kultur eine nicht phytotoxische Menge eines Dichloracetamides der Formel II enthält, worin $R_a$ Wasserstoff

$C_1$-$C_8$-Alkyl oder $C_3$-$C_8$-Cycloalkyl, unsubstituiert oder durch $-POR_1,R_2$, $-NR_3R_4$, $-(O)_mCOOR_5$, $-(X)_mCX''R_6$, $-O(AO)_{m'}-R_7$, $-XR_7$, Cyano, X"'Het, Het, $-SR_7$, $C_5$-$C_6$-Cycloalkyl, $CR(OR_8)OR_9$ oder Halogen substsituiert, oder $R_a$ ist ein $C_3$-$C_8$-Alkenyl- oder $C_3$-$C_8$-Cycloalkenylrest, der unsubstituiert oder durch Halogen, Phenyl, $C_5$-$C_6$-Cycloalkyl oder $C_1$-$C_4$-Alkyl substituiert ist, oder $R_a$ ist ein $C_3$-$C_8$-Alkinylrest, der unsubstituiert oder durch Phenyl substituiert ist, wobei die Phenylkerne unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sind, oder $R_a$ ist ein Alkoxy-iminoalkylrest $-CH(R_r)-C(R_s)=N-OR_t$, $R_r$ und $R_s$ je Wasserstoff oder $C_1$-$C_4$-Alkyl und $R_t$ Wasserstoff, $C_1$-$C_6$-Alkyl, $C_3$-$C_6$-Alkenyl oder $C_3$-$C_6$-Alkinyl,

$R_b$ Wasserstoff, dasselbe wie $R_a$ oder einen Rest $-NR_3R_4$,

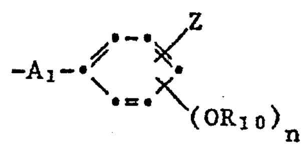

oder $-(\overset{R_{11}}{CH})_n-Het$

A eine $C_1$-$C_4$-Alkylenkette,
$A_1$ eine $C_1$-$C_8$-Alkylenkette die geradkettig oder verzweigt ist und die unsubstituiert oder durch Cyan oder $C_1$-$C_4$-Alkoxy oder einen Alkylamido- oder Halogenalkylamido-Rest substituiert ist,
Het einen gegebenenfalls durch $C_1$-$C_4$-Alkyl, $C_1$-$C_4$-Alkoxy, $C_1$-$C_4$-Alkoxycarbonyl oder $C_1$-$C_4$-Alkylthio optimal substituierten 5-6-gliedrigen Heterocyclus mit 1-3 Heteroatomen resp. $S(O)_{n'}$ Gruppen, wobei Sauerstoff und Schwefel nie direkt benachbart im Ring vorhanden sein können, sondern in einer 1.3-Anwendung vorliegen müssen, wie im 1,3-Dioxolan-2-yl-, 1,3-Dioxan-2-yl oder dem 2,4-Dioxan-1-ylrest und welcher zusätzlich durch $R_n$ in der 1-Stellung und $R'_n$ an einem Ring Kohlenstoffatom substituiert sein kann,
n Null oder eine Zahl von 1 bis 3,
m Null oder die Zahl 1,
m' Null oder eine Zahl von 1 bis 4,
n' Null oder eine Zahl von 1 bis 2,

R, R', $R_1$ und $R_2$ unabhängig voneinander je $C_1$-$C_4$-Alkyl oder $C_1$-$C_4$-Alkoxy,
$R_3$ Wasserstoff, $C_1$-$C_4$-Alkyl oder $C_3$-$C_8$-Cycloalkyl,
$R_4$ Wasserstoff, $C_1$-$C_4$-Alkyl, Phenyl, durch Halogen, $C_1$-$C_4$-Alkyl, $C_1$-$C_4$-Alkoxy, $C_1$-$C_4$-Halogenalkyl oder $C_1$-$C_4$Alkoxy substituiertes Phenyl oder einen Rest -COOR'$_5$ oder -COR'$_6$,
$R_5$ und R'$_5$ unabhängig voneinander je $C_1$-$C_4$-Alkyl, oder $C_1$-$C_4$-Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist,
$R_6$ und R'$_6$ unabhängig voneinander je Wasserstoff, $C_1$-$C_4$-Alkyl, $C_1$-$C_4$-Halogenalkyl, $C_2$-$C_5$-Alkoxyalkyl, Phenyl oder $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist, oder $R_6$ und R'$_6$ bedeuten einen $C_2$-$C_4$-Alkenyl- oder einen Rest -N($R_{12}$)($R_{13}$),
$R_7$ Wasserstoff, $C_1$-$C_4$-Alkyl, $C_3$-$C_6$-Alkenyl, $C_3$-$C_6$-Alkinyl, Phenyl und Phenyl($C_1$-$C_4$)Alkyl, wobei die Phenylkerne durch Halogen, $C_1$-$C_4$Alkyl, $C_1$-$C_4$Alkoxy oder $C_1$-$C_4$Halogenalkyl substituiert sind,
$R_8$ und $R_9$ unabhängig voneinander je $C_1$-$C_6$-Alkyl oder $C_3$-$C_6$-Alkenyl
$R_{10}$ $C_1$-$C_4$-Alkyl, $C_3$-$C_6$-Alkenyl, $C_2$-$C_6$-Alkoxyalkyl, $C_1$-$C_4$-Halogenalkenyl oder in Zusammenhang mit 2 Sauerstoffatomen auch eine $C_1$-$C_5$-Alkylenbrücke,
$R_{11}$ Wasserstoff oder $C_1$-$C_4$-Alkyl und
$R_{12}$ und $R_{13}$ unabhängig voneinander je Wasserstoff, $C_1$-$C_8$Alkyl, $C_3$-$C_8$-Cycloalkyl, $C_3$-$C_8$-Alkenyl, Phenyl oder $C_1$-$C_4$Aralkyl wobei der Phenylkern unsubstituieret oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyano, Nitro und
$R_{12}$ und $R_{13}$ zusammen eine $C_1$-$C_7$-Alkylenkette, die durch Sauerstoff, Schwefel, >NH, oder >N($C_1$-$C_4$)-Alkyl unterbrochen sein kann.
Z Wasserstoff, $C_1$-$C_4$-Alkyl, Halogen, $C_1$-$C_4$-Halogenalkyl, Nitro, $C_1$-$C_4$-Alkyl, $C_1$-$C_4$-Halogenalkoxy, $C_2$-$C_8$-Alkoxyalkoxy, der Dioxymethylenrest, ein 1.3-Dioxolan-2-yl- oder 1,3-Dioxan-2-ylrest, der über eine $C_1$-$C_4$-Alkylenbrücke gebunden sein kann,
X', X", X"' Sauerstoff, Schwefel, X Sauerstoff, Schwefel -S(O)$_n$,.

4. Mittel gemäss Anspruch 1, dadurch gekennzeichnet dass es zur Erhöhung der Toleranz der Kultur eine nicht phytotoxische Menge eines Dichloracetamides der Formel III enthält,

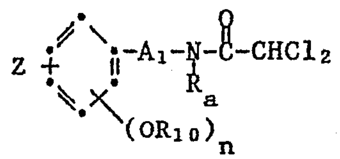

III

worin

$A_1$ eine $C_1$-$C_8$-Alkylenbrücke, die geradkettig oder verzweigt sein kann und die unsubstituiert oder durch Cyan, $C_1$-$C_4$-Alkoxy oder einen Alkylamido- oder Halogenalkenylamido-Rest substituiert sein kann,

n Null oder eine Zahl von 1 bis 3,

$R_a$ Wasserstoff $C_1$-$C_8$-Alkyl oder $C_3$-$C_8$-Cycloalkyl, unsubstituiert oder substituiert durch $-PO(R_1)(R_2)$, $-NR_3R_4$, $-(O)_mCOOR_5$, $-(X')_m-CX''R_6$, Cyano, Het, -X"'-Het, $-O-(A-O)_{m'}-R_7$, $-XR_7$, $C_5$-$C_6$-Cycloalkyl, $-CR(OR_8)(OR_9)$ oder Halogen oder $R_a$ ist ein $C_3$-$C_8$-Alkenyl- oder $C_3$-$C_8$-Cycloalkylrest, der unsubstituiert oder durch Halogen, Phenyl, $C_5$-$C_6$-Cycloalkyl oder $C_1$-$C_4$-Alkyl substituiert oder $R_a$ ist ein $C_3$-$C_8$-Alkinylrest, der unsubstituiert oder durch Phenyl substituiert ist, wobei die Phenylkerne unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sind, oder $R_a$ ist ein Alkoxy-iminoalkylrest $-CH(R_r)-C(R_s)=N-OR_t$

$R_r$ und $R_s$ je Wasserstoff oder $C_1$-$C_4$-Alkyl und $R_t$ Wasserstoff, $C_1$-$C_6$-Alkyl, $C_3$-$C_6$-Alkenyl oder $C_3$-$C_6$-Alkinyl bedeuten,

Z Wasserstoff, $C_1$-$C_4$-Alkyl, Halogen, $C_1$-$C_4$-Halogenalkyl, Nitro, $C_1$-$C_4$-Alkoxy, $C_2$-$C_8$-Halogenalkoxy, der Dioxymethylenrest, ein 1,3-Dioxolan-2-yl- oder 1,3-Dioxan-2-ylrest, der über eine $C_1$-$C_4$-Alkylenbrücke gebunden sein kann,

A eine $C_1$-$C_4$-Alkylenkette

Het einen gegebenenfalls durch $C_1$-$C_4$-Alkyl, $C_1$-$C_4$-Alkoxy, $C_1$-$C_4$-Alkoxycarbonyl oder $C_1$-$C_4$-Alkylthio optimal substituierten 5-6-gliedrigen Heterocyclus mit 1-3 Heteroatomen resp. $S(O)_{n'}$ Gruppen, wobei Sauerstoff und Schwefel nie direkt benachbart im Ring vorhanden sein können, sondern in einer 1.3-Anwendung vorliegen müssen, wie im 1,3-Dioxolan-2-yl-, 1,3-Dioxan-2-yl oder dem 2,4-Dioxan-1-ylrest und welcher zusätzlich durch $R_n$ in der 1-Stellung und $R'_n$ an einem Ring Kohlenstoffatom substituiert sein kann,

m' Null oder eine Zahl von 1 bis 4, m Null oder 1, n Null bis 3, n' Null bis 3

R, R', $R_1$ und $R_2$ unabhängig voneinander je $C_1$-$C_4$-Alkyl oder $C_1$-$C_4$-Alkoxy,

$R_3$ Wasserstoff oder $C_1$-$C_4$-Alkyl,

$R_4$ einen Rest $-COOR'_5$ oder $-COR'_6$,

$R_5$ und $R'_5$ unabhängig voneinander je $C_1$-$C_4$-Alkyl, oder $C_1$-$C_4$-Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist,

$R_6$ Wasserstoff, $C_1$-$C_4$-Alkyl, $C_1$-$C_4$-Halogenalkyl, $C_2$-$C_5$-Alkoxyalkyl, Phenyl oder $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist, oder $R_6$ bedeutet einen $C_2$-$C_4$-Alkenyl- oder einen Rest $-NR_{12}$, $R_{13}$,

$R_7$ Wasserstoff, $C_1$-$C_4$-Alkyl, $C_3$-$C_6$-Alkenyl, $C_3$-$C_6$-Alkinyl oder Benzyl,

$R_8$ und $R_9$ unabhängig voneinander je $C_1$-$C_6$-Alkyl oder $C_3$-$C_6$-Alkenyl

$R_{10}$ $C_1$-$C_4$-Alkyl, $C_3$-$C_6$-Alkenyl, $C_2$-$C_6$-Alkoxyalkyl, $C_1$-$C_4$-Halogenalkenyl oder in Zusammenhang mit 2 Sauerstoffatomen auch eine $C_1$-$C_5$-Alkylenbrücke,

$R_{12}$ und $R_{13}$ unabhängig voneinander je Wasserstoff, $C_1$-$C_8$-Alkyl, $C_3$-$C_8$-Alkenyl, Phenyl oder $C_1$-$C_4$-Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist und X', X" und X'" Sauerstoff, Schwefel,

X Sauerstoff, Schwefel oder $S(O)_n$,
bedeuten.

5. Neue Dichloracetamide der Formel III'

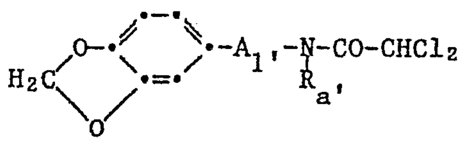

III'

worin

$A_1$, einen geradkettigen, verzweigten oder cyclischen $C_1$-$C_8$-Kohlenwasserstoffrest, welcher unsubstituiert oder durch Alkoxy, Alkylthio, Fluor, Cyan oder Halogenalkyl substituiert sein kann,

$R_a$, einen geradkettigen oder verzweigten, gesättigten oder ungesättigten $C_1$-$C_5$-Kohlenwasserstoffrest der unsubstituiert oder durch Alkoxy, poly-Alkoxy, Halogen, Cyan oder Trifluormethyl, substituiert sein kann, oder Cycloalkyl, Alkylcycloalkyl, Dialkoxyalkyl, 1,3-Dioxolan-2-ylalkyl, 1,3-Dioxolan-4-ylalkyl, 1,3-Dioxan-2-yl-alkyl, Furylalkyl, Tetrahydrofurfurylalkyl oder einen Rest $-NH-COOR_u$, $-CH_2-COOR_u$, $-CH(CH_3)COOR_u$ oder einen Alkoxyimino-alkylrest $-CH(R_r)-C(R_s)=N-OR_t$, wobei

$R_u$ Methyl, Aethyl, Propyl oder Allyl,

$R_r$ und $R_s$ je Wasserstoff oder $C_1$-$C_4$-Alkyl und

$R_t$ Wasserstoff, $C_1$-$C_6$-Alkyl, $C_3$-$C_6$-Alkenyl oder $C_3$-$C_6$-Alkinyl

bedeuten.

6. Acylamide gemäss Anspruch 5, dadurch gekennzeichnet, dass $A_1$, für $C_1$-$C_6$-Alkylen steht.

7. Acylamide gemäss Anspruch 5, dadurch gekennzeichnet, dass $A_1$, für $C_1$-$C_3$-Alkylen steht.

8. N-(3,4-Methylendioxybenzyl)-N-isopropyl-dichloracetamid gemäss Anspruch 5.

9. Verfahren zur Herstellung der Acylamide der Formel III', Anspruch 5, dadurch gekennzeichnet, dass man ein Acylhalid der Formel II'

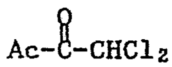 II'

worin Ac Chlor, Brom, oder den Rest $-O-CO-CHCl_2$ bedeutet in ein einem inerten organischen Lösungsmittel, in Gegenwart der mindestens äquimolaren Menge eines säurebindenden Mittels mit einem Amin der Formel IIIa' umsetzt

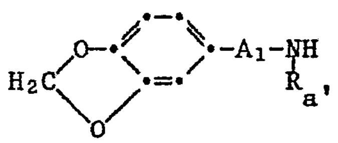 IIIa'

wobei $A_{1'}$ und $R_{a'}$ die unter Formel III', Anspruch 5 gegebene Bedeutung haben.

10. Verfahren zur Herstellung der Acylamine der Formel III', Anspruch 5, dadurch gekennzeichnet, dass man ein Amin der Formel IIIa'

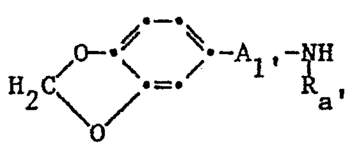 (IIIa'),

worin $A_{1'}$ und $R_{a'}$ die unter Formel III', Anspruch 5 gegebene Bedeutung haben, mit Chloral odereinem durch Addition an dessen Oxogruppe gebildeten Chloralderivat in einem wassrigen Medium und/oder einen polaren organischen Lösungsmittel, in Gegenwart säurebindender Mittel und der katalytischen Menge eines anorganischen oder organischen Cyanides umsetzt.

11. Verfahren zur Herstellung von Acylamin der Formel III' gemäss Anspruch 5, dadurch gekennzeichnet, dass man in einem inerten organischen Lösungsmittel, äquimole Mengen von Methylendioxybenzol mit N-Methylol-dichloracetamid umsetzt.

12. Mittel gemäss Anspruch 1, dadurch gekennzeichnet dass es zur Erhöhung der Toleranz der Kultur eine nicht phytotoxische Menge eines Dichloracetamides der Formel IV enthält,

$$Het-(\overset{R_{11}}{\overset{|}{C}H})_n-\underset{\underset{R_a}{|}}{N}-COCHCl_2 \qquad (IV)$$

worin Het einen gegebenenfalls durch $C_1$-$C_4$-Alkyl, $C_1$-$C_4$-Alkoxy, $C_1$-$C_4$-Alkoxycarbonyl oder $C_1$-$C_4$-Alkylthio optimal substituierten 5-6 gliedrigen Heterocyclus, mit 1-3 Heteroatomen resp. $S(O)_n$, Gruppen, wobei Sauerstoff und Schwefel nie direkt benachbart im Ring vorhanden sein können, sondern in einer 1,3-Anordnung vorliegen müssen, wie im 1,3-Dioxolan-2-yl-, 1,3-Dioxan-2-yl oder dem 2,4-Dioxan-1-ylrest und welcher zusätzlich durch $R_n$ in der 1-Stellung und $R'_n$ an einem Ring Kohlenstoff substituiert sein kann,
$R_a$ Wasserstoff $C_1$-$C_8$-Alkyl oder $C_3$-$C_8$-Cycloalkyl unsubstituiert oder substituiert durch $-PO(R_1)(R_2)$, $-NR_3,R_4$, $-(O)_mCOOR_5$, $-(X')_mCXR''_6$, $-O-(A-O-)_{m'}-R_7$, $-XR_7$, Cyano, -X"'-Het, Het, $-C_5$-$C_6$-Cycloalkyl, $-CR(OR_8)(OR_9)$ oder Halogen; oder $R_a$ ist ein $C_3$-$C_8$-Alkenyl- oder $C_3$-$C_8$-Cycloalkenylrest, der unsubstituiert oder substituiert durch Halogen, Phenyl, $C_5$-$C_6$-Cycloalkyl oder $C_1$-$C_4$-Alkyl ist oder $R_a$ ist ein $C_3$-$C_8$-Alkinylrest der unsubstituiert oder durch Phenyl substituiert ist, wobei die Phenylkerne unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sind, oder
$R_a$ ist ein Alkoxyiminoalkylrest $-CH(R_r)-CH(R_s)=NOR_t$, $R_r$ und $R_s$ je Wasserstoff oder $C_1$-$C_4$-Alkyl, $R_t$ Wasserstoff, $C_1$-$C_6$-Alkyl, $C_3$-$C_6$-Alkenyl oder $C_3$-$C_6$-Alkinyl,
A eine $C_1$-$C_4$-Alkylenkette,
m Null oder eins

m' Null oder eine Zahl von 1 bis 4,

n' Null bis 2,

n Null bis 3,

$R_1$ und $R_2$ unabhängig voneinander je $C_1$-$C_4$-Alkyl oder $C_1$-$C_4$-Alkoxy,

$R_3$ Wasserstoff $C_1$-$C_4$-Alkyl oder $C_1$-$C_8$-Cycloalkyl

$R_4$ Wasserstoff, $C_1$-$C_4$-Alkyl, Phenyl, durch Halogen, $C_1$-$C_4$-Alkyl, $C_1$-$C_4$-Alkoxy, $C_1$-$C_4$-Halogenalkyl oder $C_1$-$C_4$-Halogenalkoxy substituiertes Phenyl oder einen Rest -COOR'$_5$ oder -COR'$_6$

$R_5$ und R'$_5$ unabhängig voneinander je $C_1$-$C_4$-Alkyl oder $C_1$-$C_4$-Aralkyl wobei der Phenylkern unsubstituiert oder durch Halogen $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist.

$R_6$ und R'$_6$ unabhängig voneinander jeWasserstoff, $C_1$-$C_4$-Alkyl, $C_1$-$C_4$-Halogenalkyl, $C_{2-8}$-Alkoxyalkyl, Phenyl oder $C_1$-$C_4$-Aralkyl wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist; oder $R_6$ und R'$_6$ bedeuten einen $C_2$-$C_4$-Alkenyl- oder einen Rest -N($R_{12}$)($R_{13}$)

$R_7$ Wasserstoff, $C_1$-$C_4$-Alkyl, $C_3$-$C_6$-Alkenyl, $C_3$-$C_6$-Alkinyl oder Phenyl($C_1$-$C_4$)alkyl, wobei die Phenylkerne durch Halogen, $C_1$-$C_4$-Alkyl, $C_1$-$C_4$-Alkoxy oder $C_1$-$C_4$-Halogenalkoxy substituiert sind

$R_8$ und $R_9$ unabhängig voneinander je $C_1$-$C_6$-Alkyl oder $C_3$-$C_6$-Alkenyl,

$R_{11}$ Wasserstoff oder $C_1$-$C_4$-Alkyl,

$R_{12}$ und $R_{13}$ unabhängig voneinander je Wasserstoff, $C_1$-$C_8$-Alkyl, $C_3$-$C_6$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert,

$R_{12}$ und $R_{13}$ zusammen eine $C_1$-$C_7$-Alkylenkette, die durch Sauerstoff, Schwefel-NH- oder -N($C_1$-$C_4$)alkyl-unterbrochen sein kann,

$R_n$ Wasserstoff, $C_1$-$C_4$-Alkyl oder Phenyl welches unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist,

und X', X", X"' Sauerstoff, Schwefel und X Sauerstoff, Schwefel oder $-S(O)_n$,
bedeuten.

13. Mittel gemäss Anspruch 1, dadurch gekennzeichnet dass es zur Erhöhung der Toleranz der Kultur eine nicht phytotoxische Menge eines Dichloracetamides der Formel V enthält,

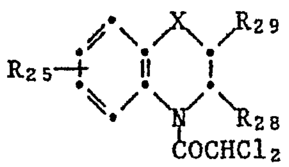

(V)

worin

$R_{25}$ Wasserstoff, $C_1$-$C_4$-Alkyl, unsubstituiert oder substituiert durch Hydroxy, $C_1$-$C_4$-Alkoxy oder Halogen,

$R_{28}$ Wasserstoff, $C_1$-$C_4$-Alkyl unsubstituiert oder substituiert durch Halogen, Cyan oder einen Rest $-COA_3$; $C_2$-$C_4$-Alkenyl, $C_2$-$C_4$-Alkinyl, $C_2$-$C_4$-Alkoxyalkyl, Cyano oder einen Rest -COA;

$R_{29}$ Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

A $C_1$-$C_4$-Alkyl

$A_3$ $C_1$-$C_4$-Alkyl, $C_2$-$C_4$-Alkenyl oder einen Rest $-OR'''_5$ oder $-N(R'''_{12})(R'''_{13})$,

$R'''_5$ $C_1$-$C_4$-Alkyl, $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyano oder Nitro substituiert ist,

$R'''_{12}$ und $R'''_{13}$ unabhängig voneinander je Wasserstoff, $C_1$-$C_8$-Alkyl, $C_2$-$C_8$-Alkenyl, Phenyl oder $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist und X Sauerstoff, Schwefel, SO, $SO_2$,
bedeuten.

14. Mittel gemäss Anspruch 1, dadurch gekennzeichnet dass es zur Erhöhung der Toleranz der Kultur eine nicht phytotoxische Menge eines Dichloracetamides der Formel VI enthält,

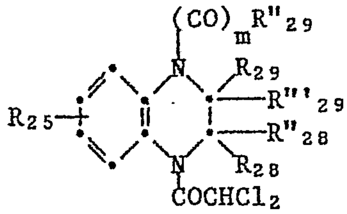

VI

worin

m Null oder Zahl 1,

$R_{25}$ Wasserstoff, $C_1$-$C_4$-Alkyl, unsubstituiert oder substituiert durch Hydroxy, $C_1$-$C_4$-Alkoxy oder Halogen,

$R_{28}$ und $R'''_{29}$ Wasserstoff, $C_1$-$C_4$-Alkyl unsubstituiert oder substituiert durch Halogen, Cyan oder einen Rest $-COA_3$; $C_2$-$C_4$-Alkenyl, $C_2$-$C_4$-Alkinyl, Cyano oder einen Rest $-COA_2$;

$R_{29}$, $R''_{29}$ und $R''_{28}$ sind unabhängig voneinander je Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl,

$R_{28}$ und $R''_{28}$ oder $R_{29}$ und $R'''_{29}$ zusammen mit dem Kohlenstoffatom auch die Oxogruppe $>C=O$

$A_2$ $C_1$-$C_4$-Alkyl, $C_2$-$C_4$-Alkenyl, $-N(R'''_{12})(R'''_{13})$,

$A_3$ $C_1$-$C_4$-Alkyl, $C_2$-$C_4$-Alkenyl oder einen Rest $-COR''_5$ oder $-N(R''_{12})(R''_{13})$,

$R''_5$ $C_1$-$C_4$-Alkyl, $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyano oder Nitro substituiert ist,

$R'''_{12}$ und $R'''_{13}$ unabhängig voneinander je Wasserstoff, $C_1$-$C_8$-Alkyl, $C_3$-$C_8$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist

bedeuten.

15. Mittel gemäss Anspruch 1, dadurch gekennzeichnet dass es zur Erhöhung der Toleranz der Kultur eine nicht phytotoxische Menge eines Dichloracetamides der Formel VII enthält,

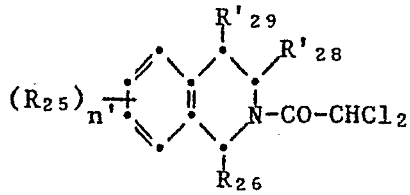

VII

worin n Null bis 2, n' Null, 1, 2 oder 3,

$R_{25}$ Wasserstoff, $C_1$-$C_4$-Alkyl, unsubstituiert oder substituiert durch Hydroxy, $C_1$-$C_4$-Alkoxy oder Halogen, $C_1$-$C_4$-Alkyl-$S(O)_n$-, Phenyl-$S(O)_n$- durch Halogen, Methyl, Methoxy substituiertes Phenyl-$S(O)_n$-

$R_{26}$ Wasserstoff, $C_1$-$C_6$-Alkyl, $C_3$-$C_6$-Alkenyl $C_3$-$C_6$-Cycloalkyl, Phenyl oder $C_1$-$C_4$-Aralkyl bedeutet wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert ist,

$R'_{28}$ Wasserstoff, $C_1$-$C_6$-Alkyl unsubstituiert oder substituiert durch Halogen, Cyan oder einen Rest $-COA_3$;

$C_3$-$C_6$-Alkenyl, $C_3$-$C_6$-Alkinyl, $C_2$-$C_6$-Alkoxyalkyl, Cyano oder $-COA_2$,

$R'_{29}$ Wasserstoff, $C_1$-$C_6$-Alkyl, $C_1$-$C_6$-Halogenalkyl, $C_3$-$C_6$-Alkenyl, $C_3$-$C_6$-Alkinyl oder $C_2$-$C_6$-Alkoxyalkyl,

$A_2$ $C_1$-$C_4$-Alkyl, $C_2$-$C_4$-Alkenyl, $-NR''_{12}R''_{13}$

$A_3$ $C_1$-$C_4$-Alkyl, $C_2$-$C_4$-Alkenyl oder einen Rest $-OR''_5$ oder $-NR''_{13}R''_{14}$,

$R''_5$ $C_1$-$C_4$-Alkyl, $C_1$-$C_4$-Aralkyl, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyano oder Nitro, substituiert ist,

$R''_{13}$ und $R''_{14}$ unabhängig voneinander je Wasserstoff, $C_1$-$C_8$-Alkyl, $C_3$-$C_8$-Alkenyl, Phenyl oder $C_1$-$C_4$-Aralkyl bedeuten, wobei der Phenylkern unsubstituiert oder durch Halogen, $C_1$-$C_4$-Alkyl,

$C_1$-$C_4$-Halogenalkyl, $C_1$-$C_4$-Alkoxy, $C_1$-$C_4$-Halogenalkoxy, Methylthio, Cyan oder Nitro substituiert sein kann,
bedeuten.

16. Neue N-Dichloracetyl-tetrahydroisochinoline der Formel VII'

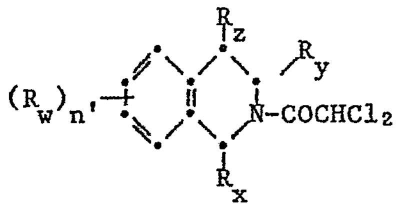

VII'

worin

n' Null bis 3

$R_w$ Wasserstoff, Halogen $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxyalkyl, Nitro, der Dioxymethylenrest,

$R_w$, $R_x$, $R_y$, $R_z$ Wasserstoff, $C_1$-$C_6$-Alkyl, $C_3$-$C_6$-Alkenyl mit der Massgabe, dass einer der Reste $R_w$, $R_x$, $R_y$, $R_z$ verschieden von Wassserstoff sein muss.

17. Verfahren zur Herstellung der N-Dichloracetyl-tetrahydroisochinoline der Formel VII', Anspruch 16, dadurch gekennzeichnet, dass man ein Acylhalid der Formel II'

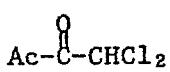

II'

worin Ac Chlor, Brom oder den Rest 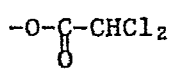 bedeutet,

in einem inerten organischen Lösungsmittel in Gegenwart der mindestens äquimolaren Menge eines säurebindenden Mittels mit einem Amin der Formel VIIa' umsetzt,

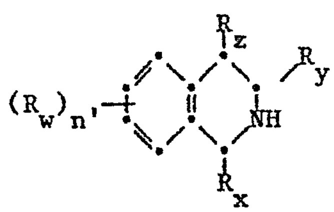

VIIa'

wobei n', $R_w$, $R_x$, $R_y$, $R_z$ die unter Formel VII', Anspruch 16, gegebene Bedeutung haben.

18. Mittel gemäss Anspruch 1, dadurch gekennzeichnet dass es zur Erhöhung der Toleranz der Kultur eine nicht phytotoxische Menge eines Dichloracetamides der Formel VIII enthält,

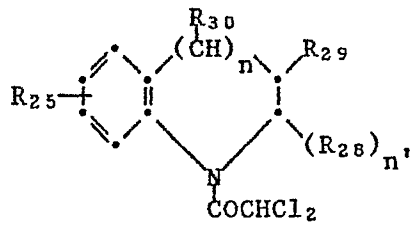

(VIII)

worin

n' Null oder eins oder zwei

n Null oder eine Zahl von 1 bis 3

$R_{25}$ Wasserstoff, $C_1$-$C_4$-Alkyl, unsubstituiert oder substituiert durch Hydroxy, $C_1$-$C_4$-Alkyl oder Halogen,

$R_{28}$ Wasserstoff, $C_1$-$C_4$-Alkyl, unsubstituiert oder substituiert durch Halogen, Cyan oder einen Rest $-COA_3$; $C_2$-$C_4$-Alkenyl oder $C_2$-$C_4$-Alkinyl;

$R_{29}$ Wasserstoff, $C_1$-$C_4$-Halogenalkyl, $C_2$-$C_4$-Alkenyl, $C_2$-$C_4$-Alkinyl oder $C_2$-$C_4$-Alkoxyalkyl;

$R_{30}$ dasselbe wie $R_{28}$ aber unabhängig davon,

$A_3$ $C_1$-$C_4$-Alkyl, $C_2$-$C_4$-Alkenyl, einen Rest $-OR''_5$ oder $-NR''_{12}$, $R''_{13}$,

$R''_5$ $C_1$-$C_4$-Alkyl, $C_1$-$C_4$-Aralky, wobei der Phenylkern unsubstituiert oder durch $C_1$-$C_4$-Alkyl, $C_1$-$C_4$-Alkoxy, Halogen, $C_1$-$C_4$-Halogenalkyl Cyan oder Nitro substituiert sein kann; wenn n Null ist kann $R_5$ auch Wasserstoff bedeuten,

$R''_{12}$ und $R''_{13}$ unabhängig voneinander je Wasserstoff, $C_1$-$C_8$-Alkyl, $C_3$-$C_6$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sein kann,

bedeuten.

19. Mittel gemäss Anspruch 1, dadurch gekennzeichnet dass es zur Erhöhung der Toleranz der Kultur eine nicht phytotoxische Menge eines Dichloracetamides der Formel IX enthält,

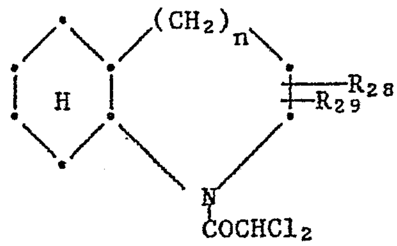

(IX)

worin n Null eins, zwei, oder drei

$R_{28}$ Wasserstoff, $C_1$-$C_4$-Alkyl unsubstituiert oder substituiert durch Halogen, Cyan oder einen Rest -CO-$A_3$

$C_2$-$C_4$-Alkenyl oder $C_2$-$C_4$-Alkinyl, $C_2$-$C_4$-Alkoxyalkyl oder Cyan;

$R_{29}$ Wasserstoff, $C_1$-$C_4$-Alkyl, $C_1$-$C_4$-Halogenalkyl, $C_2$-$C_4$-Alkenyl, $C_2$-$C_4$-Alkinyl oder $C_2$-$C_4$-Alkoxyalkyl;

$A_3$ $C_1$-$C_4$-Alkyl, $C_2$-$C_4$-Alkenyl, einen Rest -OR"$_5$ oder -NR"$_{12}$, R"$_{13}$,

$R_5$" $C_1$-$C_4$-Alkyl, $C_1$-$C_4$-Aralkyl wobei der Phenylkern unsubstituiert oder durch $C_1$-$C_4$-Alkyl, $C_1$-$C_4$-Alkoxy, Halogen, $C_1$-$C_4$-Halogenalkyl, Cyan oder Nitro substituiert sein kann; wenn m Null ist, kann $R_5$ auch Wasserstoff sein;

R"$_{12}$ und R"$_{13}$ unabhängig voneinander je Wasserstoff, $C_1$-$C_8$-Alkyl, $C_3$-$C_6$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sein kann,

bedeuten.

20. Mittel gemäss Anspruch 1, dadurch gekennzeichnet dass es zur Erhöhung der Toleranz der Kultur eine nicht phytotoxische Menge eines Dichloracetamides der Formel X enthält,

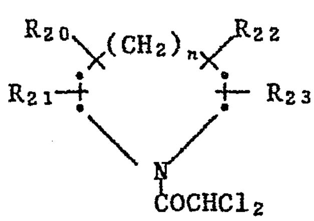

(X)

n Null eins, zwei, oder drei

$R''_{20}$ einen Rest $-COOR'_5$, $COR'_6$ der auch über eine $C_1$-$C_4$-Alkylenbrücke gebunden sein kann,

$R_{21}$, $R_{22}$ und $R_{23}$ unabhängig voneinander je Wasserstoff oder $C_1$-$C_4$-Alkyl

$R'_5$ einen $C_1$-$C_4$-Alkylrest; einen $C_1$-$C_4$-Aralkylrest der im Phenylkern unsubstituiert oder durch Halogen, $C_1$-$C_4$-Alkyl, $C_1$-$C_4$-Alkoxy, $C_1$-$C_4$-Halogenalkyl, Cyan oder Nitro substituiert ist; wenn m Null ist, kann $R'_5$ auch Wasserstoff sein,

$R'_6$ Wasserstoff, einen $C_1$-$C_4$-Alkyl, $C_1$-$C_4$-Halogenalkyl- oder $C_2$-$C_8$-Alkoxyalkylrest; Phenyl oder $C_1$-$C_4$-Aralkyl wobei der Phenylrest unsubstituiert oder durch Halogen, Cyan, $C_1$-$C_4$-Alkyl, $C_1$-$C_4$-Halogenalkyl substituiert sein kann; einen $C_2$-$C_4$-Alkenylrest oder ein Aminorest $-NR_{12}R_{13}$, wobei

$R_{12}$ und $R_{13}$ unabhängig voneinander je Wasserstoff, $C_1$-$C_8$-Alkyl, $C_3$-$C_6$-Alkenyl, Phenyl oder $C_1$-$C_4$-Aralkyl bedeuten, wobei der Phenylkern unsubstituiert oder durch Halogen, $C_1$-$C_4$-Alkyl, $C_1$-$C_4$-Halogenalkyl, $C_1$-$C_4$-Alkoxy, $C_1$-$C_4$-Halogenalkoxy, Methylthio, Cyan oder Nitro substituiert sein kann,

bedeuten.

21. Mittel gemäss Anspruch 1, dadurch gekennzeichnet dass es zur Erhöhung der Toleranz der Kultur eine nicht phytotoxische Menge eines Dichloracetamides der Formel XI enthält,

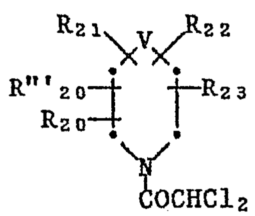

(XI)

worin

$R_{20}$ Wasserstoff oder $C_1$-$C_4$-Alkyl

$R'''_{20}$ Phenyl, durch Halogen, $C_1$-$C_4$-Alkyl, $C_1$-$C_4$-Alkoxy, $C_1$-$C_4$-Alkylthio substituiertes Phenyl,

$R_{21}$ Wasserstoff oder $C_1$-$C_4$-Alkyl

$R_{22}$ Wasserstoff oder $C_1$-$C_4$-Alkyl

$R_{23}$ Wasserstoff oder $C_1$-$C_4$-Alkyl bedeutet.

V Sauerstoff, $>CO$, $>C(OR''_{25})OR''_{26}$, $>C(R''_{25})R''_{26}$

oder $S(O)_{n'}$

n' Null 1, oder 2

$R''_{25}$ eine $C_4$-$C_6$-Alkylenbrücke, die geradkettig oder verzweigt sein kann

$R''_{26}$ dasselbe wie $R_{25}$ bedeuten.

22. Mittel gemäss Anspruch 1, dadurch gekennzeichnet dass es zur Erhöhung der Toleranz der Kultur eine nicht phytotoxische Menge eines Dichloracetamides der Formel XII enthält,

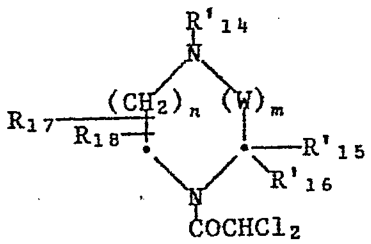

(XII)

worin

m Null oder 1

n Null, 1, 2 oder 3

$R''_6$ Wasserstoff, $C_1$-$C_4$-Alkyl, durch Halogen, $C_1$-$C_4$-Alkoxy substituiertes $C_1$-$C_4$-Alkyl, $NR_{12}R_{13}$, $C_2$-$C_4$-Alkenyl, Phenyl, $C_1$-$C_4$-Aralkyl wobei der Phenylkern durch Halogen, $C_1$-$C_4$-Alkyl, $C_1$-$C_4$-Alkoxy, $C_1$-$C_4$-Alkylthio substituiert sein kann; $C_1$-$C_8$-Alkoxy,

$R_{12}$ Wasserstoff, $C_1$-$C_8$-Alkyl, $C_3$-$C_6$-Alkenyl,

$R_{13}$ Wasserstoff, $C_1$-$C_8$-Alkyl, $C_3$-$C_6$-Alkenyl, Phenyl, $C_1$-$C_4$-Aralkyl wobei Phenyl durch Halogen, $C_1$-$C_4$-Alkyl, $C_1$-$C_4$-Alkoxy und Alkylthio substituiert sein kann,

$R'_{14}$ Wasserstoff oder $C_1$-$C_4$-Alkyl, Phenyl, $C_1$-$C_4$-Aralkyl wobei der Phenylkern durch Halogen, $C_1$-$C_4$-Alkyl, $C_1$-$C_4$-Alkoxy und Alkylthio substituiert sein kann; $-CO-R''_6$

$R'_{15}$ Wasserstoff oder $C_1$-$C_4$-Alkyl

$R'_{16}$ Wasserstoff oder $C_1$-$C_4$-Alkyl
$R_{17}$ Wasserstoff oder $C_1$-$C_4$-Alkyl
$R_{18}$ Wasserstoff oder $C_1$-$C_4$-Alkyl
$R_{17}$ und $R_{18}$ zusammen eine $C_3$-$C_5$-Alkylenkette
W einen Rest $>CO$, $>C(OR_{24})_2$ oder $>CR_{25}R_{26}$,

$R_{24}$ $C_1$-$C_4$-Alkyl,
$R_{25}$ Wasserstoff, $C_1$-$C_4$-Alkyl unsubstituiert oder substituiert durch Hydroxy, $C_1$-$C_4$-Alkoxy oder Halogen,
$R_{26}$ Wasserstoff, $C_1$-$C_4$-Alkyl, $C_5$-$C_8$-Cycloalkyl, Phenyl oder $C_1$-$C_4$-Aralkyl wobei der Phenylkern unsubstituiert oder substituiert ist durch Halogen Nitro, Cyan, $C_1$-$C_4$-Alkyl, $C_1$-$C_4$-Alkoxy, Methylthio, $C_1$-$C_4$-Halogenalkoxy, Carboxy, $C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-Alkylcarbamoyl, $C_1$-$C_4$-Alkylsulfonyl, $C_1$-$C_4$-Alkylsulfuryl, Sulfamoyl oder Di-$C_1$-$C_4$-Alkylsulfamoyl bedeuten.

23. Mittel gemäss Anspruch 1, dadurch gekennzeichnet dass es zur Erhöhung der Toleranz der Kultur eine nicht phytotoxische Menge eines Dichloracetamides der Formel XIII enthält,

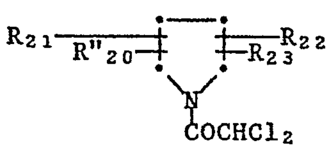 (XIII)

worin
$R''_{20}$ einen Rest $-COOR'_5$, $-COR'_6$ oder einen $C_1$-$C_4$-Alkylrest, der durch $-COOR'_5$ oder $-COR'_6$ substituiert ist,
$R_{21}$ Wasserstoff oder $C_1$-$C_4$-Alkyl,
$R_{22}$ Wasserstoff oder $C_1$-$C_4$-Alkyl,
$R_{23}$ Wasserstoff oder $C_1$-$C_4$-Alkyl,
$R'_5$ $C_1$-$C_4$-Alkyl, unsubstituiert oder ein $C_1$-$C_4$-Aralkylrest dessen Phenylkern unsubstituiert oder substituiert durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxy, $C_1$-$C_4$-Alkylcarbonyl, $C_1$-$C_4$-Alkoxy-

0190105

carbonyl, Carbamoyl, $C_1$-$C_4$-Alkylcarbamoyl, Di-$C_1$-$C_4$-alkylcarbamoyl, $C_1$-$C_4$-Alkylsulfonyl, $C_1$-$C_4$-Alkylsulfuryl, Sulfamoyl oder Di-$C_1$-$C_4$-alkylsulfamoyl,

$R'_6$ Wasserstoff, $C_1$-$C_4$-Alkyl, unsubstituiert oder substituiert durch Halogen oder $C_1$-$C_4$-Alkoxy; Phenyl oder $C_1$-$C_4$-Aralkyl, wobei der Phenylkern unsubstituiert oder durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-alkoxycarbamoyl, $C_1$-$C_4$-Alkylsulfonyl, $C_1$-$C_4$-Alkylsulfuryl, Sulfamoyl oder Di-$C_1$-$C_4$-alkylsulfamoyl einen Rest $-NR_{12}R_{13}$, $C_2$-$C_4$-Alkenyl

$R_{12}$ Wasserstoff, $C_1$-$C_8$-Alkyl, $C_3$-$C_6$-Alkenyl, Phenyl oder $C_1$-$C_4$-Aralkyl wobei der Phenylkern unsubstituiert oder wie oben angegeben substituiert sein kann,

$R_{13}$ dasselbe wie $R_{12}$

bedeuten.

24. Mittel gemäss Anspruch 1, dadurch gekennzeichnet dass es zur Erhöhung der Toleranz der Kultur eine nicht phytotoxische Menge eines Dichloracetamides der Formel XIV enthält,

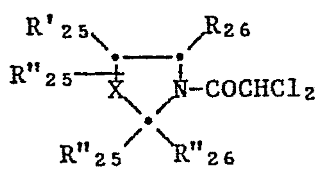

(XIV)

worin

$R'_{25}$ Wasserstoff, $C_1$-$C_4$-Alkyl, unsubstituiert oder substituiert durch Hydroxy, $C_1$-$C_4$-Alkoxy, Halogen, $C_1$-$C_4$-Alkyl-$S(O)_n$,-, Phenyl-$S(O)_n$,-

$R''_{25}$ dasselbe wie $R'_{25}$

$R'_{26}$ Wasserstoff, $C_1$-$C_4$-Alkyl unsubstituiert oder substituiert durch Reste $-S(O)'_nR_{27}$, $-OR'_{27}$, $C_5$-$C_8$-Cycloalkyl oder durch Phenyl, welches unsubstituiert oder substituiert ist durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxy-

carbonyl, Carbamoyl, $C_1$-$C_4$-Alkylcarbamoyl, Di-$C_1$-$C_4$-alkylcarbamoyl, $C_1$-$C_4$-Alkylsulfonyl, $C_1$-$C_4$-Alkylsulfuryl, Sulfamoyl oder Di-$C_1$-$C_4$-alkylsulfamoyl,

$R''_{26}$ dasselbe wie $R'_{26}$ oder Phenyl, $C_1$-$C_4$-Aralkyl wobei die Phenylkerne durch Halogen, $C_1$-$C_4$-Alkyl, $C_1$-$C_4$-Alkoxy, $C_1$-$C_4$-Alkylthio, $C_1$-$C_4$-Halogenalkyl substituiert sein können

$R_{27}$ $C_1$-$C_4$-Alkyl, unsubstituiert oder substituiert durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl,

n' null, eins oder zwei

X Sauerstoff, Schwefel oder S(O)n'

$C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-alkylcarbamoyl, $C_1$-$C_4$-Alkylsulfonyl, $C_1$-$C_4$-Alkylsulfonyl, Sulfamoyl oder Di-$C_1$-$C_4$-alkylsulfamoyl,

$R'_{27}$ Wasserstoff oder dasselbe wie $R_{27}$

bedeuten.

25. Mittel gemäss Anspruch 1, dadurch gekennzeichnet dass es zur Erhöhung der Toleranz der Kultur eine nicht phytotoxische Menge eines Dichloracetamides der Formel XV enthält,

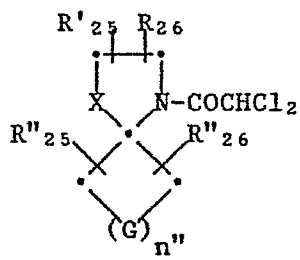

(XV)

worin

G Kohlenstoff, Sauerstoff, Schwefel, n" 4 bis 7

$R'_{25}$ Wasserstoff, $C_1$-$C_4$-Alkyl, unsubstituiert oder substituiert durch Hydroxy, $C_1$-$C_4$-Alkoxy oder Halogen,

$R''_{25}$ Wasserstoff, $C_1$-$C_4$-Alkyl, unsubstituiert oder substituiert durch Hydroxy, $C_1$-$C_4$-Alkyl oder Halogen,

$R_{26}$ und $R''_{26}$ Wasserstoff, $C_1$-$C_4$-Alkyl unsubstituiert oder substituiert durch Reste $-S(O)_{n'}R_{27}$, $-OR'_{27}$ oder durch Phenyl, welches unsubstituiert oder substituiert ist durch Halogen, Nitro, Cyan,

$C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxycarbonyl, Carbamoyl, $C_1$-$C_4$-Alkylcarbamoyl, Di-$C_1$-$C_4$-alkylcarbamoyl, $C_1$-$C_4$-Alkylsulfonyl, $C_1$-$C_4$-Alkylsulfuryl, Sulfamoyl oder Di-$C_1$-$C_4$-alkylsulfamoyl,

X Sauerstoff, oder ein Rest $S(O)_{n'}$

n' Null, 1, oder 2

n" 4, 5, 6 oder 7

$R_{27}$ $C_1$-$C_4$-Alkyl, unsubstituiert oder substituiert durch Phenyl, welches seinerseits unsubstituiert oder substituiert ist durch Halogen, Nitro, Cyan, $C_1$-$C_4$-Alkyl, $C_1$-$C_4$-Alkoxy, Methylthio, $C_1$-$C_4$-Halogenalkyl, $C_1$-$C_4$-Halogenalkoxy, Carboxyl, $C_1$-$C_4$-Alkylcarbonyl, $C_1$-$C_4$-Alkoxycarbonyl, $C_1$-$C_4$-Alkylsulfonyl, $C_1$-$C_4$-Alkylsulfuryl, Sulfamoyl oder $C_1$-$C_4$-Dialkylsulfamoyl,

$R'_{27}$ Wasserstoff oder dasselbe wie $R_{27}$

bedeuten.

26. Mittel gemäss Anspruch 1, dadurch gekennzeichnet dass es zur Erhöhung der Toleranz der Kultur eine nicht phytotoxische Menge eines Dichloracetamides der Formel XVI enthält,

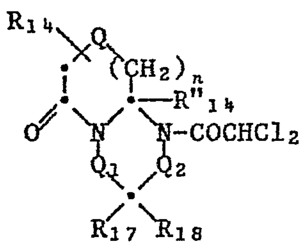

(XVI)

worin

n Null, 1, 2 oder 3,

Q einen Rest $-(CH_2)_p$

$Q_1$ einen Rest $-(CHR_{19})o^1$

$Q_2$ einen Rest $-(CHR'_{19})o^2$

$R_{14}$ Wasserstoff oder $C_1$-$C_4$-Alkyl

$R''_{14}$ Wasserstoff, $C_1$-$C_4$-Alkyl einen Rest $-COR''_6$ oder Phenyl, welches unsubstituiert oder substituiert ist durch Halogen, Nitro, Cyan, $C_1$-$C_4$-Alkyl, $C_1$-$C_4$-Alkoxy, Methylthio, $C_1$-$C_4$-Halogenalkyl,

$C_1$-$C_4$-Halogenalkoxy, Carboxyl, $C_1$-$C_4$-Alkylcarbonyl, $C_1$-$C_4$-Alkoxycarbonyl, $C_1$-$C_4$-Halogenalkoxycarbonyl, Carbamoyl, $C_1$-$C_4$-Alkylcarbamoyl, Di-$C_1$-$C_4$-Alkylcarbamoyl, $C_1$-$C_4$-Alkylsulfonyl, $C_1$-$C_4$-Alkylsulfuryl, Sulfamoyl, $C_1$-$C_4$-Alkylsulfamoyl oder Di-$C_1$-$C_4$-alkylsulfamoyl,

$R_{17}$ Wasserstoff oder $C_1$-$C_4$-Alkyl

$R_{18}$ Wasserstoff oder $C_1$-$C_4$-Alkyl

$R_{19}$ Wasserstoff oder $C_1$-$C_4$-Alkyl

$R'_{19}$ Wasserstoff oder $C_1$-$C_4$-Alkyl

$o^1$ Null oder 1,

$o^2$ Null oder 1 und

p Null oder 1

$R''_6$ Wasserstoff, $C_1$-$C_4$-Alkyl, unsubstituiert oder durch Halogen oder $C_1$-$C_4$-Alkoxy substituiert,

bedeuten.

27. Mittel gemäss Anspruch 1, dadurch gekennzeichnet, dass es zur Erhöhung der Toleranz der Kultur eine nicht-phytotoxische Menge von N,N-Diallyl-dicloracetamid enthält.

28. Mittel gemäss Anspruch 1, dadurch gekennzeichnet, dass es zur Erhöhung der Toleranz der Kultur eine nicht phytotoxische Menge von N-Allyl-N-(2-methoxy-1-methyl-äthyl)-dichloracetamid enthält.

29. Mittel gemäss Anspruch 1, dadurch gekenzeichnet, dass es zur Erhöhung der Toleranz der Kultur eine nichtphytotoxische Menge von N-Benzyloxycarbonyl-N'-dichloracetyl-hydrazinomethyl-äthylphosphinsäure-isopropylester der Formel

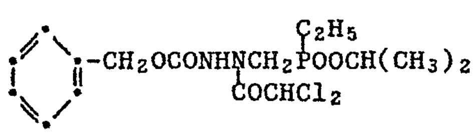

enthält.

30. Mittel gemäss Anspruch 1, dadurch gekennzeichnet, dass es zur Erhöhung der Toleranz der Kultur eine nichtphytotoxische Menge von N-Aethoxycarbonyl-N'-dichloracetyl-hydrazinomethyl-phosphonsäure-di-isopropylester der Formel

$$H_5C_2OCONHNCH_2PO(OC_3H_7iso)_2$$
$$COCHCl_2$$

enthält.

31. Mittel gemäss Anspruch 1, dadurch gekennzeichnet, dass es zur Erhöhung der Toleranz der Kultur eine nichtphytotoxische Menge von N-Allyl-N-n-butoxy-äthoxy-äthyl-dichloracetamid enthält.

32. Mittel gemäss Anspruch 1, dadurch gekennzeichnet, dass es zur Erhöhung der Toleranz der Kultur eine nicht phytotoxische Menge von N-(3,4-Dimethoxybenzyl)-N-isopropyl-dichloracetamid enthält.

33. Mittel gemäss Anspruch 1, dadurch gekennzeichnet, dass es zur Erhöhung der Toleranz der Kultur eine nichtphytotoxische Menge von 4-Dichloracetyl-2,3-dihdro-3-methyl-1,4-benzoxazin enthält.

34. Mittel gemäss Anspruch 1, dadurch gekennzeichnet, dass es zur Erhöhung der Toleranz der Kultur eine nichtphytotoxische Menge von 4-Dichloracetyl-2,3-dihydro-3,6-dimethyl-1,4-benzoxazin enthält.

35. Mittel gemäss Anspruch 1, dadurch gekennzeichnet, dass es zur Erhöhung der Toleranz der Kultur eine nicht phytotoxische Menge von 4-Dichloracetyl-2,3-dihydro-3-methyl-1,4-benzthiazin enthält.

36. Mittel gemäss Anspruch 1, dadurch gekennzeichnet, dass es zur Erhöhung der Toleranz der Kultur eine nichtphytotoxische Menge von N-Dichloracetyl-1,2,3,4-tetrahydro-isochinolin enthält.

37. Mittel gemäss Anspruch 1, dadurch gekennzeichnet, dass es zur Erhöhung der Toleranz der Kultur eine nichtphytotoxische Menge von 5-Dichloracetyl-3,3,6-trimethyl-9-oxo-1,5-diaza-bicyclo[4,3,0]nonan der Formel

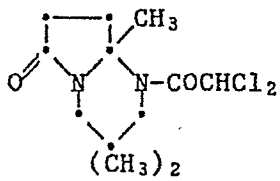

enthält.

38. Mittel gemäss Anspruch 1, dadurch gekennzeichnet, dass es zur Erhöhung der Toleranz der Kultur eine nichtphytotoxische Menge von 2,2-Dimethyl-N-Oxazolidinyl-dichloracetamid enthält.

39. Mittel gemäss Anspruch 1, dadurch gekennzeichnet, dass es zur Erhöhung der Toleranz der Kultur eine nichtphytotoxische Menge von N,N-bis-Dichloracetylpiperazin enthält.

40. Verfahren zur selektiven Bekämpfung von unerwünschtem Pflanzenwuchs, dadurch gekennzeichnet, dass man das herbizide Mittel nach Anspruch 1 vor, bei oder nach Aussaat der Kulturpflanzen, vor oder während des Auflaufens der Kulturpflanzen ausbringt.

41. Verfahren zur selektiven Bekämpfung von unerwünschtem Pflanzenwuchs, dadurch gekennzeichnet, dass man N-(2-Methoxycarbonylphenylsulfonyl)-N'-(4,6-bis-difluoromethoxypyrimidin-2-yl)-harnstoff der Formel I und Dichloracetamide der Formel II vor, bei oder nach der Aussaat der Kulturpflanzen, vor oder während des Auflaufens der Kulturpflanzen gleichzeitig oder nacheinander in beliebiger Reihenfolge ausbringt.

42. Verfahren zur selektiven Bekämpfung von unerwünschtem Pflanzenwuchs, dadurch gekennzeichnet, dass man das Saatgut der Kulturpflanzen mit einem Dichloracetamid der Formel II behandelt.

43. Verfahren nach Anspruch 40, dadurch gekennzeichnet, das die Kulturpflanzen Mais, Reis, Hirse oder Getreidearten sind.

44. Verfahren nach Anspruch 40, dadurch gekennzeichnet, das die Kulturpflanze Mais ist.

45. Verfahren gemäss Anspruch 40, dadurch gekennzeichnet, dass der Sulfonylharnstoff und das Antidot in einer Aufwandmenge von jeweils 0,005 bis 10 kg pro ha Kulturboden verwendet werden.

46. Verfahren gemäss Anspruch 40, dadurch gekennzeicnet, dass der Sulfonylharnstoff und das Antidot in einer Aufwandmenge von jeweils 0,05 bis 1 kg pro ha Kulturboden verwendet werden.

FO 7.5 NU/sm*